# EUROPEAN PATENT SPECIFICATION

(11) **EP 1 962 862 B1**
(45) Date of publication and mention of the grant of the patent: **04.09.2013**
(21) Application number: 06847032.7
(22) Date of filing: 19.12.2006
(51) Int. Cl.: A61P 31/10, A61K 31/661

(54) **ALKYL PHOSPHOLIPID DERIVATIVES WITH REDUCED CYTOTOXICITY AND USES THEROF**
ALKYLPHOSPHOLIPIDDERIVATE UND DEREN VERWENDUNG
NOUVEAUX ALKYLPHOSPHOLIPIDES ET LEUR UTILISATION

(30) Priority: 19.12.2005 US 751438 P; 20.12.2005 EP 05027823
(43) Date of publication of application: 03.09.2008
(73) Proprietor: Æterna Zentaris GmbH, 60314 Frankfurt am Main (DE)
(72) Inventor: PERRISSOUD, Daniel, 61348 Bad Homburg (DE); PIETRAS, Mathias, 61279 Grävenwiesbach (DE); ENGEL, Jürgen, 63755 Alzenau (DE)
(86) International application number: PCT/EP2006/069873
(87) International publication number: WO 2007/071658

(56) References cited:
- EP-A- 0 108 565
- EP-A- 0 579 939
- EP-A1- 0 521 353
- EP-A1- 0 534 445
- EP-A2- 0 507 337
- WO-A-00/33917
- WO-A-03/028736
- WO-A-03/061669
- WO-A-03/080071
- WO-A-2004/012744
- WO-A-2004/041167
- WO-A-2006/122716
- DE-A1- 3 942 933
- DE-A1- 19 835 611
- US-A1- 2003 216 355
- US-A1- 2004 242 543
- VERMA NAVIN K ET AL: "Possible mechanism of miltefosine-mediated death of Leishmania donovani." ANTIMICROBIAL AGENTS AND CHEMOTHERAPY. AUG 2004, vol. 48, no. 8, August 2004 (2004-08), pages 3010-3015, XP002372749 ISSN: 0066-4804
- WALOCHNIK JULIA ET AL: "Cytotoxic activities of alkylphosphocholines against clinical isolates of Acanthamoeba spp." ANTIMICROBIAL AGENTS AND CHEMOTHERAPY. MAR 2002, vol. 46, no. 3, March 2002 (2002-03), pages 695-701, XP001146164 ISSN: 0066-4804
- SUNDAR S ET AL: "Trial of oral miltefosine for visceral leishmaniasis" LANCET, vol. 352, no. 9143, 5 December 1998 (1998-12-05), pages 1821-1823, XP004835202 ISSN: 0140-6736
- KOUFAKI M ET AL: "Alkyl and alkoxyethyl antineoplastic phospholipids." JOURNAL OF MEDICINAL CHEMISTRY. 21 JUN 1996, vol. 39, no. 13, 21 June 1996 (1996-06-21), pages 2609-2614, XP002372751 ISSN: 0022-2623
- AVLONITIS N ET AL: "Antileishmanial ring-substituted ether phospholipids" JOURNAL OF MEDICINAL CHEMISTRY, AMERICAN CHEMICAL SOCIETY. WASHINGTON, US, vol. 46, no. 5, 27 February 2003 (2003-02-27), pages 755-767, XP002383657 ISSN: 0022-2623
- STEKAR JURIJ ET AL: "SYNTHESIS, ANTITUMOR ACTIVITY, AND TOLERABILITY OF PHOSPHOLIPIDS CONTAINING NITROGEN HOMOLOGS" ANGEWANDTE CHEMIE. INTERNATIONAL EDITION, WILEY VCH VERLAG, WEINHEIM, DE, vol. 34, no. 2, 3 February 1995 (1995-02-03), pages 238-240, XP008078174 ISSN: 1433-7851
- KUCERA L S ET AL: "NOVEL MEMBRANE-INTERACTIVE ETHER LIPID ANALOGS THAT INHIBIT INFECTIOUS HIV-1 PRODUCTION AND INDUCE DEFECTIVE VIRUS FORMATION" AIDS RESEARCH AND HUMAN RETROVIRUSES, NEW YORK, NY, US, vol. 6, no. 4, April 1990 (1990-04), pages 491-501, XP008003515 ISSN: 0889-2229
- SGAMBATTI DE ANDRADE ET AL: "Randomised trial of efficacy of benznidazole in treatment of early Trypanosoma cruzi infection" LANCET THE, LANCET LIMITED. LONDON, GB, vol. 348, no. 9039, 23 November 1996 (1996-11-23), pages 1407-1413, XP005108591 ISSN: 0140-6736
- KONSTANTINOV S M ET AL: "HUMAN URINARY BLADDER CARCINOMA CELL LINES RESPOND TO TREATMENT WITH ALKYLPHOSPHOCHOLINES" CANCER LETTERS, NEW YORK, NY, US, vol. 144, no. 2, 1 October 1999 (1999-10-01), pages 153-160, XP008078176 ISSN: 0304-3835
- KONSTANTINOV, SPIRO M. ET AL: "Alkylphosphocholines induce apoptosis in HL-60 and U-937 leukemic cells" CANCER CHEMOTHERAPY AND PHARMACOLOGY , 41(3), 210-216 CODEN: CCPHDZ; ISSN: 0344-5704, 1998, XP008061677
- ENGEL, J. ET AL: "Perifosine: oncolytic, ether phospholipid" DRUGS OF THE FUTURE , 25(12), 1257-1260 CODEN: DRFUD4; ISSN: 0377-8282, 2000, XP008061690
- CRUL M ET AL: "Phase I and pharmacological study of daily oral administration of perifosine (D-21266) in patients with advanced solid tumours." EUROPEAN JOURNAL OF CANCER (OXFORD, ENGLAND : 1990) AUG 2002, vol. 38, no. 12, August 2002 (2002-08), pages 1615-1621, XP002431102 ISSN: 0959-8049
- HILGARD P ET AL: "D-21266, a new heterocyclic alkylphospholipid with antitumour activity." EUROPEAN JOURNAL OF CANCER (OXFORD, ENGLAND : 1990) MAR 1997, vol. 33, no. 3, March 1997 (1997-03), pages 442-446, XP002431103 ISSN: 0959-8049
- KONSTANTINOV, S. M. ET AL: "Efficacy of anticancer alkylphosphocholines in Trypanosoma brucei subspecies" ACTA TROPICA , 64(3,4), 145-154 CODEN: ACTRAQ; ISSN: 0001-706X, 1997, XP008078173
- KANETANI, FUJIO ET AL: "Synthesis, and physicochemical and antimicrobial properties of alkylphosphorylcholines" NIPPON KAGAKU KAISHI , (9), 1452-8 CODEN: NKAKB8; ISSN: 0369-4577, 1984, XP008080964
- SEIFERT, KARIN ET AL: "Effects of miltefosine and other alkylphosphocholines on human intestinal parasite Entamoeba histolytica" ANTIMICROBIAL AGENTS AND CHEMOTHERAPY , CODEN: AMACCQ; ISSN: 0066-4804, vol. 45, no. 5, 2001, pages 1505-1510, XP001147747
- COOPER, DAVID L. ET AL: "Molecular similarity of anti-HIV phospholipids" JOURNAL OF THE AMERICAN CHEMICAL SOCIETY , 115(26), 12615-16 CODEN: JACSAT; ISSN: 0002-7863, 1993, XP002444651
- BLAHA C ET AL: "In vitro activity of hexadecylphosphocholine (miltefosine) against metronidazole-resistant and -susceptible strains of Trichomonas vaginalis." THE JOURNAL OF ANTIMICROBIAL CHEMOTHERAPY FEB 2006, vol. 57, no. 2, February 2006 (2006-02), pages 273-278, XP002444652 ISSN: 0305-7453
- CROFT SIMON L ET AL: "Antiprotozoal activities of phospholipid analogues." MOLECULAR AND BIOCHEMICAL PARASITOLOGY, vol. 126, no. 2, February 2003 (2003-02), pages 165-172, XP002444653 ISSN: 0166-6851
- PAPAZAFIRI PANAGIOTA ET AL: "Structure-activity relationships of antineoplastic ring-substituted ether phospholipid derivatives" CANCER CHEMOTHERAPY AND PHARMACOLOGY, vol. 56, no. 3, September 2005 (2005-09), pages 261-270, XP019334189 ISSN: 0344-5704
- DUIJSINGS DANIËL ET AL: "Hexadecylphosphocholine causes rapid cell death in canine mammary tumour cells." EUROPEAN JOURNAL OF PHARMACOLOGY 19 OCT 2004, vol. 502, no. 3, 19 October 2004 (2004-10-19), pages 185-193, XP004598397 ISSN: 0014-2999
- STEKAR JURIJ ET AL: "Antineoplastic activity and tolerability of a novel heterocyclic alkylphospholipid, D-20133" CANCER CHEMOTHERAPY AND PHARMACOLOGY, SPRINGER VERLAG, BERLIN, DE, vol. 32, no. 6, 1993, pages 437-444, XP008080887 ISSN: 0344-5704
- UNGER C ET AL: "IN VIVO ANTILEISHMANIAL ACTIVITY OF HEXADECYLPHOSPHOCHOLINE AND OTHER ALKYLPHOSPHOCHOLINES" DRUGS OF TODAY / MEDICAMENTOS DE ACTUALIDAD, J.R. PROUS SS.A. INTERNATIONAL PUBLISHERS, ES, vol. 34, 1998, pages 133-140, XP008078170 ISSN: 0025-7656

## Description

### Technical field

The invention relates to alkyl phospholipid derivatives with reduced cytotoxicity that are useful in the treatment of various diseases and/or pathophysiological conditions in mammals caused by microorganisms, in particular fungi. Such alkyl phospholipids can be employed as single drugs or in combination therapies.

### Prior art

Alkyl phospholipids (APL) as a substance class have been known for several decades to possess properties and exhibit biological activity that can advantageously be exploited for treatment in various medicinal indications.

A comprehensive overview of different effects and uses of for instance alkyl phosphocholines is given in Drugs of Today, Vol. 34, Suppl. F, 1998.

Further literature with regard to alkyl phospholipids, their various uses as well as relevant indications (including respective standard therapies) comprise the following.

EP 0 108 565 discloses alkyl phosphocholines that are claimed to possess anti-neoplastic properties. WO 87/03478 describes alkyl phospholipids for use as anti-tumor medicaments. US 5,219,866 describes octadecyl-[2-(N-methylpiperidino)-ethyl]-phosphate as useful for the treatment of cancer as well as a process for its preparation. US 6,172,050, US 6,479,472 and EP 0 579 939 all disclose specific phospholipid derivatives and methods of using them as therapeutics, in particular against tumors. US 5,449,798 and US 5,958,906 are directed to phospholipid derivatives containing higher elements of the fifth group that are said to act as anti-neoplastic. US 6,093,704 describes the use of dopamine receptor antagonists in palliative tumor therapy reducing potential side effects of alkyl phosphocholines, such as miltefosine.

WO 2004/012744 relates to the use of alkyl phosphocholines in combination with antitumor medicaments.

EP 0 108 565 discloses alkyl phosphocholines that are claimed among others to have anti-fungal properties. Lu et al. describe the use of the natural bisphosphocholine irlbacholine and synthetic analogues thereof as antifungal agents (Lu Q et al., J. Nat. Prod. 1999, 62(6):824-828). Ganendren and co-workers studied for compounds with structural similarities to phospholipid substrates as inhibitors of phospholipases from the fungal pathogen Cryptococcus neoformans (Ganendren R et al., Antimicrob. Agents Chemother. 2004, 48(5):1561-1569).

Koufaki M et al. describe alkyl and alkoxyethyl phospholipids as antineoplastics for the treatment of tumors (Koufaki et al., J. Med. Chem. 1996, 39:2609-2614). Konstatinov et al. elucidate the apoptotic mode of action of selected alkylphospholipids (Konstatinov et al., Cancer Chemother. Pharmacol. 1998, 41:210-216). Engel et al. discuss the pharmacological activity of perifosine as an anti-tumor medicament (Engel et al., Drugs of the Future 2000, 25(12):1257-1260). WO 00/33917 discloses agents based on liposomes that may contain miltefosine or perifosine and may be used for treating tumors. EP 0 284 395 describes novel glycerol derivatives and antihypertensive agents for reducing blood pressure. Andresen and co-workers studied the biological activity of synthesized anticancer ether lipids that are specifically released by phospholipase A2 in tumor tissue (Andresen TL et al., J. Med. Chem. 2005, 48:7305-7314).

Protozoal diseases persist to be a worldwide burden. For most of these diseases curing therapies are not available. Leishmaniasis and Chagas disease are two prominent members of this class of diseases.

Leishmaniasis ranks third in the WHO neglected diseases list in terms of fatality numbers per year. With annually 60.000 cases of death only Malaria and tuberculosis cause more victims. It is estimated that worldwide 350 million people are at risk and currently 12 million people are infected. The disease is found in 88 countries of the new and old world with most infected people living in India, Bangladesh, Brazil and Sudan. Each year, 1-1.5 million new cases are reported. The DALY burden has been reported by WHO/TDR to be 860.000 for men and 1.200.000 for women.

Leishmaniasis is caused by protozoa of the genus Leishmania which are transmitted by sand flies (Phlebotomus sp. and Lutzomyia sp.). Two forms of the disease exist. Internal or visceral leishmaniasis is the most dangerous form and causes death within 6-12 month if untreated. The other form, called cutaneous leishmaniasis, leads to lesions in the skin and if untreated to ulcers. In cases were no spontaneous healing occurs it results in scars on body and face. Possible complications of untreated leishmaniasis comprise secondary infections of ulcer and development of the mucocutaneous form which can result in destruction of facial skin and mucosal parts.

The visceral form is found in the old (Leishmania species: L. donovani, L. infantum) and new (L. chagasi) world. It affects the subindian continent (India, Bangladesh and Nepal), parts of East Africa (Sudan and Ethiopia) and parts of South America (Brazil and Colombia). The number of new cases per year is 500.000 with a high rate of mortality. Visceral leishmaniasis is associated with fever, weight loss, enlargement of spleen and liver. If it is not treated it tends to be fatal.

The cutaneous form is widespread in both worlds. In the old world, Leishmania major and Leishmania tropica are the prominent pathogens. L. major is found in rural areas whereas L. tropica is found in urban areas. The main countries are Afghanistan, Pakistan and the whole Middle East, especially Iran, Iraq, Syria and Saudi-Arabia.

Currently, there is a tendency to leave the patients untreated because scars and painful ulcers are considered as non-life threatening. However, it is more likely to be the high risk of side effects caused by current treatment that could explain the wait-and-see position. In the new world the situation is more dangerous. Patients suffering from cutaneous leishmaniasis may develop the mucocutaneous form which leads to painful and disfiguring disruption of parts of the face. The disease is found all over Middle and South America with focal points in Venezuela, Peru, Bolivia and Guatemala.

The current standard therapy has to be given parenterally in hospitals and is highly toxic. AIDS and other immunosuppressive conditions such as malnutrition increase the risk. As a matter of fact, in many of the countries where visceral Leishmaniasis prevails they are also hot spots for AIDS and malnutrition. The current treatment options are limited. Since 50 years parenteral antimonials (sodium stiboglucanant, SSG, Pentostam™ and meglumine stiboglucanat Glucantime™) have been the standard therapy for Leishmaniasis. The side effects are very serious including vomiting, nausea, diarrhoea and anorexia. Creatine values have to be observed and ECG values have to be monitored during the whole course of treatment and a regular ECG monitoring is required.

The alternative and second line treatment amphotericin B has its advantage in the absence of resistance. However, in addition to the high cost for hospitalization there is a higher drug price. Side effects are similarly serious with additional drug-induced fevers and the drug is only approved for visceral leishmaniasis. A further alternative to overcome side effects is the use of liposomal amphotericin B. The drug paromomycin currently undergoes clinical phase III trials and it is reported that it is efficient in 95% of the cases and generally well tolerable All treatments are parenteral and require a longer period of hospitalization.

Compared to Leishmaniasis, for Chagas disease the situation is much more problematic. Chagas disease, also called American trypanosomiasis, is caused by the parasite protozoon Trypanosoma cruzi. It is endemic in 21 countries of South and Latin America. Currently 16-18 million people are infected and 100 million people are at risk. The disease is transmitted via blood-sucking insects the parasites being transmitted from the gastric-enteral path while the insect is incorporating blood of its victim.

In human the disease starts with an acute phase followed by a life-long chronic phase. In the acute phase it is sometimes associated with fever, swelling of lymph glands, enlargement of the liver and spleen as well as local inflammations. Because the acute phase is often not correctly if at all diagnosed, the infection stays untreated. As a consequence, Chagas disease enters the chronic phase which is characterized by the parasite's penetration of the heart muscle, severe local inflammation and chronic heart disease. Usually, patients die early on because of these cardiac diseases.

[General literature on Chagas Disease: Guzman-Bracho C, Trends Parasitol. 2001, 17(8):372-376; Roberts A et al., J. Am. Acad. Nurse Pract. 2001, 13(4):152-153; Tarleton RL et al., Parasitol. Today. 1999, 15(3):94-99; Anez N et al., Mem. Inst. Oswaldo Cruz 2004, Rio de Janeiro, 99(8): 781-787; Second Report of the WHO Expert Committee, WHO technical report series 905, WHO 2002; Behbehani K, Bull World Health Organ. 1998, 76 (Suppl. 2):64-67; Umezawa ES et al., Lancet. 2001, 357(9258):797-799].

So far the treatment options have been limited. There are only two drugs approved for Chagas disease: Benznidazole (Radanil™) and Nifurtimox (Lampit™). They are very toxic and their use is limited to the acute phase of the disease only. Only poor evidence has been reported that at least one of the two drugs is of some efficiency in the chronic phase.

Currently, the drug of choice is Benznidazole which is given at 5-7 mg per kg bodyweight for 60 days. Side effect can be very severe and request an immediate notification to the physician. Very common side effects reported are convulsions (seizures), numbness, tingling pain, weakness in hands or feet, reddish discoloration of skin, abdominal or stomach pain, diarrhoea, nausea and vomiting. Rare side effects are fever or chills, pinpoint red spots on skin, skin rash, sore throat, unusual bleeding or bruising, confusion, dizziness, headache, restlessness, temporary loss of memory, trouble in sleeping, difficulty in concentrating, unusual tiredness or weakness.

The older substance Nifurtimox is much less in use than Benzidazole. It has to be given for 50 days at 8-10 mg per kg bodyweight. Known side effects are abdominal or stomach pain, dizziness, headache, loss of appetite, nausea, vomiting, weight loss, skin rash, chills or sore throat, clumsiness or unsteadiness, confusion, convulsions (seizures), decreased sexual drive or ability, fever, forgetfulness, irritability, mood or mental changes, muscle weakness, numbness, tingling, pain, weakness in hands or feet, trembling, trouble in sleeping, uncontrolled back-and-forth and/or rolling eye movements, unusual excitement, nervousness and restlessness.

[Literature on Chagas disease (standard) treatment: Coura JR et al., Mem. Inst. Oswaldo. Cruz 2002, Rio de Janeiro, 97(1): 3-24; Docampo R, Curr. Pharm. Des. 2001, 7(12):1157-1164; Kayser O et al., Pharm. Unserer Zeit. 1999, 28(4):177-185; Cerecetto H et al., Curr. Top. Med. Chem. 2002, 2(11):1187-1213; Urbina JA, Curr. Opin. Infect. Dis. 2001, 14(6):733-741; Paulino M et al., Mini-Reviews in Medicinal Chemistry 2005, 5: 499-519; Campos RF et al., Rev. Soc. Bras. Med. Trop. 2005, 38(2):142-146; Garcia S et al., Antimicrob. Agents Chemother. 2005, 49(4):1521-1528; Schenone H et al., Rev. Méd. Chile 2003; 131:1089-1090; Marcondes MC et al., Microbes Infect. 2000, 2(4):347-352; Corrales M et al., Antimicrob. Agents Chemother. 2005, 49(4):1556-1560; Urbina JA et al., Int. J. Antimicrob. Agents 2003, 21(1):39-48; Maya JD et al., Biochem. Pharmacol. 2003, 65(6):999-1006; Lockman JW et al., Curr. Med. Chem. 2005, 12(8):945-959].

The use of alkyl phospholipids in protozoal diseases, in particular Leishmaniasis and Chagas disease has also been reported. Miltefosine has been shown to be as high effective in the treatment of Leishmaniasis as currently used amphotericin B. It has been registered as the first oral drug in several countries for cutaneous and visceral leishmaniasis. However, there is still a need to improve therapy schemes and efficacy in the course of Leishmaniasis treatment. There is an increasing danger that due to the long metabolic half-life period of the drug and the long lasting therapy course of 28 days - which may not be completely followed by the patients - development of drug resistance will occur. Alkyl phospholipids have also been shown in preclinical test to be active in acute phase *in vivo* and *in vitro* against Trypanosoma cruzi.

[Literature on use of APL in Leishmaniasis and Chagas disease: Croft SL et al., Mol. Biochem. Parasitol. 2003, 126(2):165-172; Saraiva VB et al., Antimicrob. Agents Chemother. 2002, 46(11):3472-3477; de Castro SL et al., Mini-Reviews in Medicinal Chemistry 2004, 4:141-151; Berman J, Expert Opin. Pharmacother. 2005, 6(8):1381-1388; Bhattacharya SK et al., Clin. Infect. Dis. 2004, 38(2):217-221; Jha TK et al., N. Engl. J. Med. 1999, 341 (24):1795-1800; Jacobs S, N. Engl. J. Med. 2002, 347(22):1737-1738; Sundar S et al., N. Engl. J. Med. 2002, 347(22):1739-1746; Sindermann H et al., Clin. Infect. Dis. 2004, 39(10):1520-1523; Soto J et al., Clin. Infect. Dis. 2004, 38(9):1266-1272; Soto J et al., Clin. Infect. Dis. 2001, 33(7):E57-61; Sundar S et al., Pediatr. Infect. Dis. J. 2003, 22(5):434-438; Croft SL et al., J. Antimicrob. Chemother. 1996, 38(6):1041-1047; Santa-Rita RM et al., Acta Trop. 2000, 75(2):219-228; Sundar S et al., Lancet. 1998, 352 (9143): 1821-1823; Sundar S et al., Ann. Trop. Med. Parasitol. 1999, 93(6):589-597; Sundar S et al., Clin. Infect. Dis. 2000, 31(4):1110-1113; Lux H et al., Mol. Biochem. Parasitol. 2000, 111(1):1-14; US 5,980,915, US 6,521,879, US 6,506,393; US 2003/0216355; US 2004/0242543; Verma NK et al., Antimicrob. Agents Chemother. 2004, 48(8):3010-3015; Walochnik J et al., Antimicrob. Agents Chemother. 2002, 46(3):695-701; Seifert K et al., Antimicrob. Agents Chemother. 2006, 50(1):73-79].

As for protozoal diseases, in particular Leishmaniasis and Chagas disease, combination therapy approaches have only been rarely reported. The efficacy of picroliv in combination with miltefosine was described by Gupta and co-workers (Gupta S et al., Acta Trop. 2005, 94(1):41-47). A potential anti-proliferative synergy of lysophospholipid analogues and ketoconazole against Trypanosoma cruzi was discussed by Santa-Rita and collaborators (Santa-Rita RM et al., J. Antimicrob. Chemother. 2005, 55(5):780-784). Mechanism of action of anti-proliferative lysophospholipid analogues against the protozoan parasite Trypanosoma cruzi were elucidated by Lira et al. who postulated a potentiation of in vitro activity by the sterol biosynthesis inhibitor ketoconazole (Lira R et al., J. Antimicrob. Chemother. 2001, 47(5):537-546). Araujo and co-workers showed that a combination of benznidazole and ketoconazole enhances efficacy of chemotherapy of experimental Chagas disease (Araujo MS et al., J. Antimicrob. Chemother. 2000, 45(6):819-824).

Kanetani/Kanaya F et al. describe the synthesis, physicochemical properties and antimicrobial properties of long chain alkylphosphorylcholines. The authors show that the compounds studied exhibit virtually no antibacterial properties against Escherichia coli and Staphylococcus aureus. However, two of them exhibit an antifungal effect against Aspergillus oryzae (Kanetani/Kanaya F et al., Nippon Kagaku Zasshi 1984, 9:1452-1458). Berger and co-workers studied the influence of dexadecylphosphocholine on tumor regression and virus-infected cells (Berger MR et al., J. Cancer Res. Clin. Oncol. 1993, 119:541-548). Ng et al. investigated the correlation of antifungal activity with fungal phospholipase inhibition using a series of bisquaternary ammonium salts (Ng et al., J. Med. Chem 2006, 49:811-816). Widmer et al. describe the fungicidal activity of hexadecylphosphocholine in a mouse model of cryptococcosis (Widmer F et al., Antimicrob. Agents Chemother. 2006, 50(2):414-421).

The closest prior art EP 0 579 939 A1 describes alkyl phospholipid derivatives of a general formula, processes for the preparation of the derivatives and methods for treating diseases. It is stated that the tested compounds demonstrate anti-tumoral activity. EP 0 579 939 A1 teaches furthermore that pharmaceutical compositions comprising compounds according to the patent are useful to treat protozoal diseases, fungal diseases, autoimmune diseases, skin disorders and bone marrow damage. Particulars of the activities like data or test results are not detailed. It is therefore impossible to make a comparison between the different solutions of EP 0 579 939 A1 and the invention.

However, alkyl phospholipid derivatives known in the prior art and uses thereof do show inherent disadvantages. In particular, standard drug medicamentation (APL and others) against bacterial, fungal, protozoal and/or viral diseases implicate several drawbacks, such as resistance of the microorganisms to be targeted, severe side effects caused by the high toxicity of the compounds to be applied and long courses of treatment.

### Description of the invention

The present invention has the object to provide alkyl phospholipid derivatives which can be employed for the treatment of diseases or pathophysiological conditions in mammals caused by microorganisms, in particular fungi. A further object of the present invention is to provide novel combination therapies of alkyl phospholipid derivatives with suitable known drugs for the treatment of diseases or pathophysiological conditions in mammals caused by fungi.

The object of the present invention has suprisingly been solved in one aspect by providing alkyl phospholipid derivatives according to formula (I) 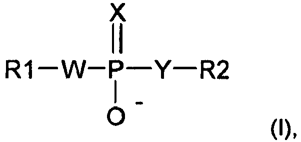 wherein:
W, X, Y independently are selected from the group consisting of: "oxygen atom, sulphur atom";
R1 is "-R5";
R2 is "-R8";
R5 is independently selected from the group consisting of: "substituted or unsubstituted C8-C30alkyl.
R8 is selected from the group consisting of: "substituted or unsubstituted heterocycle", where heterocycle is
   (i) a 5-, 6- or 7-membered saturated, partially unsaturated or aromatic monocyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom,
and where heterocycle if substituted is substituted with at least one radical R12,
which in case of two or more radicals R12 are independently from each other identical, partly identical or different;
R12 is independently from each other selected from the group consisting of:
   "hydrogen atom, substituted or unsubstituted C1-C18alkyl, substituted or
   unsubstituted C3-C8cycloalkyl, substituted or unsubstituted (C1-C12alkyl)ₛ-B-(C1-C12alkyl)ₜ-C-(C1-C12alkyl)ᵤ, substituted or unsubstituted aryl, substituted or
   unsubstituted heteroaryl, substituted or unsubstituted alkoxy, -OH, halogen, -F, - Cl, -Br, -I, =O, -C(O)O-(C1-C12alkyl), -C(O)O-(C3-C8cycloalkyl), -C(O)O-aryl, - C(O)O-heteroaryl, -C(O)O-heterocyclyl, -C(O)-(C1-C12alkyl), -C(O)-(C3-C8cycloalkyl), -C(O)-aryl, -C(O)-heteroaryl, -C(O)-heterocyclyl";
B, C are independently from each other selected from the group consisting of "oxygen atom; sulphur atom; S(O₂)";
s, t, u independently from each other are 0 or 1;
that can be used for the manufacture of a medicament for the treatment or prophylaxis of diseases and/or pathophysiological conditions in mammals that are caused by fungi.

In another aspect, the object of the invention has surprisingly been solved by providing novel alkyl phospholipid derivatives selected from the group consisting of:

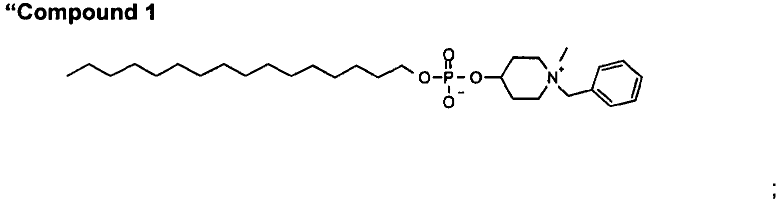

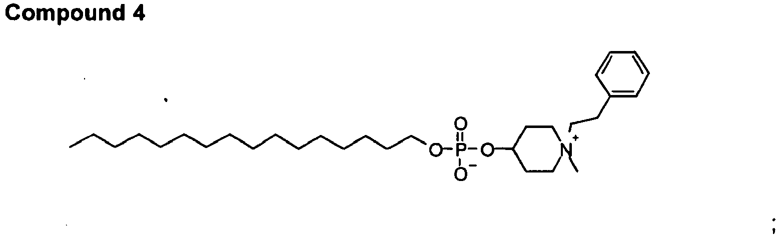

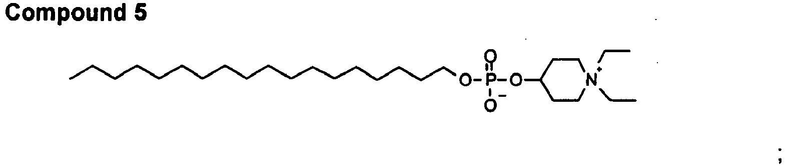

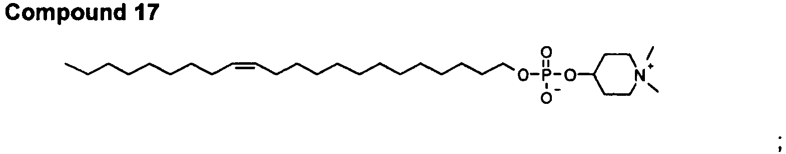

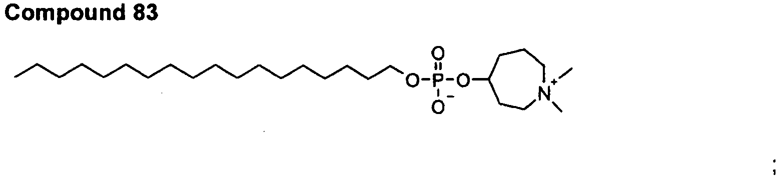

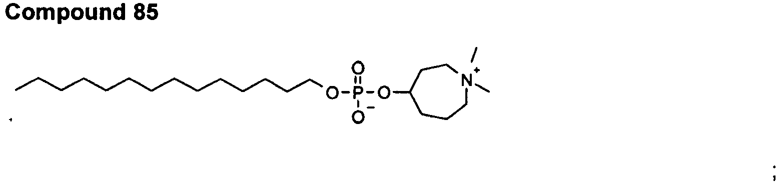

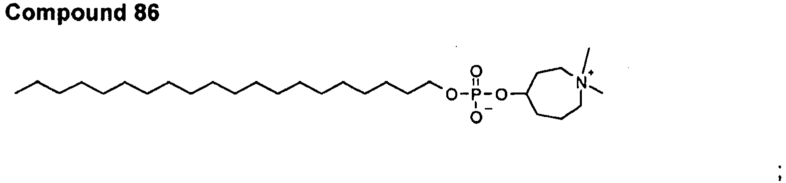

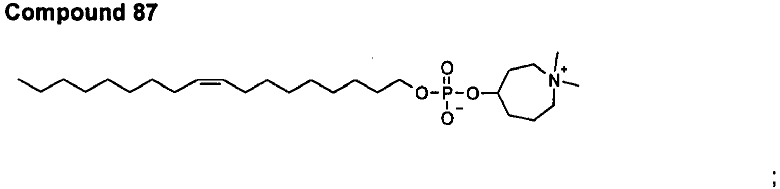

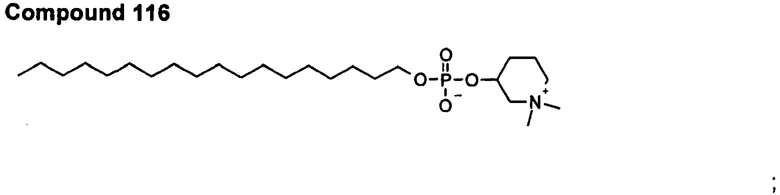

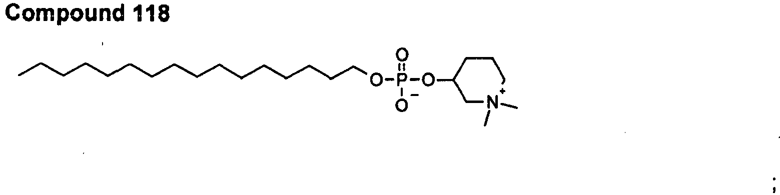

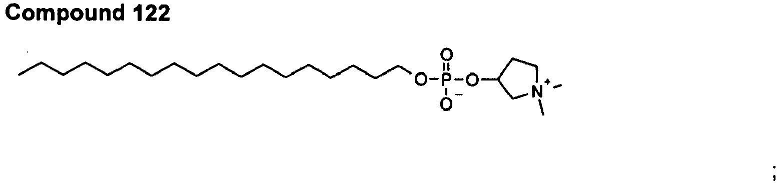

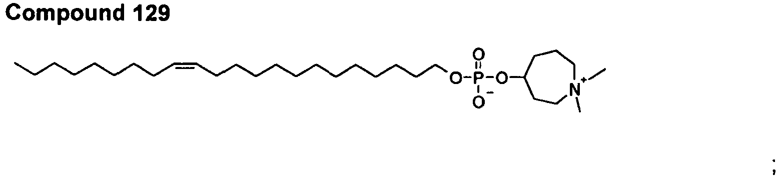

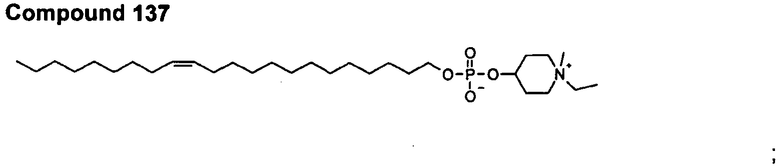

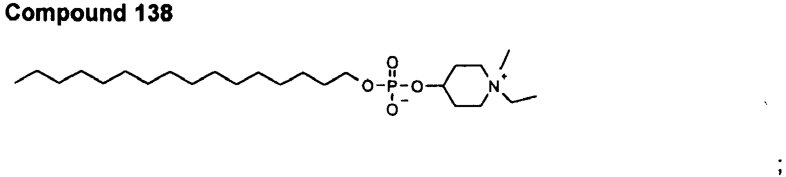

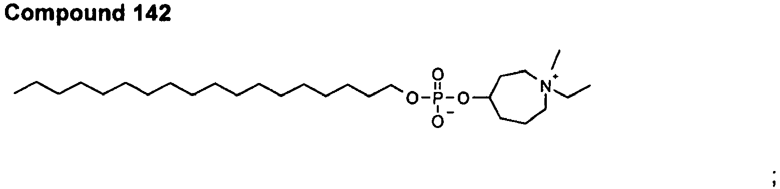

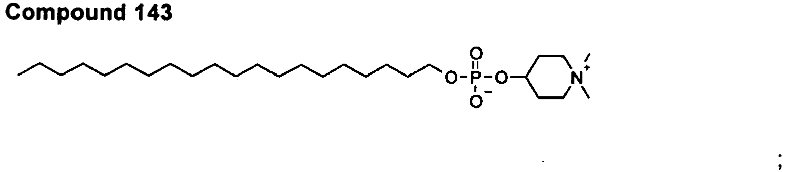

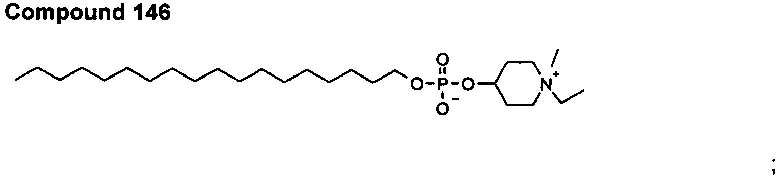

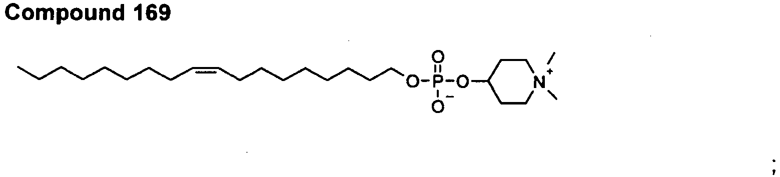

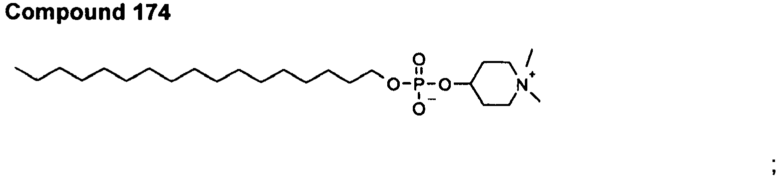

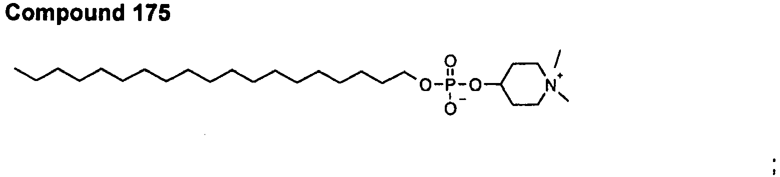

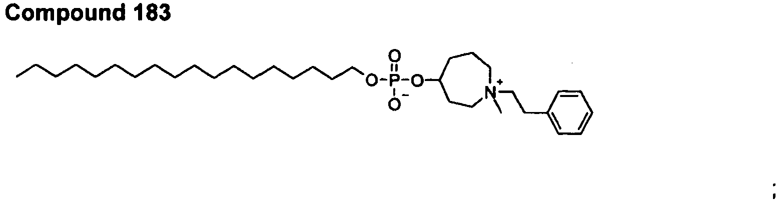

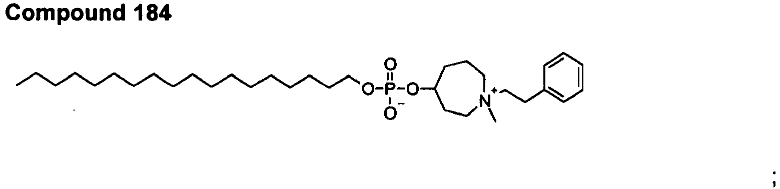

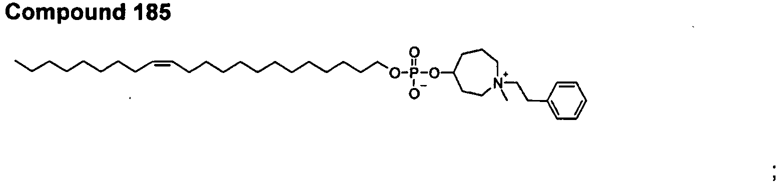

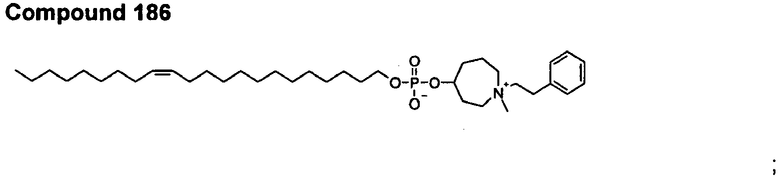

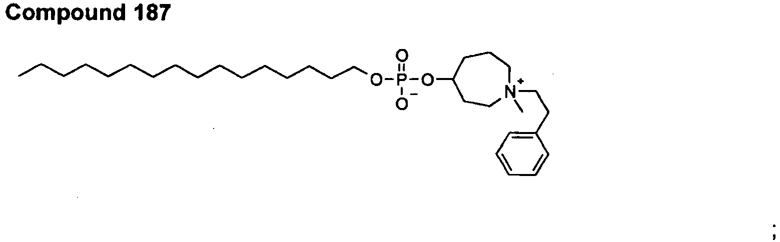

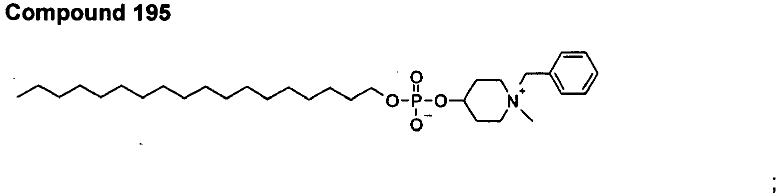

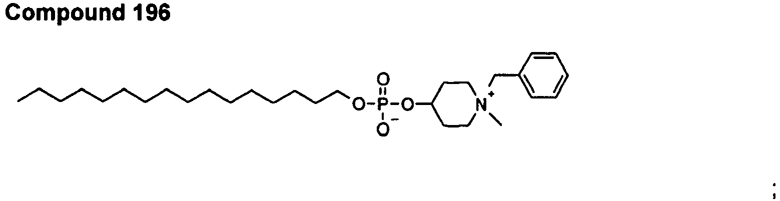

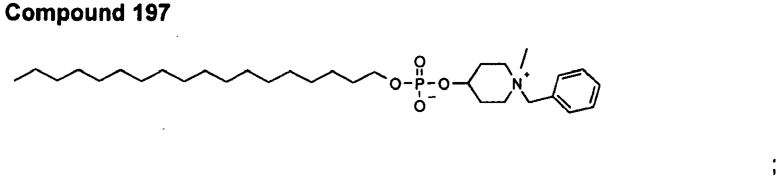

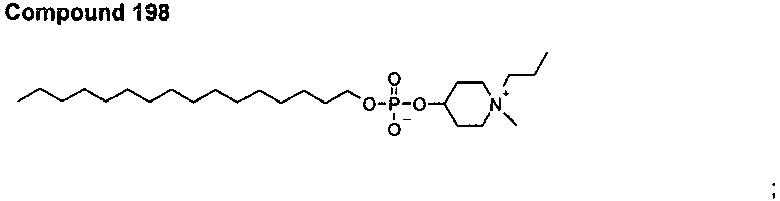

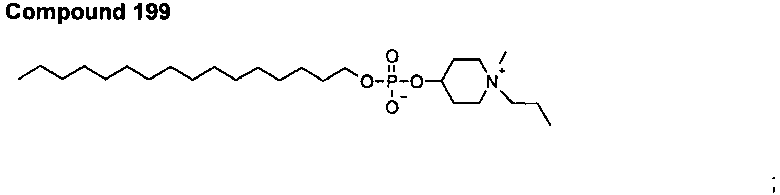

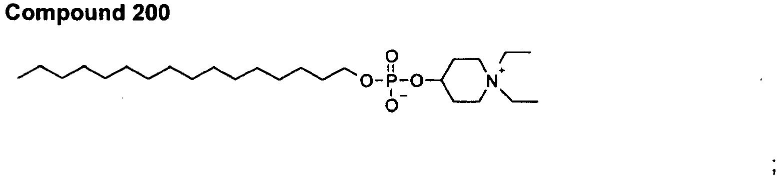

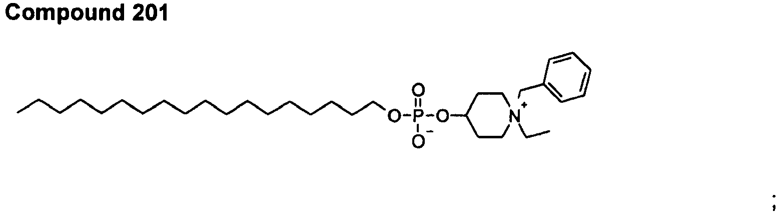

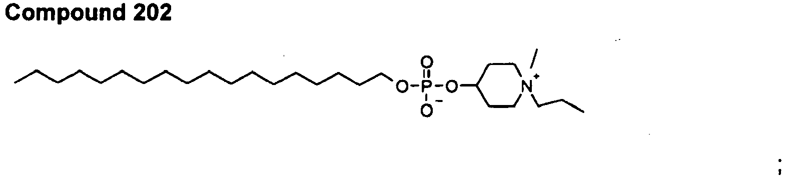

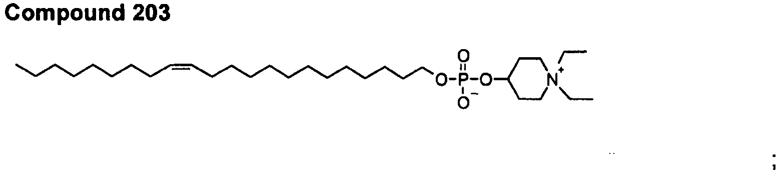

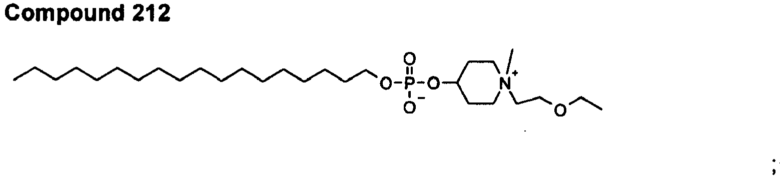

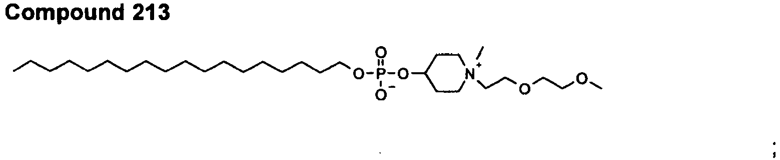

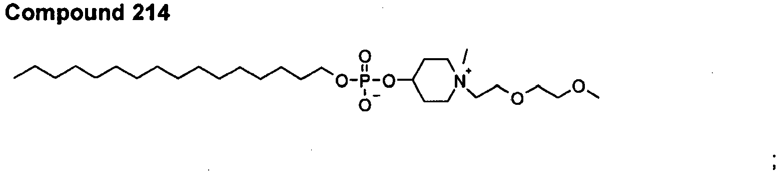

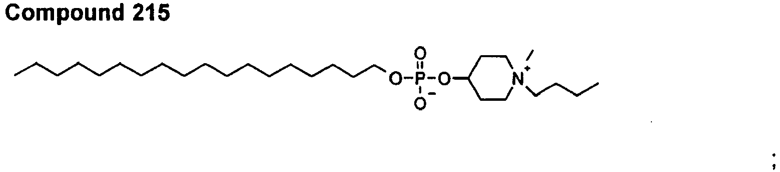

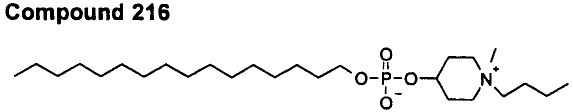

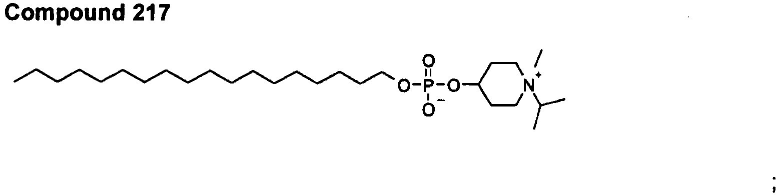

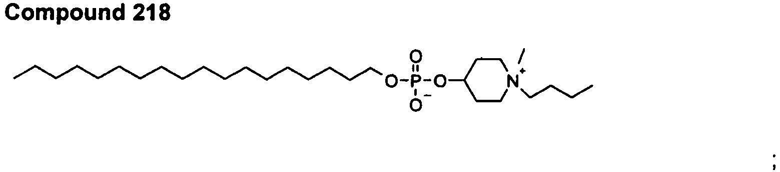

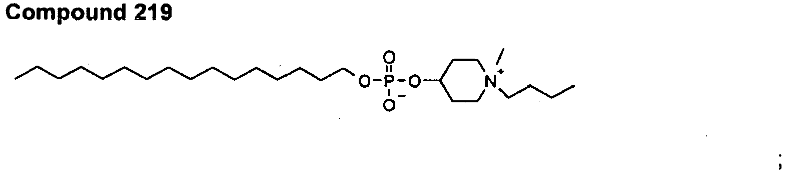

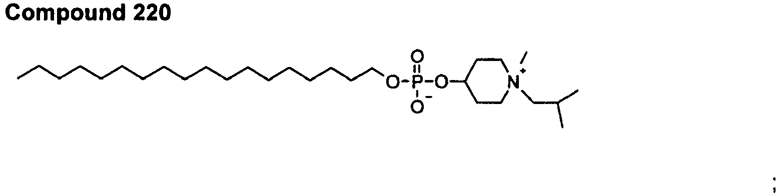

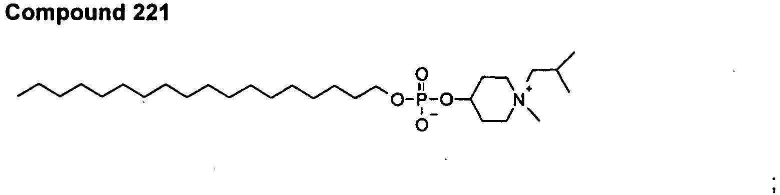

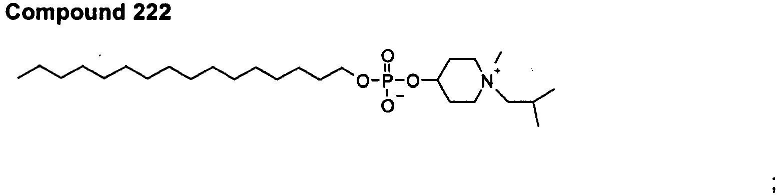

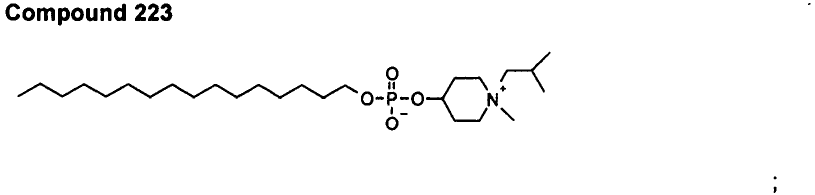

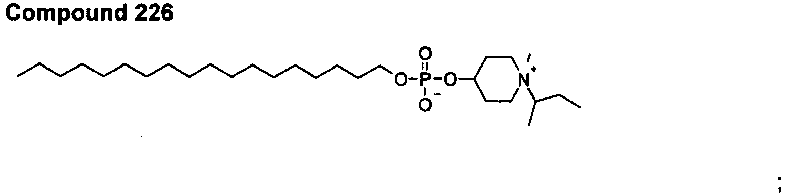

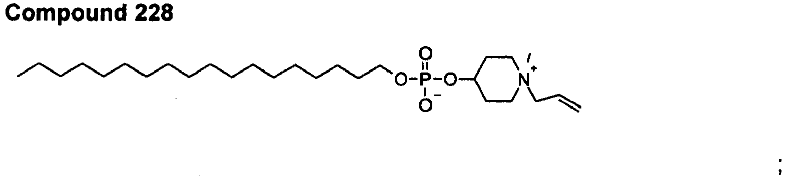

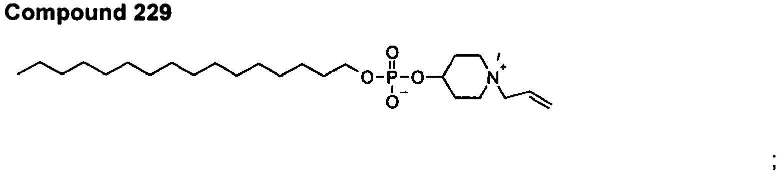

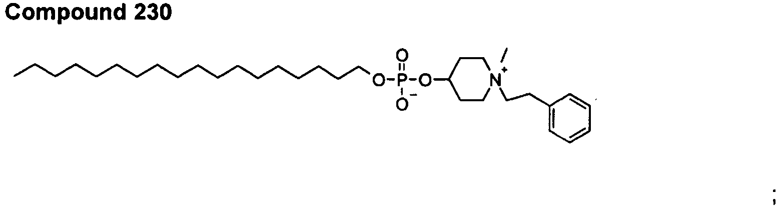

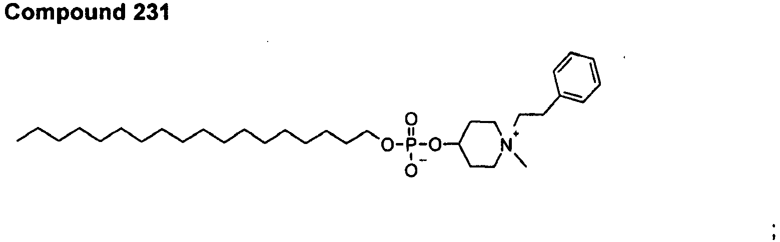

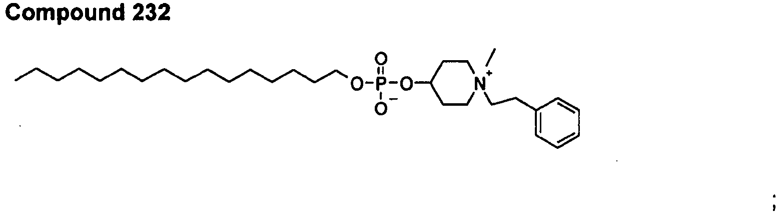

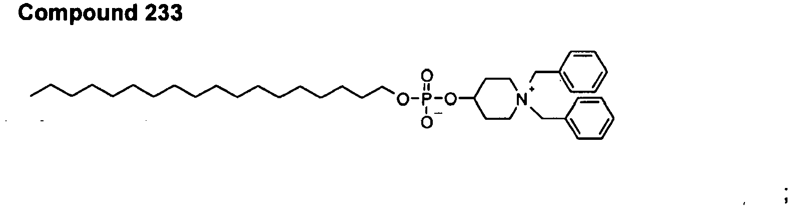

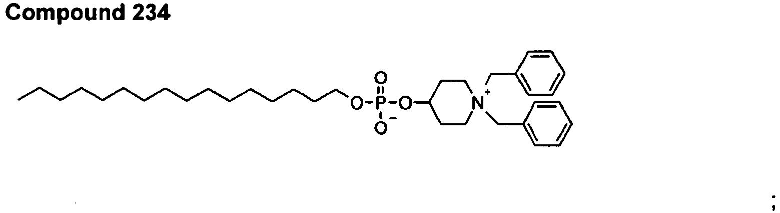

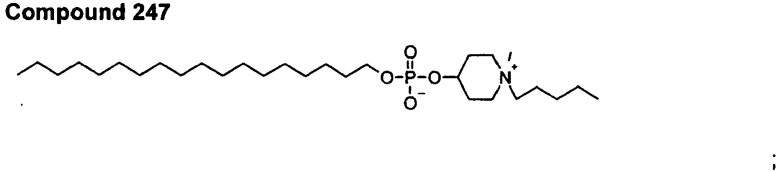

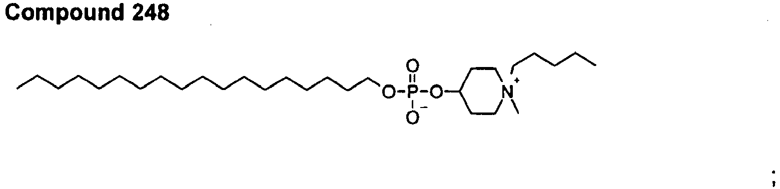

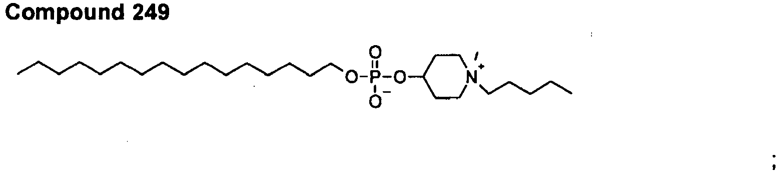

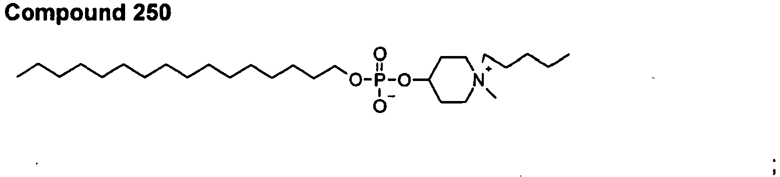

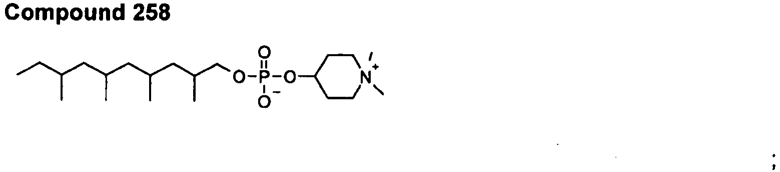

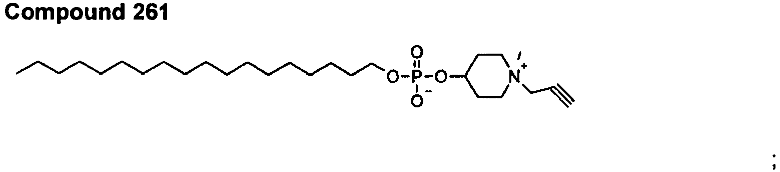

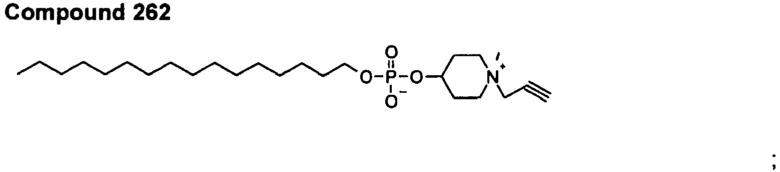

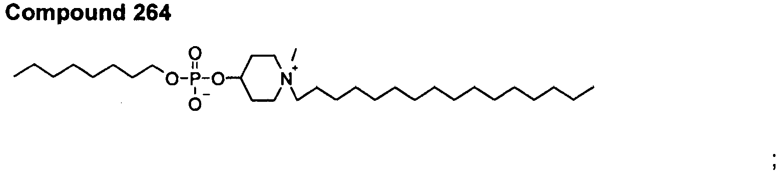

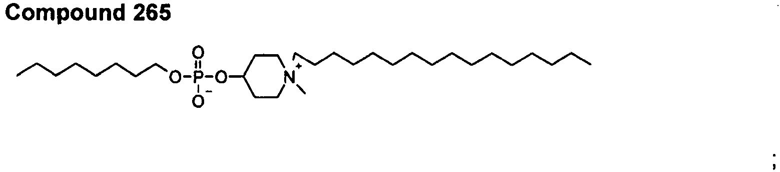

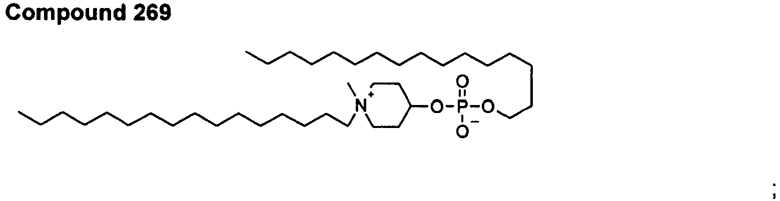

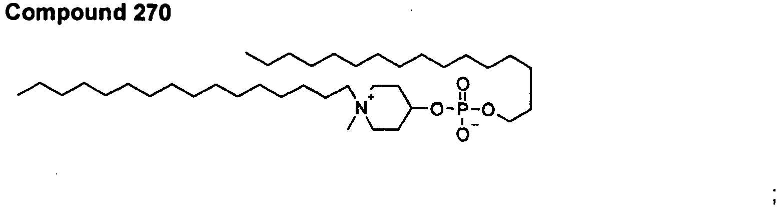

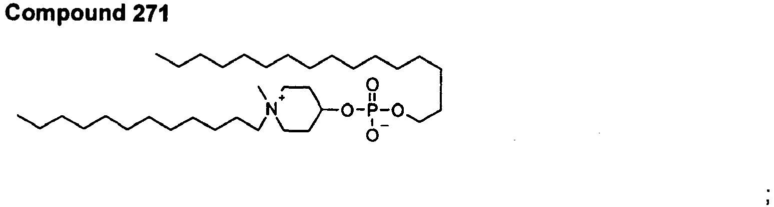

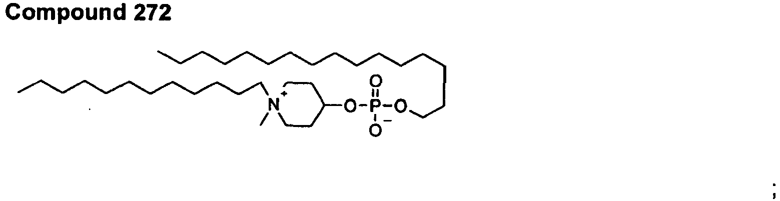

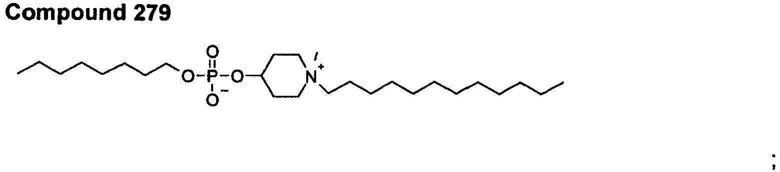

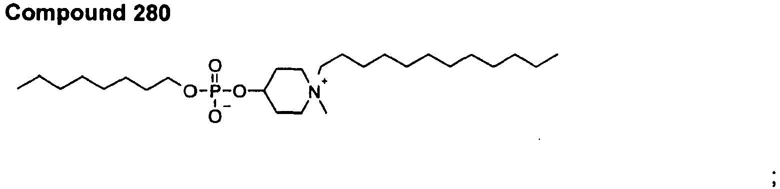

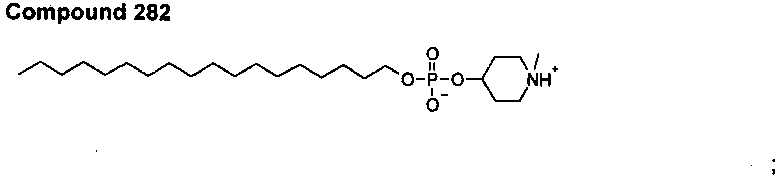

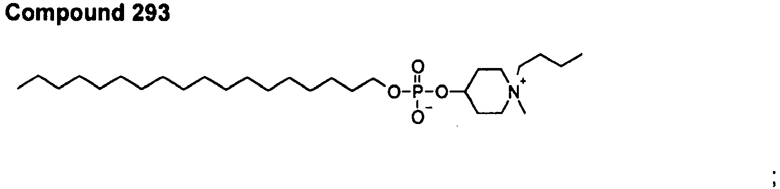

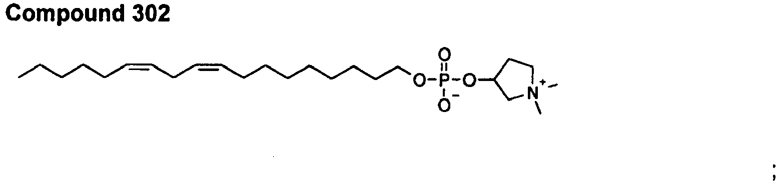

These alkyl phospholipid derivatives (Compounds 1 to 302) can be used for the manufacture of a medicament for the treatment or prophylaxis of diseases and/or pathophysiological conditions in mammals that are caused by microorganisms.

For the avoidance of doubt, if chemical name and chemical structure of the above illustrated compounds do not correspond by mistake, the chemical structure is regarded to unambigously define the compound.

In a preferred embodiment, all the above generically or explicitly disclosed alkyl phospholipid derivatives, including preferred subsets of formula (I) and Compounds 1 to 302, hereinafter referred to as compounds of the (present) invention, can be used for the manufacture of a medicament for the treatment or prophylaxis of diseases and/or pathophysiological conditions in mammals that are caused by microorganisms, where the microorganism is selected from the group consisting of "fungus".

The terms indicated for explanation of the above compounds of the invention always, unless indicated otherwise in the description or in the claims, have the following meanings:
The term "unsubstituted" means that the corresponding radical, group or moiety has no substituents.
The term "substituted" means that the corresponding radical, group or moiety has one or more substituents. Where a radical has a plurality of substituents, and a selection of various substituents is specified, the substituents are selected independently of one another and do not need to be identical.
The term "alkyl" includes for the purposes of this invention acyclic and cyclic saturated, unsaturated or partially unsaturated hydrocarbons which may be straight-chain or branched or cyclic or also include cyclic hydrocarbons as part of a straight-chained or branched hydrocarbon system and which may contain one or more double and/or triple bonds. In this connection, C1-C6alkyl, C1-C12alkyl, C1-C18alkyl, C8-C30alkyl and the like refer to above definition having 1 to 6, 1 to 12, 1 to 18, 8 to 30, respectively, and the like carbon atoms.

Examples of suitable alkyl radicals are methyl, ethyl, n-propyl, isopropyl, n-butyl, isobutyl, sec-butyl, tert-butyl, n-pentyl, iso-pentyl, neo-pentyl, tert-pentyl, 2- or 3-methylpentyl, n-hexyl, 2-hexyl, isohexyl, n-heptyl, n-octyl, n-nonyl, n-decyl, n-undecyl, n-dodecyl, n-tetradecyl, n-hexadecyl, n-octadecyl, n-icosanyl, n-docosanyl, ethylenyl (vinyl), propenyl (-CH₂CH=CH₂; -CH=CH-CH₃, -C(=CH₂)-CH₃), butenyl, pentenyl, hexenyl, heptenyl, octenyl, octadienyl, octadecenyl, octadec-9-enyl, icosenyl, icos-11-enyl, (Z)-icos-11-enyl, docosnyl, docos-13-enyl, (Z)-docos-13-enyl, ethynyl, propynyl (-CH₂-C≡CH, -C≡C-CH₃), butynyl, pentynyl, hexynyl, heptynyl, octynyl, cyclopropyl, cyclobutyl, cyclopentyl, cyclohexyl, cycloheptyl, cyclooctyl, cyclodecyl, cyclohexenyl, cyclopentenyl, cyclooctadienyl.

The term "C3-C8cycloalkyl" stands for a saturated or partially unsaturated nonaromatic cyclic hydrocarbon group/radical, containing 3 to 8 carbon atoms that may optionally be linked via an alkyl group where the alkyl has the meaning as defined herein, preferably a (C3-C8)-cycloalkyl-(C1-C4)-alkyl radical. Such "C3-C8cycloalkly" radicals can be linked via any ring member.

Examples of suitable cycloalkyl radicals are cyclopropyl, cyclobutyl, cyclopentyl, cyclohexyl, cycloheptyl, cyclooctyl, cyclohexenyl, cyclopentenyl, cyclooctadienyl, cyclopropylmethyl, cyclohexylmethyl, cyclopentylethyl, cyclohexenylethyl.

The term "aryl" refers to aromatic hydrocarbon systems having 3 to 14, preferably 5 to 14, carbon atoms. The term "aryl" also includes systems in which the aromatic cycle is part of a bi- or polycyclic saturated, partially unsaturated and/or aromatic system, such as were the aromatic cycle is fused to an "aryl", "C3-C8cycloalkyl", "heteroaryl" or "heterocyclyl" group as defined herein via any desired and possible ring member of the aryl radical. Such "aryl" radicals can be linked via any ring member. Examples of "aryl" are inter alia phenyl, biphenyl, naphthyl and anthracenyl, but also indanyl, indenyl, or 1,2,3,4-tetrahydronaphthyl. The terms "aryl" is also intended to comprise radicals in which the aryl radical is linked via a C1-C18alkyl group, preferably C1-C12alkyl group. Most preferred are aryl-C1-C4alkyl radicals, preferably benzyl or phenylethyl radicals.

The term "heteroaryl" refers to a 5-, 6- or 7-membered cyclic aromatic radical which comprises at least 1, where appropriate also 2, 3, 4 or 5 heteroatoms, preferably nitrogen, oxygen and/or sulfur, where the heteroatoms are identical or different. The number of nitrogen atoms is preferably 0, 1, 2, or 3, and that of the oxygen and sulfur atoms is independently 0 or 1. The term "heteroaryl" also includes systems in which the aromatic cycle is part of a bi- or polycyclic saturated, partially unsaturated and/or aromatic system, such as were the aromatic cycle is fused to an "aryl", "C3-C8cycloalkyl", "heteroaryl" or "heterocyclyl" group as defined herein via any desired and possible ring member of the heteroaryl radical. Such "heteroaryl" radicals can be linked via any ring member. Examples of "heteroaryl" include pyrrolyl, thienyl, furyl, imidazolyl, thiazolyl, isothiazolyl, oxazolyl, oxadiazolyl, isoxazolyl, pyrazolyl, pyridinyl, pyrimidinyl, pyrazinyl, indolyl, quinolinyl, isoquinolinyl, imidazolyl, triazolyl, tetrazolyl, pyridazinyl, phthalazinyl, indazolyl, indolizinyl, quinoxalinyl, quinazolinyl, pteridinyl, carbazolyl, phenazinyl, phenoxazinyl, phenothiazinyl, acridinyl. The terms "heteroaryl" is also intended to comprise radicals in which the heteroaryl radical is linked via a C1-C18alkyl group, preferably C1-C12alkyl group. Most preferred are heteroaryl-C1-C4alkyl radicals, preferably indolyl-(C₁-C₄)-alkyl radicals, such as 1H-indole-3-yl-methyl or 2-(1 H-indole-3-yl)-ethyl.

The term "heterocyclyl" refers to a mono- or polycyclic system of 3 to 20, preferably 5 or 6 to 14 ring atoms comprising carbon atoms and 1, 2, 3, 4, or 5 heteroatoms, in particular nitrogen, oxygen and/or sulfur which are identical or different. The cyclic system may be saturated, mono- or polyunsaturated but may not be aromatic. In the case of a cyclic system consisting of at least two rings the rings may be fused or spiro-or otherwise connected. Such "heterocyclyl" radicals can be linked via any ring member. The term "heterocyclyl" also includes systems in which the heterocycle is part of a bi- or polycyclic saturated, partially unsaturated and/or aromatic system, such as where the heterocycle is fused to an "aryl", "C3-C8cycloalkyl", "heteroaryl" or "heterocyclyl" group as defined herein via any desired and possible ring member of the heterocycyl radical. Examples of "heterocyclyl" include pyrrolidinyl, thiapyrrolidinyl, piperidinyl, piperazinyl, oxapiperazinyl, oxapiperidinyl, oxadiazolyl, tetrahydrofuryl, imidazolidinyl, thiazolidinyl, tetrahydropyranyl, morpholinyl, tetrahydrothiophenyl, dihydropyranyl. The term "heterocyclyl" is also intended to comprise radicals in which the heterocyclyl group is linked via an C1-C18alkyl group, preferably C1-C12alkyl group. Most preferred are hetercyclyl-C1-C4alkyl radicals.

The term "alkoxy" refers to radicals in which a "alkyl", "C3-C8cycloalkyl", "aryl", "heteroaryl", and/or "heterocyclyl" group is linked via an oxygen atom (-O-group), where "alkyl", "C3-C8cycloalkyl", "aryl", "heteroaryl" and "heterocyclyl" have the meanings as defined herein.

The term "halogen", "halogen atom" or "halogen substituent" (Hal-) refers to one, where appropriate, a plurality of fluorine (F, fluoro), bromine (Br, bromo), chlorine (Cl, chloro), or iodine (I, iodo) atoms. The designations "dihalogen", "trihalogen" and "perhalogen" refer respectively to two, three and four substituents, where each substituent can be selected independently from the group consisting of fluorine, chlorine, bromine and iodine. "Halogen" preferably means a fluorine, chlorine or bromine atom.

The term "substituted" in connection with "alkyl", in particular C1-C6alkyl, C1-C12alkyl, C1-C18alkyl, C8-C30alkyl, "C3-C8cycloalkyl", "aryl", "heteroaryl", "heterocyclyl", "heterocycle" and "alkoxy", unless not explicitly otherwise defined in the description or in the claims, means the independent replacement/substitution of one ore more hydrogen atoms by a substituent independently selected from the group consisting of "-NO₂, -NO, -CN, -OH, halogen, F, Cl, Br, I, -NH₂, -NHNH₂, -N₃, -SH, - SO₃H, -COOH, -CONH₂, -CHO, -CHNOH, -NH-C(NH₂)=NH, -C(NH₂)=NH, -CF₃, - OCF₃, -OSO₃H, -OP(O)(OH)₂, -P(O)(OH)₂, -NH-(C1-C12alkyl), -N(C1-C12alkyl)₂, - NH-aryl, -N(aryl)₂, -N-A5A6, -NH-C(NH₂)=N-(C1-C12alkyl),-C(NH₂)=N-(C1-C12alkyl), -NH-C(NH₂)=N-aryl, -C(NH₂)=N-aryl, -OP(O)(O-(C1-C12alkyl))₂, - OP(O)(O-aryl)₂, -OP(O)(O-heteroaryl)₂, -OP(O)(O-A7)(O-A8), -O-aryl, -O-heteroaryl, -O-(C3-C8cycloalkyl), -O-heterocyclyl, -S-(C1-C12alkyl), -S-aryl, -S-heteroaryl, -S-(C3-C8cycloalkyl), -S-heterocyclyl, -SO-(C1-C12alkyl), -SO-aryl, - SO-heteroaryl, -SO-(C3-C8cycloalkyl), -SO-heterocyclyl, -SO₂-(C1-C12alkyl), - SO₂-aryl, -SO₂-heteroaryl, -SO₂-(C3-C8cycloalkyl), -SO₂-heterocyclyl, -SO₃-(C1-C12alkyl), -SO₃-aryl, -SO₃-heteroaryl, -SO₃-(C3-C8cycloalkyl), -SO₃-heterocyclyl, - OC(O)-(C1-C12alkyl), -OC(O)-(C3-C8cycloalkyl), -OC(O)-aryl, -OC(O)-heterocyclyl, -OC(O)-heteroaryl, -C(O)-(C1-C12alkyl), -C(O)-(C3-C8cycloalkyl), -C(O)-aryl, - C(O)-heterocyclyl, -C(O)-heteroaryl, -C(O)O-(C1-C12alkyl), -C(O)O-(C3-C8cycloalkyl), -C(O)O-aryl, -C(O)O-heterocyclyl, -C(O)O-heteroaryl, -OC(O)NH-(C1-C12alkyl), -OC(O)NH-(C3-C8cycloalkyl), -OC(O)NH-aryl, -OC(O)NH-heteroaryl, -OC(O)NH-heterocyclyl, -OC(O)N-A1A2, -OC(O)N-heterocyclyl, -C(O)NH-(C1-C12alkyl), -C(O)NH-(C3-C8cycloalkyl), -C(O)NH-aryl, -C(O)NH-heteroaryl, - C(O)NH-heterocyclyl, -C(O)N-A3A4, -C(O)N-heterocyclyl, C1-C12alkyl, C3-C8cycloalkyl, aryl, heteroaryl, heterocyclyl" where A1, A2, A3, A4, A5, A6, A7, A8, A9, A10 independently are selected from the group consisting of "C1-C12alkyl, C3-C8cycloalkyl, aryl, heteroaryl, heterocyclyl".

For the purpose of the present invention, the terms indicated for explanation of the above group of microorganisms always, unless indicated otherwise in the description or in the claims, have the following meanings:
The term "fungus" or "fungi" is intended to comprise all known single-cell/unicellular and/or multi-cellular members of the kingdom of fungi, such as Chytridiomycota, Zygomycota, Glomeromycota, Ascomycota and/or Basidiomycota, as well as in addition Myxomycota, Oomycota and/or Hypochytriomycota.

Examples of such fungi are Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. and/or Wangiella spp.

Further examples of such fungi are Absidia corymbifera, Acremonium falciforme, Acremonium recifei, Aspergillus flavus, Aspergillus fumigatus, Aspergillus glaucus, Aspergillus nidulans, Aspergillus niger, Aspergillus terreus, Bipolaris australiensis, Candida albicans, Candida glabrata, Candida krusei, Candida parapsilosis, Candida tropicalis, Cladophialophora bantiana, Cladophialophora carrionii, Coccidioides immitis, Coniothyrium fuckelii, Cryptococcus gattii, Cryptococcus neoformans, Cunninghamella bertholletitae, Epidermophyton floccosum, Exophiala jeanselmei, Exophiala spinifera, Fonsecaea compacta, Fonsecaea pedrosoi, Fusarium oxysporum, Fusarium solani, Histoplasma capsulatum capsulatum, Histoplasma capsulatum duboisii, Lacazia loboi, Lasiodiplodia theobromae, Leptosphaeria senegalensis, Madurella grisea, Madurella mycetomatis, Microsporum audouinii, Microsporum canis, Microsporum gypseum, Neotestudina rosatii, Ochroconis gallopava, Onychocola Canadensis, Paecilomyces lilacinus, Paracoccidioides brasiliensis, Phialophora parasitica, Phialophora repens, Phialophora verrucosa, Pseudallesheria boydii, Pyrenochaeta romeroi, Rhizomucor pusillus, Rhizopus arrhizus, Rhizopus oryzae, Scedosporium apiospermum, Scedosporium inflatum, Scedosporium prolificans, Scopulariopsis brevicaulis, Scytalidium dimidiatum, Sporothrix schenckii, Trichophyton mentagrophytes, Trichophyton rubrum, Trichophyton schoenleinii, Trichophyton tonsurans and/or Wangiella dermatitidis.

For the purpose of the present invention, the term "treatment" is also intended to include prophylactic treatment or alleviation.

In a preferred embodiment, the compounds of the invention can be used for the manufacture of a medicament for the treatment or prophylaxis of diseases and/or pathophysiological conditions in mammals that are caused by microorganisms, where the microorganism is a fungus and selected from the group consisting of "Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. and/or Wangiella spp." More preferably, the fungus is selected from the group consisting of "Absidia spp., Aspergillus spp., Bipolaris spp., Candida spp., Cryptococcus spp., Cunninghamella spp., Exophiala spp., Fusarium spp., Paecilomyces spp., Rhizopus spp. and/or Scedosporium spp." Most preferably, the fungus is selected from the group consisting of "Absidia corymbifera, Aspergillus flavus, Aspergillus fumigatus, Aspergillus terreus, Bipolaris australiensis, Candida albicans, Candida glabrata, Candida krusei, Candida parapsilosis, Candida tropicalis, Cryptococcus gattii, Cryptococcus neoformans, Cunninghamella bertholletitae, Exophiala jeanselmei, Exophiala spinifera, Fusarium solani, Paecilomyces lilacinus, Rhizopus oryzae, Scedosporium apiospermum and/or Scedosporium prolificans. Even more preferably, the alkyl phospholipid derivative is selected from the group consisting of: **"Compound 1, 4** and/or **compound 5".**

All stereoisomers of the compounds of the invention are contemplated, either in a mixture or in pure or substantially pure form. The compounds of the invention can have asymmetric centers at any of the carbon atoms. Consequently, they can exist in the form of their racemates, in the form of the pure enantiomers and/or diastereomers or in the form of mixtures of these enantiomers and/or diastereomers. The mixtures may have any desired mixing ratio of the stereoisomers.

Thus, for example, the compounds of the invention which have one or more centers of chirality and which occur as racemates or as diastereomer mixtures can be fractionated by methods known per se into their optical pure isomers, i.e. enantiomers or diastereomers. The separation of the compounds of the invention can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

The compounds of the invention may be present in the form of their double bond isomers as "pure" E or Z isomers, or in the form of mixtures of these double bond isomers.

Where possible, the compounds of the invention may be in the form of the tautomers.

The compounds of the invention can, if they have a sufficiently basic group such as, for example, a secondary or tertiary amine, be converted with inorganic and organic acids into salts. The pharmaceutically acceptable salts of the compounds of the invention are preferably formed with hydrochloric acid, hydrobromic acid, iodic acid, sulfuric acid, phosphoric acid, methanesulfonic acid, p-toluenesulfonic acid, carbonic acid, formic acid, acetic acid, sulfoacetic acid, trifluoroacetic acid, oxalic acid, malonic acid, maleic acid, succinic acid, tartaric acid, racemic acid, malic acid, embonic acid, mandelic acid, fumaric acid, lactic acid, citric acid, taurocholic acid, glutaric acid, stearic acid, glutamic acid or aspartic acid. The salts which are formed are, inter alia, hydrochlorides, chlorided, hydrobromides, bromides, iodides, sulfates, phosphates, methanesulfonates, tosylates, carbonates, bicarbonates, formates, acetates, sulfoacetates, triflates, oxalates, malonates, maleates, succinates, tartrates, malates, embonates, mandelates, fumarates, lactates, citrates, glutarates, stearates, aspartates and glutamates. The stoichiometry of the salts formed from the compounds of the invention may moreover be an integral or non-integral multiple of one.

The compounds of the invention can, if they contain a sufficiently acidic group such as, for example, the carboxy, sulfonic acid, phosphoric acid or a phenolic group, be converted with inorganic and organic bases into their physiologically tolerated salts. Examples of suitable inorganic bases are ammonium, sodium hydroxide, potassium hydroxide, calcium hydroxide, and of organic bases are ethanolamine, diethanolamine, triethanolamine, ethylenediamine, t-butylamine, t-octylamine, dehydroabietylamine, cyclohexylamine, dibenzylethylene-diamine and lysine. The stoichiometry of the salts formed from the compounds of the invention can moreover be an integral or non-integral multiple of one.

It is likewise possible for the compounds of the invention to be in the form of their solvates and, in particular, hydrates which can be obtained for example by crystallization from a solvent or from aqueous solution. It is moreover possible for one, two, three or any number of solvate or water molecules to combine with the compounds of the invention to give solvates and hydrates.

In a preferred embodiment, the compounds of the invention are available in the form of their hydrates, with any number of water molecules combined/complexed to them, including integer and non-integer ratios, such as 1:0,5; 1:1; 1:1,5; 1:2; 1:2,5; 1:3; 1:3,5; 1:4 etc.

It is known that chemical substances form solids which exist in different order states which are referred to as polymorphic forms or modifications. The various modifications of a polymorphic substance may differ greatly in their physical properties. The compounds of the invention can exist in various polymorphic forms and certain modifications may moreover be metastable. All these polymorphic forms of the compounds are to be regarded as belonging to the invention.

The compounds of the invention are suitable for the treatment or prophylaxis of diseases and/or pathophysiological conditions in mammals that are caused by microorganisms, in particular where the microorganism is selected from the group consisting of "fungus".

For the purpose of the present invention, all known diseases and/or pathophysiological conditions in mammals are intended to be comprised that are caused by fungi.

Examples of such fungal diseases and/or pathophysiological conditions are aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium and/or zygomycosis as well as their different forms and subforms.

In a preferred embodiment, the compounds of the invention can be used for the manufacture of a medicament for the treatment or prophylaxis of diseases and/or pathophysiological conditions in mammals that are caused by microorganisms, where the disease and/or pathophysiological condition is selected from the group consisting of "aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium, zygomycosis".

As illustrated supra, the compounds of the invention are useful for the treatment or prophylaxis of diseases and/or pathophysiological conditions in mammals as defined herein. They can be administered to various mammalian species, including human.

For the purpose of the present invention, all mammalian species are regarded as being comprised. In a preferred embodiment, such mammals are selected from the group consisting of "human, domestic animals, cattle, livestock, pets, cow, sheep, pig, goat, horse, pony, donkey, hinny, mule, hare, rabbit, cat, dog, guinea pig, hamster, rat, mouse". More preferably, such mammals are humans.

In a further aspect of the present invention, the compounds of the invention are used in combination with at least one additional pharmacologically active substance.

Depending on the purpose of the combined use, such additional pharmacologically active substance may be other alkyl phospholipid derivatives (the compounds of the invention and/or known alkyl phospholipid derivatives, such as perifosine and/or other "suitable therapeutic agents" useful in the treatment and/or prophylaxis of the aforementioned diseases and/or pathophysiological conditions. Selection and combination of the additional pharmacologically active substance(s) can be easily performed by the skilled artisan on the basis of his expert knowledge and depending on the purpose of the combined use and diseases and/or pathophysiological conditions targeted.

The above mentioned "suitable therapeutic agents" include benznidazole (N-Benzyl-2-nitroimidazol-1-yl-acetamide; CAS Registry Number: 22994-85-0); nifurtimox [3-Methyl-4-(5-nitrofurfuryl-idenamino)tetrahydro-1,4-thiazine-1,1-dioxide; CAS Registry Number: 23256-30-6]; amphotericin B [(1R, 3S, 5R, 6R, 9R, 11 R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1]nonatriaconta-19, 21, 23, 25, 27, 29, 31-heptaene-36-carboxylic acid; CAS Registry Number: 1397-89-3]; liposomal amphotericin B (AmBisome™; Gilead Sciences, Inc., Astellas Pharma US, Inc.), sitamaquine (N,N-diethyl-N'-(6-methoxy-4-methyl-8-quinolinyl)-1,6-Hexanediamine; CAS Registry Number: 57695-04-2) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine; CAS Registry Number: 7542-37-2] and preferably, a "suitable therapeutic agents" is selected of the group consisting of these agents.

The above other therapeutic agents, when employed in combination with the compounds of the present invention, may be used, for example, in those amounts indicated in the Physicians' Desk Reference (PDR) or as otherwise determined by one of ordinary skill in the art.

In a preferred embodiment, the compounds of the invention are used for the treatment and/or prophylaxis of the aforementioned diseases and/or pathophysiological conditions in the form of a medicament, where such medicament comprises at least one additional pharmacologically active substance.

In another preferred embodiment, the compounds of the invention are used for the treatment and/or prophylaxis of the aforementioned diseases and/or pathophysiological conditions in the form of a medicament, where the medicament is applied before and/or during and/or after treatment with at least one additional pharmacologically active substance.

In a preferred embodiment, the compounds of the invention are used for the treatment and/or prophylaxis of the aforementioned diseases and/or pathophysiological conditions in the form of a medicament, where such medicament comprises at least one compound of the invention and at least one additional pharmacologically active substance selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B, sitamaquine (N,N-diethyl-N'-(6-methoxy-4-methyl-8-quinolinyl)-1,6-Hexanediamine; CAS Registry Number: 57695-04-2) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

In another preferred embodiment, the compounds of the invention are used for the treatment and/or prophylaxis of the aforementioned diseases and/or pathophysiological conditions in the form of a medicament, where the medicament comprising at least one compound of the invention is applied before and/or during and/or after treatment with at least one additional pharmacologically active substance selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B, sitamaquine (N,N-diethyl-N'-(6-methoxy-4-methyl-8-quinolinyl)-1,6-Hexanediamine; CAS Registry Number: 57695-04-2) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

In yet a further aspect, the object of the invention has surprisingly been solved by using alkyl phospholipid derivatives selected from the group consisting of "perifosine (octadecyl-1,1-dimethyl-piperidino-4-yl-phosphate)" in the form of a medicament for the treatment or prophylaxis of the aforementioned diseases and/or pathophysiological conditions in mammals that are caused by microorganisms as variously illustrated herein, where such medicament comprises at least one additional pharmacologically active substance selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-manno-pyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B, sitamaquine (N,N-diethyl-N'-(6-methoxy-4-methyl-8-quinolinyl)-1,6-Hexanediamine; CAS Registry Number: 57695-04-2) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]" and an alkyl phospholipid derivative selected from the group consisting of "perifosine (octadecyl-1,1-dimethyl-piperidino-4-yl-phosphate)".

In yet a further aspect, the object of the invention has surprisingly been solved by using alkyl phospholipid derivatives selected from the group consisting of "perifosine (octadecyl-1,1-dimethyl-piperidino-4-yl-phosphate)" in the form of a medicament for the treatment and/or prophylaxis of the aforementioned diseases and/or pathophysiological conditions in mammals that are caused by microorganisms as variously illustrated herein, where the medicament is applied before and/or during and/or after treatment with at least one additional pharmacologically active substance selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-manno-pyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B, sitamaquine (N,N-diethyl-N'-(6-methoxy-4-methyl-8-quinolinyl)-1,6-Hexanediamine; CAS Registry Number: 57695-04-2) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

In a preferred embodiment, the compounds of the invention or alkyl phospholipid derivatives selected from the group consisting of "perifosine (octadecyl-1,1-dimethyl-piperidino-4-yl-phosphate)" are used for the treatment and/or prophylaxis of the aforementioned diseases and/or pathophysiological conditions in the form of a pharmaceutical kit, where such pharmaceutical kit comprises at least one additional pharmacologically active substance as described herein.

The compounds of the invention are surprisingly characterized by their improved action and/or improved efficacy against the various fungi as well as in the treatment of respective diseases and/or pathophysiological conditions thereof. Due to their surprisingly strong action and/or efficacy, the compounds of the invention may be advantageously administered at lower doses compared to other known but less potent APL or other relevant therapeutic agents while still achieving equivalent or even superior desired biological effects. In addition, such a dose reduction may translate into less or even no medicinal adverse effects.

Furthermore, the compounds of the invention are surprisingly less cytotoxic than known APL or other relevant therapeutic agents while being of at least equivalent if not superior potency. Therefore, use of the compounds of the invention in the treatment of the herein mentioned diseases and/or pathophysiological conditions - even at unreduced doses - may lead to less or even no medicinal adverse effects.

Besides, the compounds of the invention are surprisingly less embryotoxic or even not embryotoxic at all compared to known APL or other relevant therapeutic agents while being of at least equivalent if not superior potency. Embryotoxicity is the ability of a substance to cause harm/be toxic to the embryo, resulting in abnormal development or death.

Moreover, combined use of the compounds of the invention, but also perifosine with known standard therapeutic agents (e.g. benznidazole, nifurtimox, amphotericin B, liposomal amphotericin B and/or paromomycin) in the treatment of herein mentioned diseases and/or pathophysiological conditions is surprisingly characterized by a superior efficacy compared to the (standard) treatment with the single therapeutic agents alone.

Besides, such combination treatment surprisingly allows a dose reduction of the additional therapeutic agents (e.g. benznidazole, nifurtimox, amphotericin B, liposomal amphotericin B and/or paromomycin) applied compared to their single use while being of at least equivalent if not superior efficacy. In addition, such a dose reduction may translate into less or even no medicinal adverse effects.

Furthermore, such combination treatment surprisingly allows a significant time reduction, i.e. shorter courses of treatment, which is advantageous in view of patient compliance and economic healthcare aspects.

The alkyl phospholipid derivative compounds disclosed herein and/or where appropriate additional pharmacologically active substances can be administered in a known manner. The route of administration may thereby be any route which effectively transports the active compound to the appropriate or desired site of action, for example orally or non-orally, in particular topically, transdermally, pulmonary, rectally, intravaginally, nasally or parenteral or by implantation. Oral administration is preferred.

The alkyl phospholipid derivative compounds disclosed herein and/or where appropriate additional pharmacologically active substances are converted into a form which can be administered and are mixed where appropriate with pharmaceutically acceptable carriers or diluents. Suitable excipients and carriers are described for example in Zanowiak P, Ullmann's Encyclopedia of Industrial Chemistry 2005, Pharmaceutical Dosage Forms, 1-33; Spiegel AJ et al., Journal of Pharmaceutical Sciences 1963, 52: 917-927; Czetsch-Lindenwald H, Pharm. Ind. 1961, 2: 72-74; Fiedler HP, Lexikon der Hilfsstoffe für Pharmazie, Kosmetik and angrenzende Gebiete 2002, Editio Cantor Verlag, p65-68.

Oral administration can take place for example in solid form as tablet, capsule, gel capsule, coated tablet, granulation or powder, but also in the form of a drinkable solution. The alkyl phospholipid derivative compounds disclosed herein can for oral administration be combined with known and ordinarily used, physiologically tolerated excipients and carriers such as, for example, gum arabic, talc, starch, sugars such as, for example, mannitol, methylcellulose, lactose, gelatin, surface-active agents, magnesium stearate, cyclodextrins, aqueous or nonaqueous carriers, diluents, dispersants, emulsifiers, lubricants, preservatives and flavorings (e.g. essential oils). The alkyl phospholipid derivative compounds disclosed herein can also be dispersed in a microparticulate, e.g. nanoparticulate, composition.

Non-oral administration can take place for example by intravenous, subcutaneous, intramuscular injection of sterile aqueous or oily solutions, suspensions or emulsions, by means of implants or by ointments, creams or suppositories. Administration as sustained release form is also possible where appropriate. Implants may comprise inert materials, e.g. biodegradable polymers or synthetic silicones such as, for example, silicone rubber. Intravaginal administration is possible for example by means of vaginal rings. Intrauterine administration is possible for example by means of diaphragms or other suitable intrauterine devices. Transdermal administration is additionally provided, in particular by means of a formulation suitable for this purpose and/or suitable means such as, for example, patches.

The dosage may vary within a wide range depending on type and/or severity of the disease and/or pathophysiological condition, the mode of administration, the age, gender, bodyweight and sensitivity of the subject to be treated. It is within the ability of a skilled worker to determine a "pharmacologically effective amount" of an alkyl phospholipid derivative compound as disclosed herein and/or additional pharmacologically active substance. Administration can take place in a single dose or a plurality of separate dosages.

A suitable unit dose is, for example, from 0.001 mg to 100 mg of the active ingredient, i.e. at least one alkyl phospholipid derivative compounds as disclosed herein and, where appropriate, at least one additional pharmacologically active substance, per kg of a patient's bodyweight.

### Chemical synthesis

Synthesis of alkyl phospholipid derivative compounds as disclosed herein is a procedure well described in the prior art and known to the skilled artisan due to his expert knowledge. In this context reference is expressively made to the following patent literature as well as the literature cited therein: EP 0 108 565 A2; WO 87/03478; US 5,980,915; US 6,254,879; US 6,506,393; US 6,172,050; US 6,479,472; US 5,449,798; US 5,958,906.

In the following, further details of syntheses of specific head and tail groups are given. For the avoidance of doubt, it is explicitly stated that the different R residues named in the chemical synthesis section do not correspond to those defined above for formula (I) and specific subsets. Hence, they may differ or have the same meaning.

### B)

Other nitrogen containing head groups were synthesized by methylation (see table 2 and general procedure 2).

**Table 2**

| **Compound** | **Methylated Nitrogen Containing Head Group** |
|---|---|
| **1** | 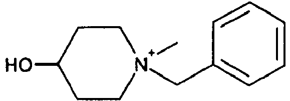 |
| **4** | 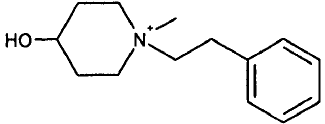 |
| **17** | 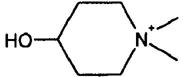 |

### General procedure 2:

where R3 represents a choline derivative and R4 represent the herein disclosed substituted and/or unsubstituted alkyl radicals.

0,5 mol of the choline derivative and 125 ml CH₃CN are placed in a reaction vessel. 0,5 mol p-toluene sulfonic acid methyl ester in 125 ml CH₃CN are added dropwise while keeping reaction temperatures below 10°C. After stirring for 30 min at room temperatur, the mixture is heated to reflux for another 30 min and cooled to room temperatur. The resulting solid ("tosylate") is isolated (if applicable under inert atmosphere), crystallized from 125 ml isopropanol and dryed over P₂O₅ in vacuo. Yields vary from 40 to 60%.

Alternatively, methyl halogenids may also be used for methylation.

### C)

Other nitrogen containing head groups were synthesized by ethylation (see table 3 and general procedure 3).

**Table 3**

| **Compound** | **Nitrogen Containing Head Group** |
|---|---|
| **5** | 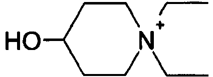 |

### General procedure 3:

where R5 represents a choline derivative and R6 represents the herein disclosed substituted and/or unsubstituted alkyl radicals.

0,5 mol of the choline derivative and 125 ml CH₃CN are placed in a reaction vessel. 0,5 mol ethyl bromide in 125 ml CH₃CN are added dropwise while keeping reaction temperatures below 10°C. After stirring for 30 min at room temperatur, the mixture is heated to reflux for another 30 min and cooled to room temperatur. The resulting solid ("bromide") is isolated (if applicable under inert atmosphere), recrystallized from 125 ml isopropanol and dryed over P₂O₅ in vacuo. Yields vary from 40 to 60%.

Alternatively, ethyl iodine may also be used.

### D)

The various lipophilic tail groups, which are attached to the phosphate moiety, may be used as exemplified in the following ways (see table 4).

**Table 4**

| **Compound** | **Lipophilic Tail Group** |
|---|---|
| **1,5,17** | Saturated alkyl chains: C16, C18 |
| | Unsaturated alkylchains: C20, C22 |

### E)

Alkyl phospholipid derivative compounds 1, 4, 5, 17 were synthesized according to the below mentioned general procedure 4, where "tosylate" denotes the nitrogen containing head group and "alcohol" denotes the lipophilic tail group.

### General procedure 4:

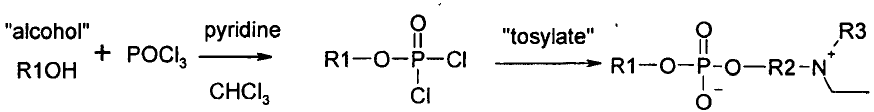

In a reaction vessel, 0.1 mol POCl₃ are placed in 50 ml Chloroform and cooled in an ice bath. A solution of 0.9 mol "alcohol" and 32 ml pyridine in 100ml CH₂Cl₂ is dropped to the POCl₃ solution while keeping the temperature between 5-12° C. After stirring for 30min at room temperature, 0.12 mol "tosylate" are added, cooled to 10° C and 40 ml pyridine is dropped to this solution. The mixture is stirred for 2.5 h at room temperature. 15 ml water are added (T< 20° C) and stirring is continued for another 30 min. The mixture is washed with 200 ml H₂O : MeOH (1:1 v/v), 200 ml 3% HCl : MeOH (1:1 v/v) and 200 ml H2O : MeOH (1:1 v/v). The organic phase is concentrated in vacuo (isopropanol is added in order to diminish foaming). The crude product is recrystallized from 200 ml methyl ethyl ketone. The resulting solid is heated in 150 ml EtOH, filtrated, cooled for 4 h to 5-7° C and again filtrated. 85 g Amberlite MB3 are added to the filtrate and stirred for 3 h at room temperature. After filtrating, the clear solution is concentrated in vacuo and recrystallized from 150ml methyl ethyl ketone. If applicable, the product is purified by column chromatography (CH₂Cl₂ / MeOH / NH₃ (25%) 80 : 25 : 5). Yields vary from 25 to 50%.

The invention is explained in more detail by means of the following examples without, however, being restricted thereto.

### Examples

### I) Synthesis / physicochemical characterization of selected alkyl phospholipid derivate compounds

### Example 1: Compound 1

### Phosphoric acid 1-benzyl-1-methyl-piperidin-4-yl ester hexadecyl ester

3.1g (6%) of compound 1 was obtained starting from hexadecan-1-ol and 1-benzylpiperidine-4-ol according to general procedure 4 after methylating 1-benzyl-piperidine-4-ol according to general procedure 2.

¹H-NMR (600MHz, CDCl₃-d1, 300K):δ = 7.59 (2H, d), 7.47-7.40 (3H, m), 4.78 (2H, broad-s), 4.55 (1 H, broad-s), 3.86-3.77 (4H, m), 3.56 (2H, d), 3.13 (3H, s), 2.27 (2H, d), 2.13 (2H, t), 1.57 (2H, quintet), 1.31-1.18 (26H, m), 0.88 (3H, t) ppm

ESI-MS: found: m/z 510.4 [M+H], calculated: 510.7 g/mol

### Example 4: Compound 4

### Phosphoric acid hexadecyl ester 1-methyl-1-phenethyl-piperidin-4-yl ester

6.3g (12%) of compound 4 was obtained starting from hexadecan-1-ol and 1-phenethyl-piperidine-4-ol according to general procedure 4 after methylating 1-phenethyl-piperidine-4-ol according to general procedure 2.

¹H-NMR (600MHz, CDCl₃-d1, 300K): δ = 7.34-7.22 (5H, m), 4.49 (1H, broad-s), 3.80 (2H, q), 3.77-3.58 (6H, m), 3.29 (3H, s), 3.10 (2H, dd), 2.24 (2H, m), 2.10 (2H, m), 1.59 (2H, quintet), 1.31-1.19 (26H, m), 0.88 (3H, t) ppm

ESI-MS: found: m/z 524.4 [M+H], calculated: 524.8 g/mol

### Example 5: Compound 5

### Phosphoric acid 1,1-diethyl-piperidin-4-yl ester octadecyl ester

6.9g (14%) of compound 5 was obtained starting from octadecan-1-ol and 1-ethyl-piperidin-4-ol according to general procedure 4 after alkylating 1-ethyl-piperidin-4-ol according to general procedure 3.

¹H-NMR (600MHz, CDCl₃-d1, 300K): δ = 4.50 (1 H, broad-s), 3.81 (2H, q), 3.71 (2H, m), 3.62 (2H, m), 3.51 (2H, q), 3.46 (2H, q), 2.25-2.09 (4H, m), 1.58 (2H, quintet), 1.38-1.21 (36H, m), 0.88 (3H, t) ppm

ESI-MS: found: m/z 490.6 [M+H]+ / calculated: 490.8 g/mol

Physicochemical data on further examplified alkyl phospholipid derivative compounds are compiled in Table 5 below:

**Table 5: Examplified alkyl phospholipid derivative compounds with synthesis schemes and MS data:**

| **No.** | **Synthesis schemes** | **[M+H] (calculated)** | **ESI-MS found (M+H)⁺** |
|---|---|---|---|
| **17** | 2,4 | 516.8 | 516.5 |
| **75** | | 434.6 | 434.5 |
| **98** | | 462.7 | 462.3 |
| **116** | | 462.7 | 462.4 |
| **138** | | 448.7 | 448.3 |
| **146** | | 476.7 | 476.5 |
| **169** | | 460.7 | 460.5 |
| **195** | | 538.8 | 538.5 |
| **197** | | 538.8 | 538.5 |
| **213** | | 550.8 | 550.4 |
| **215** | | 504.7 | 504.5 |
| **233** | | 614.9 | 614.4 |

### II). Assay for assessing cytotoxicity against mammalian cell lines

Cytotoxicity of selected compounds of the invention against different mammalian cell lines was assessed as follows.

Human tumor cell lines KB/HeLa (ATCC CCL17, human cervix carcinoma), PC3 (ATCC CRL1435, human prostate carcinoma) and RKOp21 (human colon adenocarcinoma; Schmidt et al., Oncogene 2000, 19: 2423-2429) cells were used in an automated XTT screening assay for the assessment of cytotoxicity.

Further mammalian cell lines that can be tested in the automated XTT screening assay for the assessment of cytotoxicity are FDCP-1 (DSMZ ACC 368, mouse bone marrow), H9c2 (2-1) (ECACC 88092904, rat heart), L8 (ECACC 95102434, rat skeletal muscle), C2C12 (ECACC 91031101, mouse skeletal muscle), CHO (ECACC 85050302, Chinese Hamster ovary), NRK-52E (ECACC 87012902, rat kidney), NRK-49F (ECACC 86101301, rat kidney), MDCK (ECACC 84121903/ATCC CCL-34, canine cocker spaniel kidney), HepG2 (ATCC HB-8065, human hepatocellular carcinoma), NIH3T3 (ATCC CRL-1658, mouse fibroblast), HaCaT (Deutsches Krebsforschungszentrum (DKFZ), human keratinocyte) as well as primary cells, such as primary rat hepatocytes.

The XTT assay quantifies cellular metabolic activity which correlates with cell viability and cell number. Test compounds (selected compounds of the invention and known substances as controls) are dissolved in culture medium at 600µM and added to the tumor cells in 10 different concentrations in a semi-logarithmic fashion starting from 100 µM as highest concentration.

48h later cellular metabolic activity of cells treated with test compounds is quantified by measurement of the absorbance at 490nm (conversion of XTT dye) in comparison to untreated control cells (100% viability).

KB/HeLa and PC3 cells were cultivated in RPMI 1640 medium (Gibco, Cat.No. 42401-018) supplemented with 10% heat inactivated fetal calf serum (FCS, Biochrom AG, Cat.No. S0115), 2 mM L-glutamine (Gibco, Cat.No. 25030-24) and 2% Penicillin-Streptomycin (PenStrep, Gibco, Cat.No. 15140-122). RKOp21 cells were grown in DMEM + GlutaMAXTM-I medium (Gibco, Cat.No. 61965-026) supplemented with 10% heat inactivated fetal calf serum (FCS, Biochrom AG, Cat.No. S0115), 2 mM L-glutamine (Gibco, Cat.No. 25030-24), 2% Penicillin-Streptomycin (PenStrep, Gibco, Cat.No. 15140-122) and 1% 1M HEPES (Gibco, Cat.No. 15630-056). All cells were cultured at 37°C in 5% CO₂ humidified air.

On the first day of the experiment cells were harvested from exponential phase cultures by trypsination, counted and plated into 96 well flat-bottom micro titer plates depending on the cell line as listed below:

| Cell Line | Number / Well | Volume / Well |
|---|---|---|
| KB/HeLa | 2500 | 125 µl |
| PC3 | 6000 | 125 µl |
| RKOp21 | 6000 | 125 µl |

After a 24h recovery to allow the cells to resume exponential growth, the test compounds were diluted immediately prior to use and transferred in 25µl volume to the wells.

Following 2 days of continuous drug exposure 50µl of freshly prepared XTT-PMS solution {2% 0.386mg/ml PMS (N-Methyl Dibenzopyrazine Methylsulphate; Sigma, Cat.No. P9625) in PBS + 98% 1mg/ml XTT (Sodium 2,3-bis(2-methoxy-4-nitro-5-sulfophenyl)-5-[(phenylamino)carbonyl]-2H-Tetrazolium; Serva, Cat.No. 38450) in RPMI 1640 medium without phenol red} was added to the wells. To measure the proportion of living cells, the cells were incubated for 3 h with XTT-PMS reagent at 37°C, 5% CO₂, humidified atmosphere allowing to form the formazan salt. The amount of soluble formazan salt produced by cellular reduction of XTT is quantified by measuring the absorbance at 490nm using the Biomek^{®} 2000 ELISA plate reader.

%-inhibition (cytotoxicity) of each test compound was calculated in relation to untreated control cells. Inhibition curves were generated and IC₅₀ values calculated by using GraphPad Prism.

In the following **table 6** cytotoxicity results (IC₅₀ values) obtained for selected compounds of the invention are presented in comparison to examples of the prior art (perifosine).

**Table 6: Cytotoxicity assay results for mammalian RKOp21, KB/HELA and PC3 cell lines (IC₅₀ values for a number of selected exemplary compounds)**

| **Compound** | **RKOp21** [IC₅₀ (µM)] | **PC3** [IC₅₀ (µM)] | **KB/HELA** [IC₅₀ (µM)] |
|---|---|---|---|
| **17** | 32,47 | 18,28 | 16,59 |
| **85** | >100 | 32,90 | 43,66 |
| **86** | >100 | >100 | 75,64 |
| **116** | 39,97 | 50,25 | 37,72 |
| **185** | >100 | 30,36 | >100 |
| **200** | >100 | 34,52 | 66,38 |
| **203** | >100 | 63,12 | >100 |
| **258** | >100 | > 100 | >100 |
| **262** | >100 | 31,25 | 10,10 |
| **282** | >100 | >100 | >100 |
| **302** | >100 | >100 | >100 |
| **perifosine** | 3,31 | 3,22 | 2,37 |

The result presented in **table 6** clearly demonstrate the favorable lower cytotoxicity of the selected compounds of the invention compared to prior art compound perifosine.

### III) Assay for assessing embryotoxicity against mammalian cell lines

Interaction of chemicals with the differentiation process during foetal development can cause embryotoxicity. The model of *in vitro* embryonic stem cell test (EST) has the potential to screen chemical compounds *in vitro* for their ability to interact and disturb this differentiation process.

Since the first organ that is formed during organogenesis is the heart, differentiation of mouse embryonic stem cells (D3 pluripotent embryonic stem cell line) into cardiomyocytes *in vitro* has been documented. The *in vitro* process for the differentiation into heart beating cells is well characterised and highly standardised. In the presence of mLIF (mouse leukaemia inhibiting factor) D3 cells can be maintained in the undifferentiated stage as a continuous cell line. In the absence of mLIF the cells will form embryonic bodies that subsequently spontaneously differentiate into functional cardiomyocytes and beating can be observed by simple microscopic evaluation.

Determination of embryotoxicity of selected compounds of the invention is performed by this embryonic stem cell test (EST). Two permanent mouse cell lines are used to assess the embryotoxic potential of test compounds: 3T3 fibroblasts (ATCC CCL-92; ATCC CRL-1658, ATCC CCL-163) and the embryonic stem cell line D3 (ATCC CRL-1934).

Inhibition of differentiation and growth are determined in embryonic stem cells and compared to inhibition of growth in 3T3 fibroblasts, which serves as surrogate for adult cells. Three endpoints are used to classify the embryotoxic potential of test compounds in the EST: Inhibition of growth of embryonic stem cells and 3T3 fibroblasts in MTT assay (MTT cell viability assay kit, Cat.No. 30006, Biotium Inc., www.biotium.com) by 50% of the control (IC50 D3, IC50 3T3) and the inhibition of the differentiation of embryonic stem cells into spontaneously contracting cardiomyocytes by 50% (ID50).

The embryotoxic potential of selected compounds of the invention is determined by ranking experiments in relation to prior art APL.

### V) Anti-fungal activity assay

Anti-fungal activity of selected compounds of the invention was assessed by means of anti-fungal susceptibility tests as follows.

The antifungal activities of the compounds of the invention against different fungi were measured according to standard broth microdilution methods of the US National Committee for Clinical Laboratory standards (NCCLS) for yeasts (NCCLS document M27-A, 1997) and filamentous fungi (NCCLS document M38-A, 2002).

Compounds of the invention were tested for *in vitro* antifungal activity against different fungal strains (listed in **Table 10).**

**Table 10**

| | **Test strain** | **Morphology** | **Origin** | **ATCC Medium** | **Temp. ATCC** |
|---|---|---|---|---|---|
| **A** | Aspergillus fumigatus ATCC 204305 | filamentous | ATCC | Malt extract agar Malt extract 20g Glucose 20g Peptone 1g ad aqua dest. 1l | 25°C |
| **B** | Aspergillus fumigatus 322-384 | filamentous | isolate | | |
| **C** | Aspergillus fumigatus 040-200167 | filamentous | isolate | | |
| **D** | Candida albicans ATCC 10231 | yeast | ATCC | Yeast mold broth | 25°C |
| **E** | Candida albicans ATCC 90028 | yeast | ATCC | Yeast mold broth | 35°C |
| **F** | Candida albicans TS3 (Shrikantha et al., Journal of Bacteriology 2000, 182(6):1580-1591) | yeast | Auxotroph strain of ura 3. | Cells were maintained on agar containing modified Lee's medium supplemented with 0.01 mM uridine. | |
| **G** | Candida parapsilosis ATCC 22019 | yeast | ATCC | Yeast mold broth | 35°C |
| **H** | Cryptococcus neoformans ATCC 90112 | yeast | ATCC | Yeast mold broth | 24°C |
| **I** | Cryptococcus neoformans H99 (Ganendren et al., Antimicrobial Agents and Chemotherapy, 2004, 48(5):1561-1569) | yeast | Clinical isolate from human cerebrospinal fluid; | RPMI broth medium | |
| **J** | Cryptococcus gattii ATCC 32608 | yeast | ATCC | Malt agar medium Malt extract 30g Agar, Bacto 15g ad aqua dest 1l; pH 5.5 | 26°C |

Culture conditions of ATCC strains were obtained from the web site (www.atcc.org).

Stock solutions of compounds of the invention were freshly prepared on the days of experiments equal to 1.28mg/10ml media. Serial dilutions were performed to achieve the following testing concentrations: 64µg/ml, 32µg/ml, 16µg/ml, 8µg/ml, 4µg/ml, 2µg/ml, 1µg/ml, 0.5µg/ml, 0.25µg/ml and 0.125µg/ml.

An inoculum of 10⁶ CFU/ml of the fungi strain was prepared in the appropriate medium and transferred to test tubes for incubation.

The method is used to determine the minimum inhibitory concentrations (MIC) of drugs on fungi strains. The MIC was defined by photometric measurement at 595nm after 48h of culture at 35°C at that concentration which demonstrated more than 80% inhibition of visible growth for yeast protocol. For the filamentous fungi protocol, MIC determination took place after 48 to 72 h of culture at 35°C and was defined at that concentration which produced more than 50% visible growth inhibition.

**Table 11** displays the results (MIC values in µg/mL) of the anti-fungal activity assay obtained for selected compounds of the invention (compounds 1, 5) in comparison to prior art example erucylphosphocholine (ErPC).

**Table 11: Susceptibilities of different fungal species (MICs in µg/mL) to selected alkyl phospholipid derivative compounds**

| **fungus** | **1** | **5** | **ErPC** |
|---|---|---|---|
| **A** | 2 | 6 | >64 |
| **B** | 2 | 8 | >64 |
| **C** | 4 | 24 | >64 |
| **D** | 2 | 2 | >64 |
| **E** | 1 | 2 | >64 |
| **F** | 1,5 | 2 | 64 |
| **G** | 3 | 3 | 64 |
| **H** | 1,5 | 1,5 | 4 |
| **I** | 2 | 2 | 3 |
| **J** | 1 | 0,75 | 1,5 |

The result presented in table 11 show the strong antifungal activity of the selected compounds of the invention compared to prior art compound erucylphosphocholine (ErPC).

## Claims

1. Use of an alkyl phospholipid derivative according to formula (I) 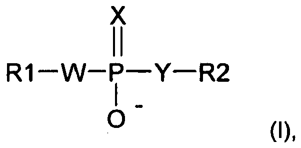 wherein:
W, X, Y independently are selected from the group consisting of: "oxygen atom, sulphur atom";
R1 is "-R5";
R2 is "-R8";
R5 is independently selected from the group consisting of: "substituted or unsubstituted C8-C30alkyl;
R8 is selected from the group consisting of: "substituted or unsubstituted heterocycle",
where heterocycle is
(i) a 5-, 6- or 7-membered saturated, partially unsaturated or aromatic monocyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom,
and where heterocycle if substituted is substituted with at least one radical R12, which in case of two or more radicals R12 are independently selected from each other identical, partly identical or different;
R12 is independently from each other selected from the group consisting of: "hydrogen atom, substituted or unsubstituted C1-C18alkyl, substituted or unsubstituted C3-C8cycloalkyl, substituted or unsubstituted (C1-C12alkyl)ₛ-B-(C1-C12alkyl)ₜC-(C1-C12alkyl)ᵤ, substituted or unsubstituted aryl, substituted or unsubstituted heteroaryl, substituted or unsubstituted alkoxy, -OH, halogen, -F, -Cl, -Br, -I, =O, -C(O)O-(C1-C12alkyl), -C(O)O-(C3-C8cycloalkyl), -C(O)CO-aryl, -C(O)O-heteroaryl, - C(O)O-heterocyclyl, -C(O)-(C1-C12alkyl), -C(O)-(C3-C8cycloalkyl), -C(O)-aryl, -C(O)-heteroaryl, -C(O)-heterocyclyl";
B, C are independently from each other selected from the group consisting of "oxygen atom; sulphur atom; S(O₂)";
s, t, u independently from each other are 0 or 1;
for the manufacture of a medicament for the treatment or prophylaxis of diseases and/or
pathophysiological conditions in mammals that are caused by a fungus selected from the group consisting of "Absidia spp., Acremonium spp., Alternaria spp., Aspergillus spp.,
Bipolaris spp., Candida spp., Cladophialophora spp., Cladosporium spp., Coccidioides spp., Coniothyrium spp., Cryptococcus spp., Cunninghamella spp., Curvularia spp.,
Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp.,
Phialophora spp., Pseudallesheria spp., Pyrenochaeta spp., Rhizomucor spp., Rhizopus spp., Scedosporium spp., Scopulariopsis spp., Scytalidium spp., Sporothrix spp.,
Trichophyton spp. and/or Wangiella spp."

2. The use of an alkyl phospholipid derivative as claimed in claim 1, where the fungus is preferably selected from the group consisting of "Absidia spp., Aspergillus spp., Bipolaris spp., Candida spp., Cryptococcus spp., Cunninghamella spp., Exophiala spp., Fusarium spp., Paecilomyces spp., Rhizopus spp. and/or Scedosporium spp.".

3. The use as claimed in any of claims 1 to 2, where the alkyl phospholipid derivative is selected from the group consisting of:

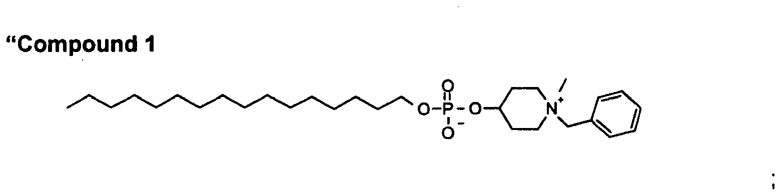

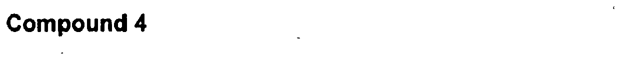

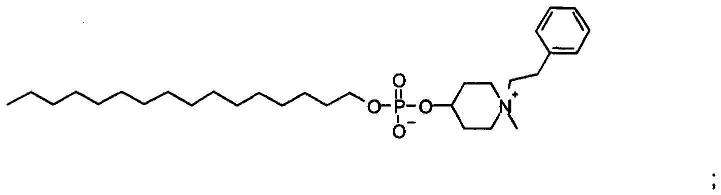

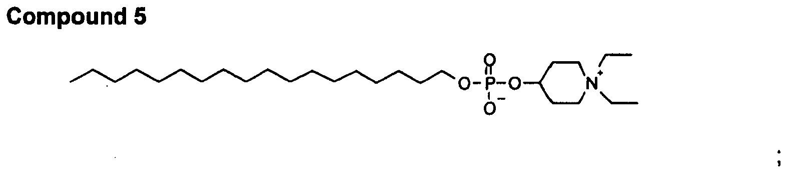

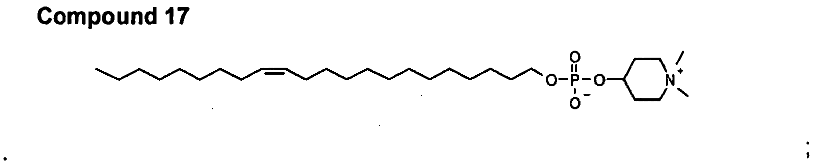

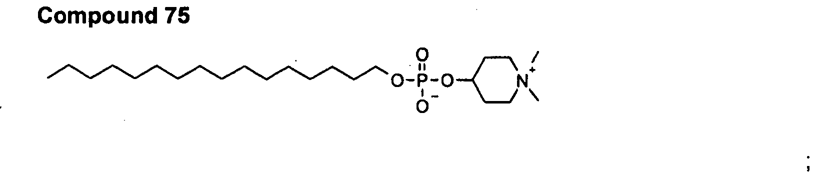

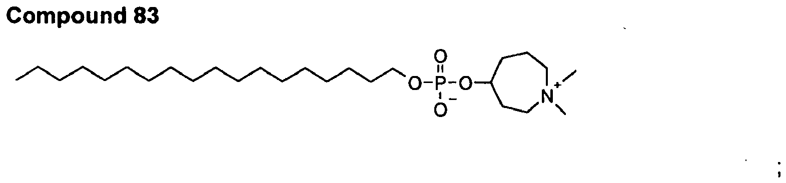

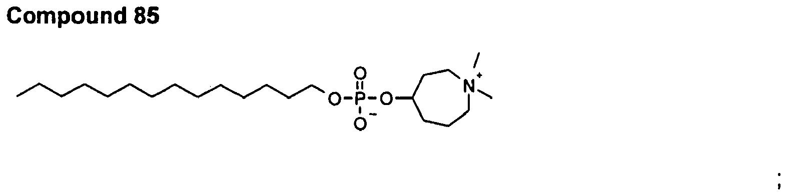

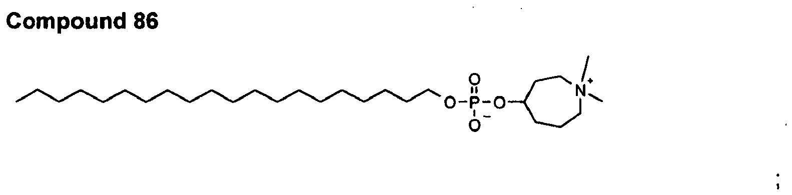

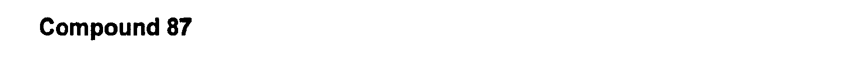

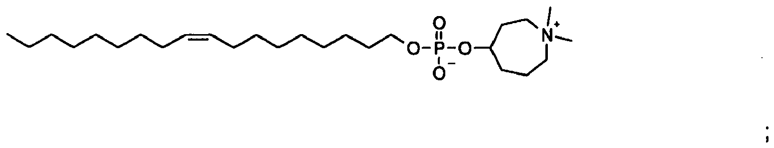

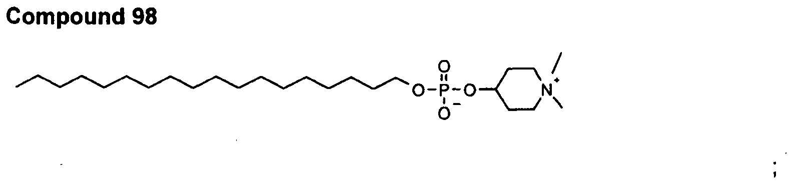

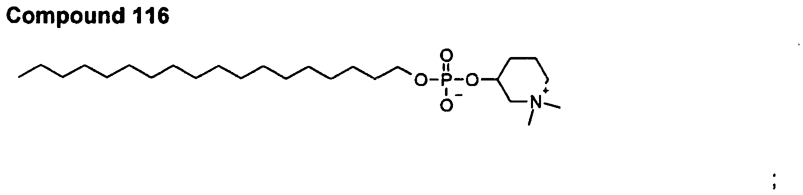

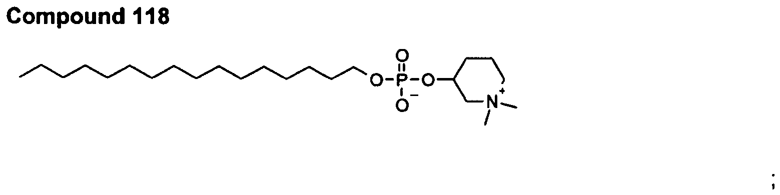

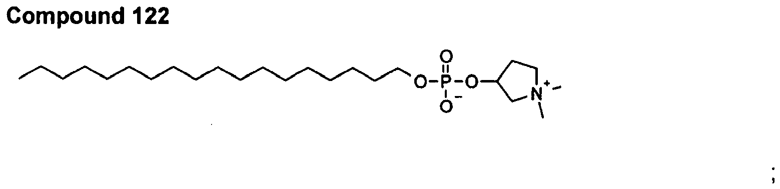

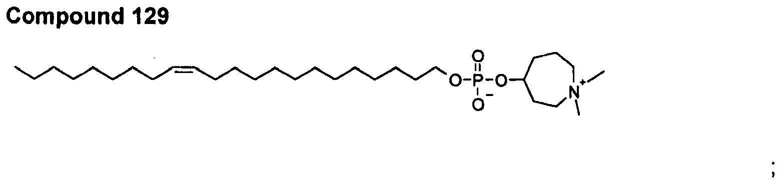

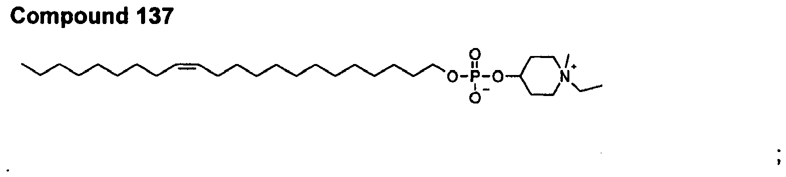

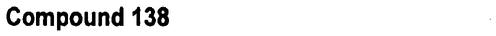

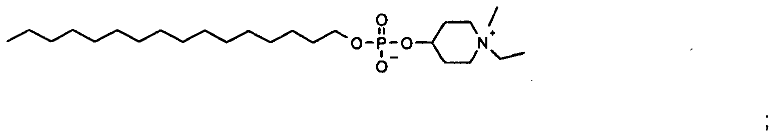

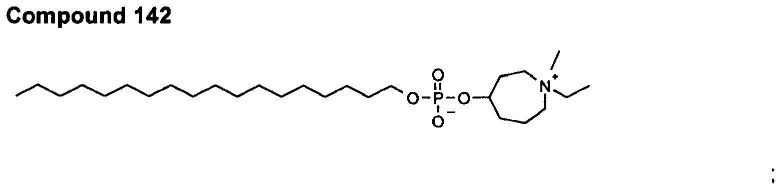

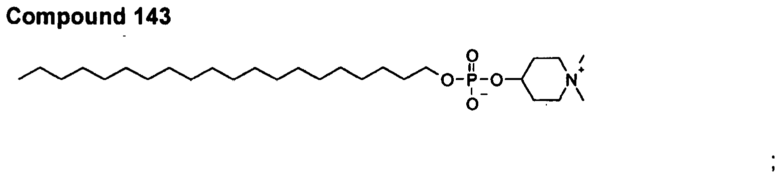

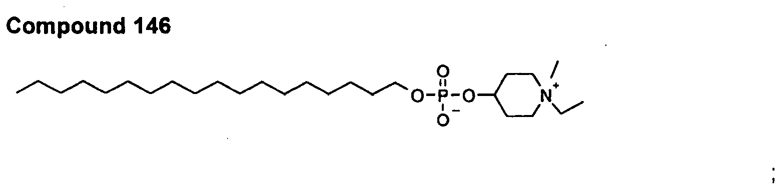

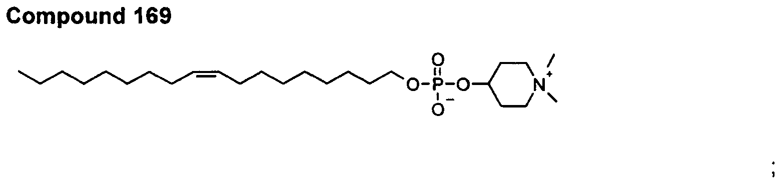

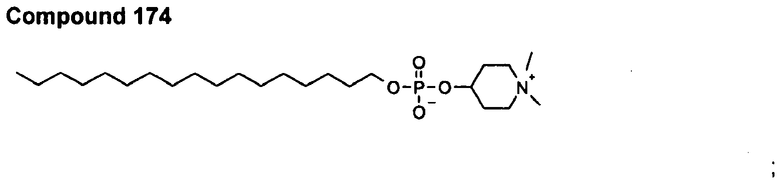

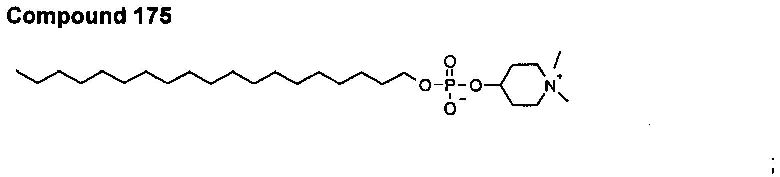

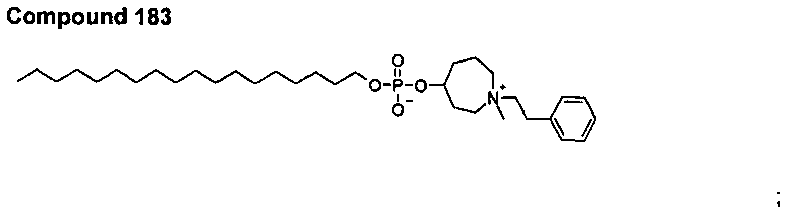

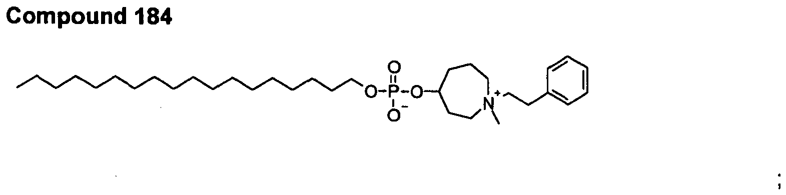

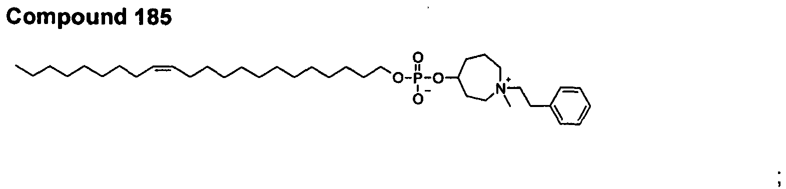

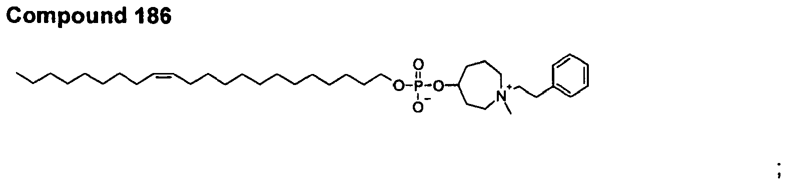

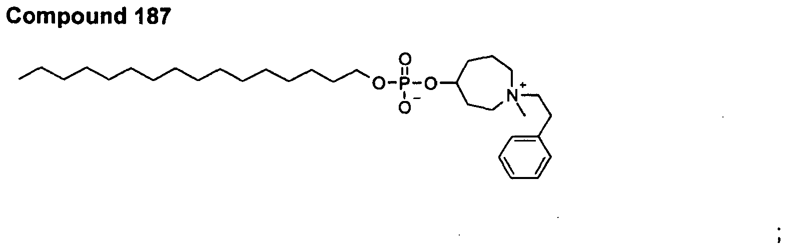

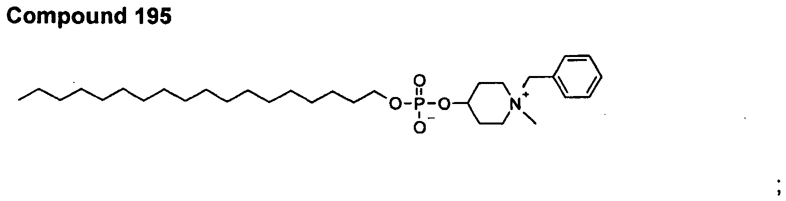

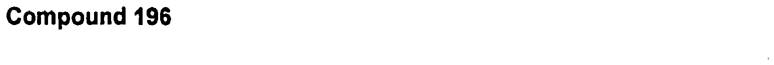

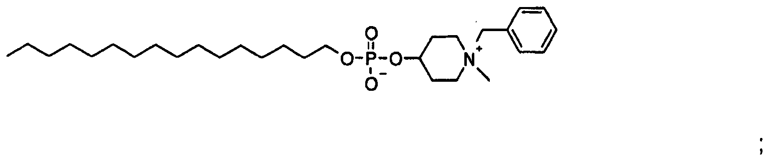

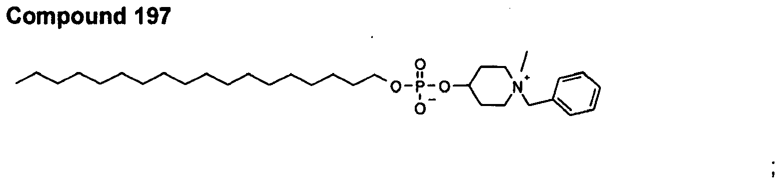

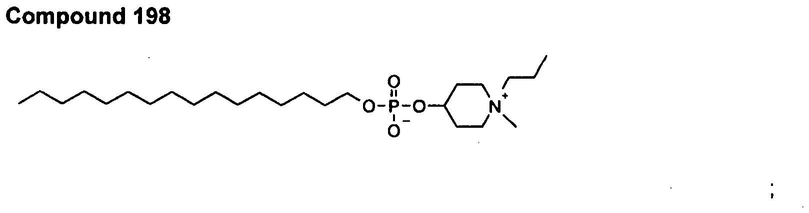

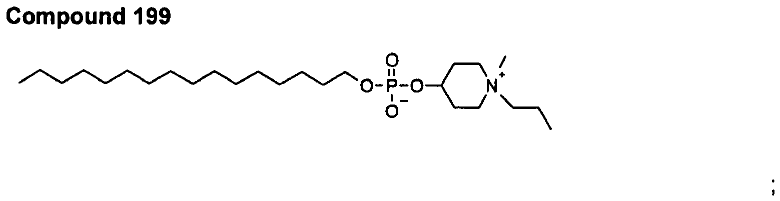

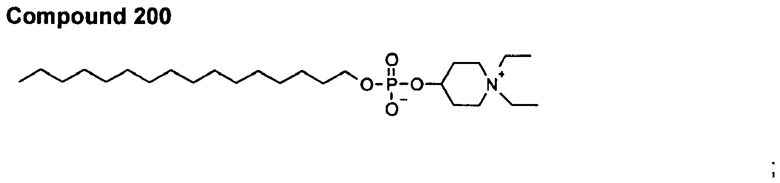

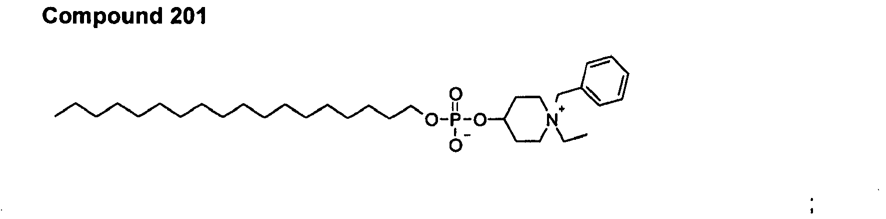

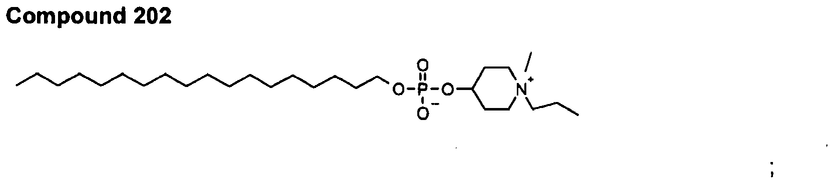

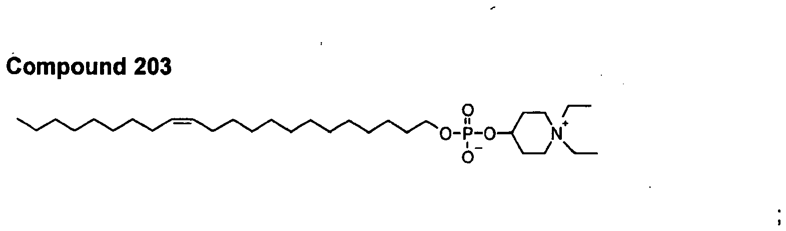

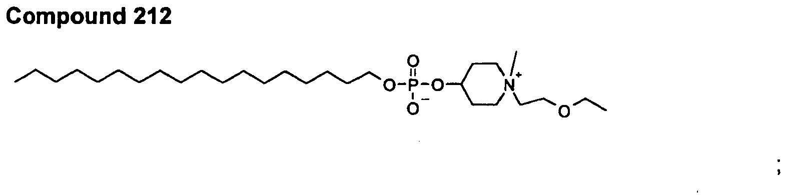

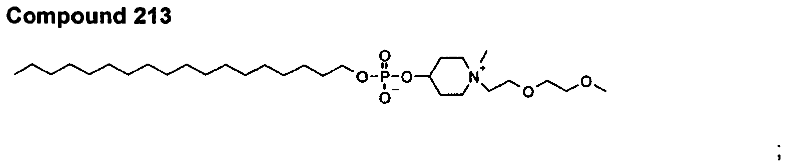

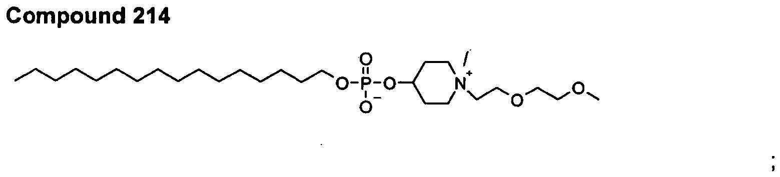

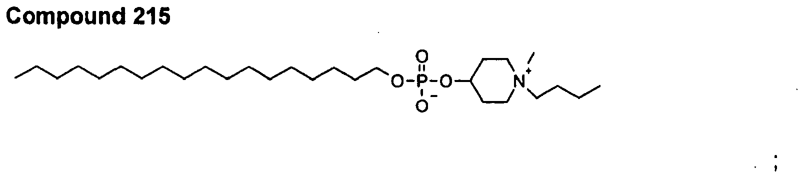

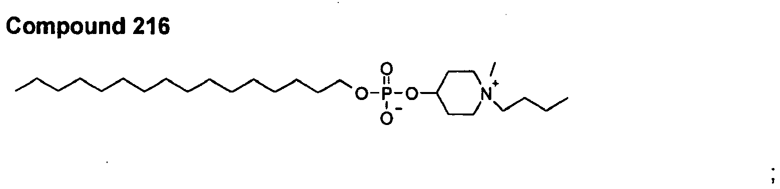

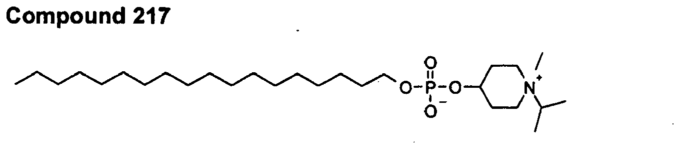

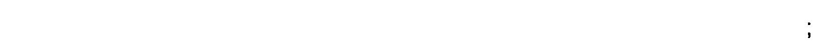

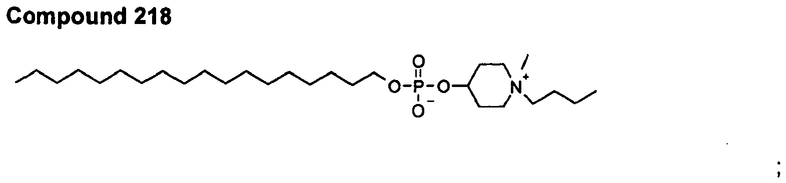

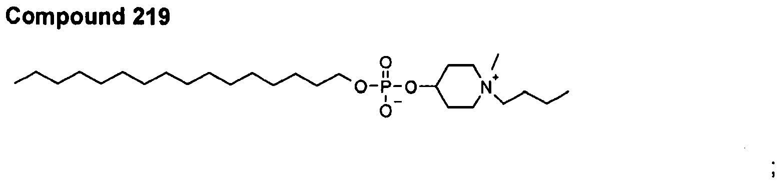

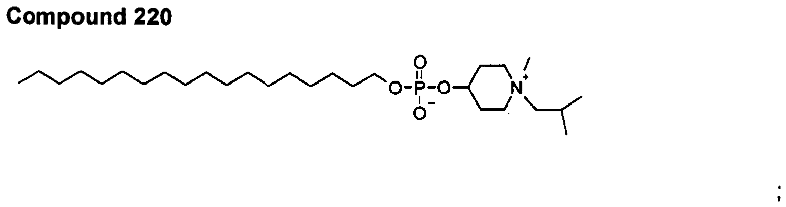

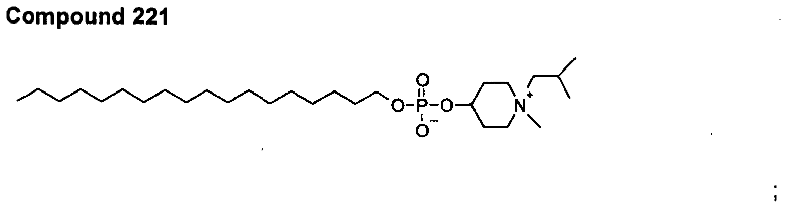

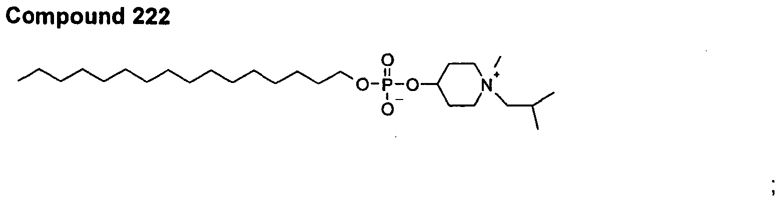

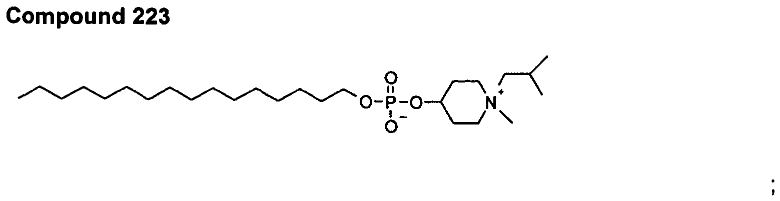

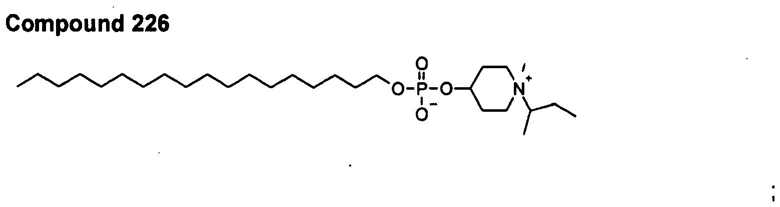

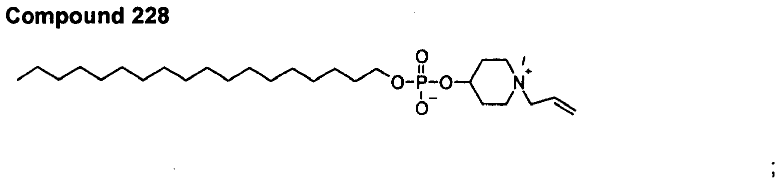

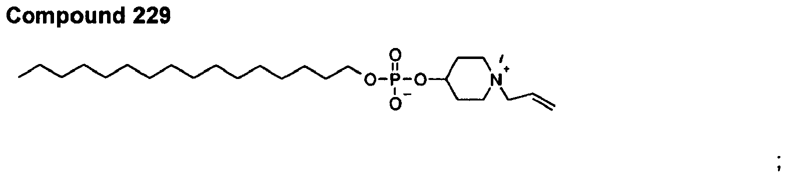

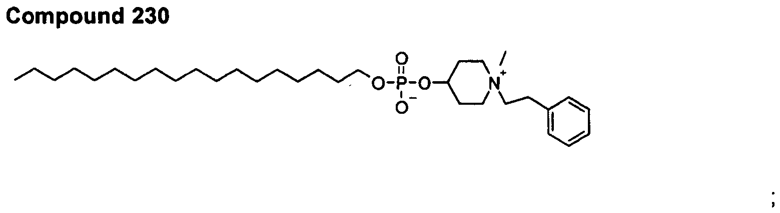

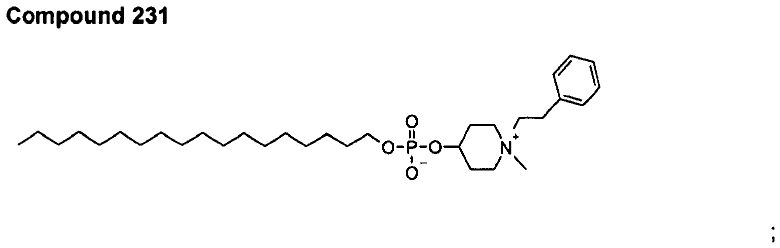

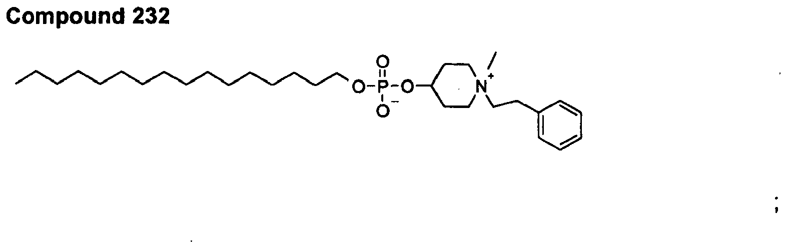

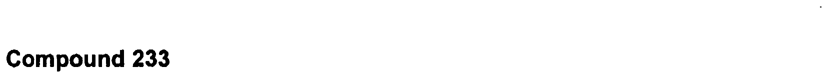

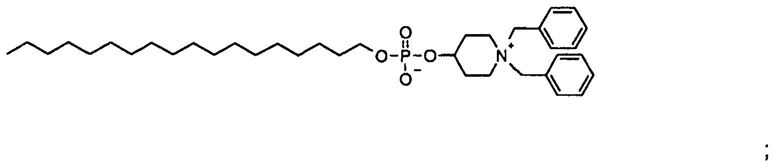

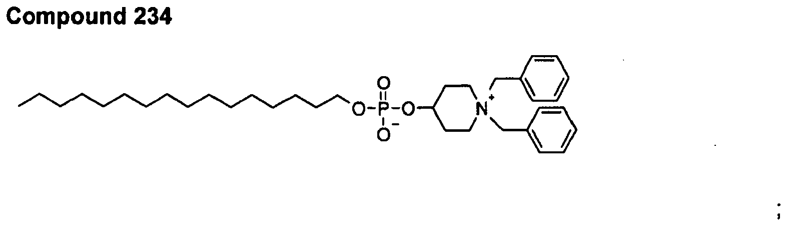

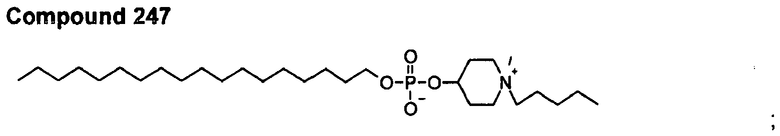

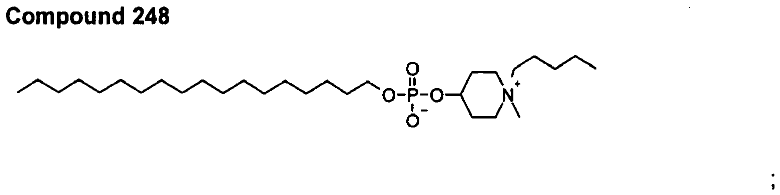

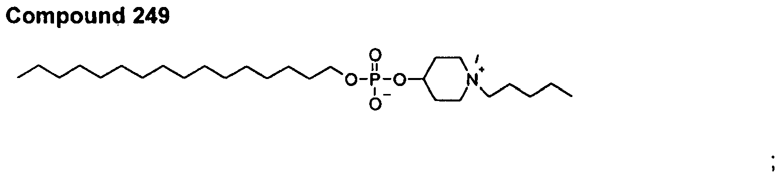

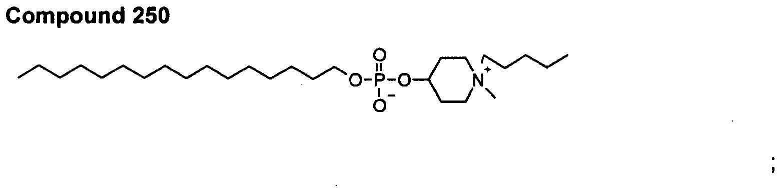

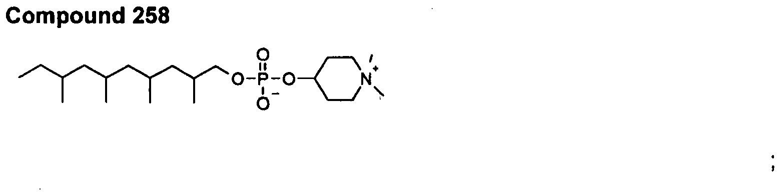

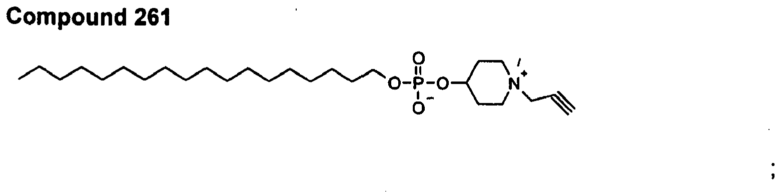

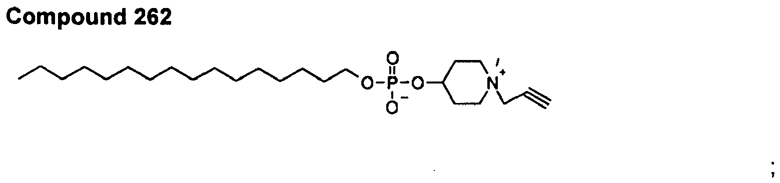

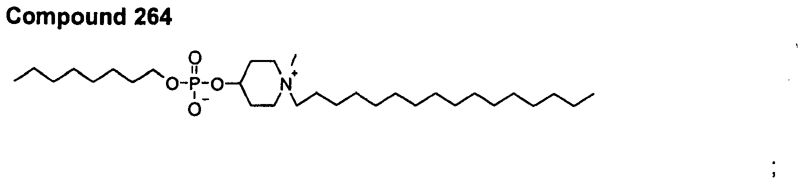

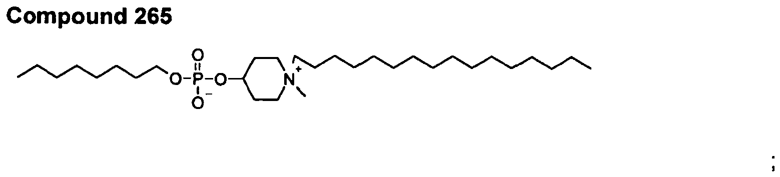

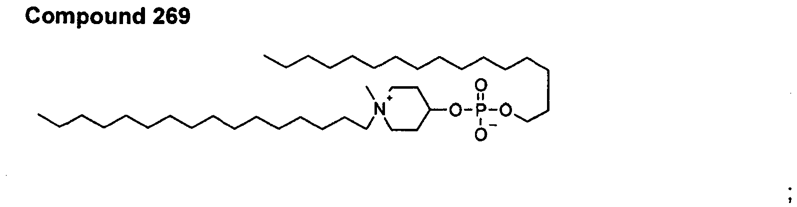

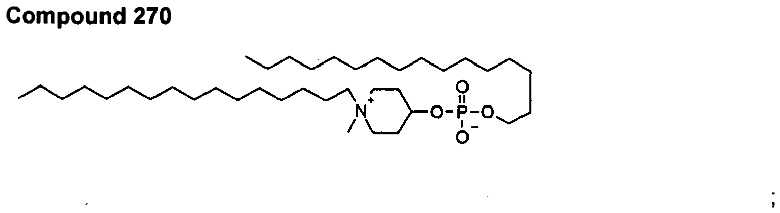

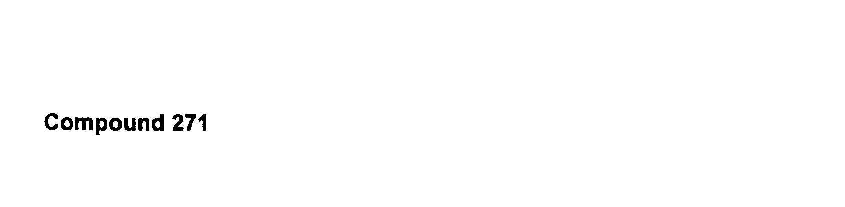

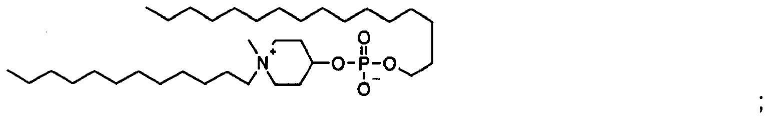

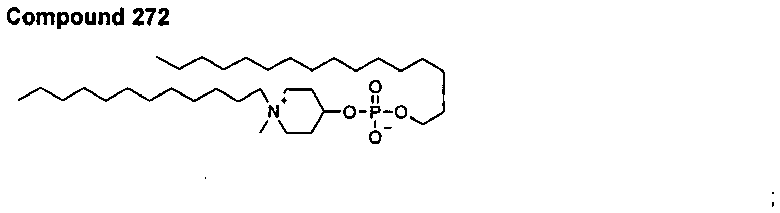

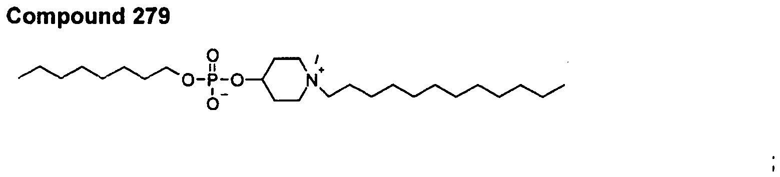

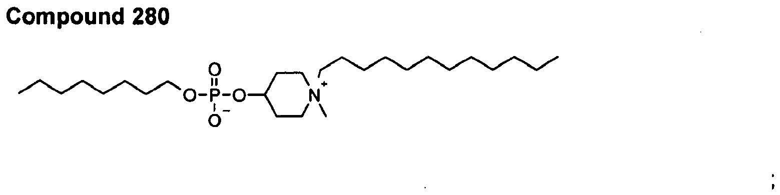

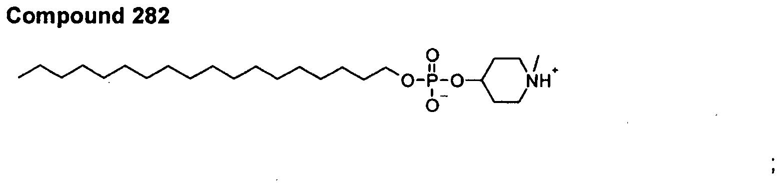

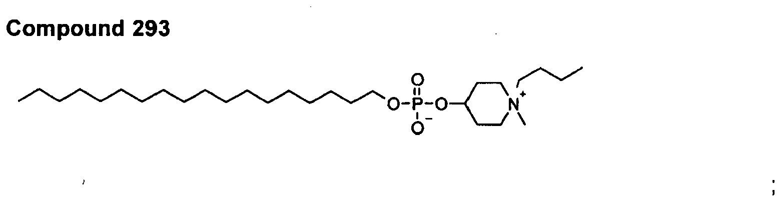

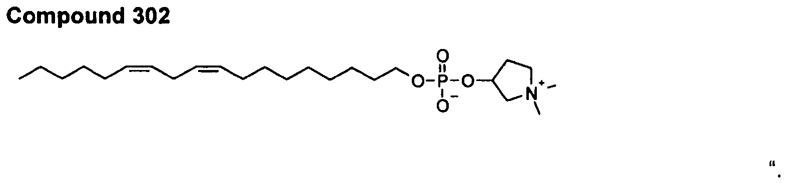

4. The use as claimed in claims 1 to 3, where the alkyl phospholipid derivative is selected from the group consisting of: **"Compound 1, 4, 5** and/or **compound 17".**

5. The use as claimed in any of claims 1 to 4, where the disease and/or pathophysiological condition is selected from the group consisting of "aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium, zygomycosis as well as their different forms and subforms".

6. The use as in any of claims 1 to 5, where the mammal is selected from the group consisting of "human, domestic animals, cattle, livestock, pets, cow, sheep, pig, goat, horse, pony, donkey, hinny, mule, hare, rabbit, cat, dog, guinea pig, hamster, rat, mouse" and preferably is human.

7. The use as claimed in any of claims 1 to 6, where such medicament comprises at least one additional pharmacologically active substance.

8. The use as claimed in any of claims 1 to 7, where the medicament is be applied before and/or during and/or after treatment with at least one additional pharmacologically active substance.

9. The use as claimed in any of claims 7 to 8, where such at least one additional pharmacologically active substance is selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1]-nonatriaconta-19, 21, 23, 25, 27, 29, 31-heptaene-36-carboxylic acid]; liposomal amphotericin B, sitamaquine (N,N-diethyl-N'-(6-methoxy-4-methyl-8-quinolinyl)-1,6-Hexanediamine) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

10. The use as claimed in any of claims 7 to 8, where the alkyl phospholipid derivative is "perifosine (octadecyl-1, 1-dimethyl-piperidino-4-yl-phosphate)" and the at least one additional pharmacologically active substance is selected from the group consisting of "benznidazole (N-Benzyl-2-nitroimidazol-1-yl-acetamide); nifurtimox (3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1]-nonatriaconta-19,21, 23, 25, 27, 29, 31-heptaene-36-carboxylic acid]; liposomal amphotericin B, sitamaquine (N,N-diethyl-N'-(6-methoxy-4-methyl-8-quinolinyl)-1,6-Hexanediamine and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

11. The use as claimed in any of claims 7 to 10, where the at least one alkyl phospholipid derivative and the at least one additional pharmacologically active substance are to be applied as a pharmaceutical kit.

## Patentansprüche

1. Verwendung eines Alkylphospholipidderivats der Formel (I) 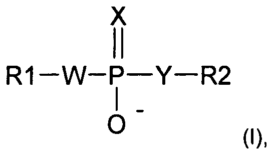 wobei:
W, X, Y unabhängig voneinander aus der aus: "Sauerstoffatom, Schwefelatom" bestehenden Gruppe ausgewählt sind;
R1 für "-R5" steht;
R2 für "-R8" steht;
R5 unabhängig voneinander aus der aus: "substituiertes oder unsubstituiertes C8-C30-Alkyl" bestehenden Gruppe ausgewählt ist;
R8 aus der aus: "substituierter oder unsubstituierter Heterocyclus" bestehenden Gruppe ausgewählt ist, wobei Heterocyclus für
(i) ein 5-, 6- oder 7-gliedriges gesättigtes, teilweise ungesättigtes oder aromatisches monocyclisches Kohlenstoffatomringsystem mit mindestens einem aus der aus: "Stickstoffatom, Sauerstoffatom, Schwefelatom, Arsenatom" bestehenden Gruppe ausgewählten Heteroatom steht, mit der Maßgabe, dass es sich bei mindestens einem Heteroatom um ein quartäres Stickstoffatom oder ein quartäres Arsenatom handelt,
und wobei der Heterocyclus, wenn er substituiert ist, mit mindestens einem Rest R12 substituiert ist, wobei bei zwei oder mehr Resten die Reste R12 unabhängig voneinander ausgewählt sind und identisch, teilweise identisch oder verschieden sind;
R12 unabhängig von den anderen aus der aus: "Wasserstoffatom, substituiertes oder unsubstituiertes C1-C18-Alkyl, substituiertes oder unsubstituiertes C3-C8-Cycloalkyl; substituiertes oder unsubstituiertes (C1-C12-Alkyl)ₛ-B-(C1-C12-alkyl)ₜ-C-(C1-C12-alkyl)ᵤ, substituiertes oder unsubstituiertes Aryl, substituiertes oder unsubstituiertes Heteroaryl, substituiertes oder unsubstituiertes Alkoxy, -OH, Halogen, -F, -Cl, -Br, -I, =O, -C(O)O-(C1-C12-Alkyl), -C(O)O-(C3-C8-Cycloalkyl), -C(O)O-Aryl, -C(O)O-Heteroaryl, -C(O)O-Heterocyclyl, -C(O)-(C1-C12-Alkyl), -C(O)-(C3-C8-Cycloalkyl), -C(O)-Aryl, -C(O)-Heteroaryl, -C(O)-Heterocyclyl" bestehenden Gruppe ausgewählt ist;
B, C unabhängig voneinander aus der aus "Sauerstoffatom; Schwefelatom; S(O₂)" bestehenden Gruppe ausgewählt sind;
s, t, u unabhängig voneinander für 0 oder 1 stehen;
zur Herstellung eines Medikaments zur Behandlung oder Prophylaxe von Krankheiten und/oder pathophysiologischen Leiden in Säugetieren, die durch einen aus der aus "Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. und/oder Wangiella spp." bestehenden Gruppe ausgewählten Pilz verursacht werden.

2. Verwendung eines Alkylphospholipidderivats nach Anspruch 1, wobei der Pilz vorzugsweise aus der aus "Absidia spp., Aspergillus spp., Bipolaris spp., Candida spp., Cryptococcus spp., Cunninghamella spp., Exophiala spp., Fusarium spp., Paecilomyces spp., Rhizopus spp. und/oder Scedosporium spp." bestehenden Gruppe ausgewählt ist.

3. Verwendung nach Anspruch 1 oder 2, wobei das Alkylphospholipidderivat aus der aus den folgenden Verbindungen bestehenden Gruppe ausgewählt ist:

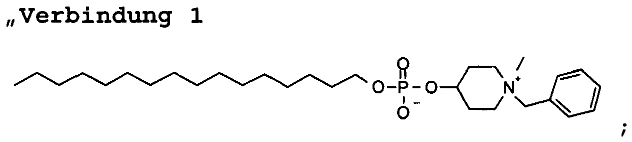

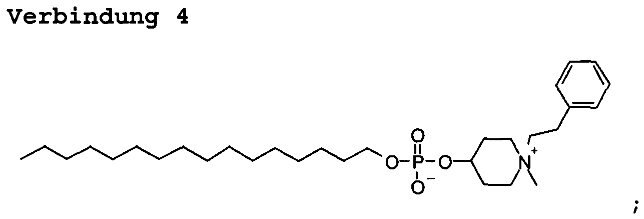

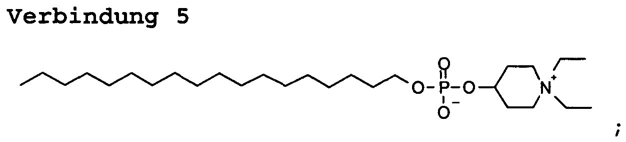

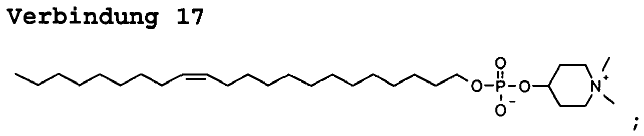

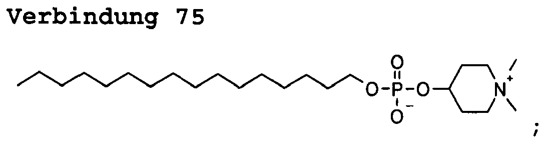

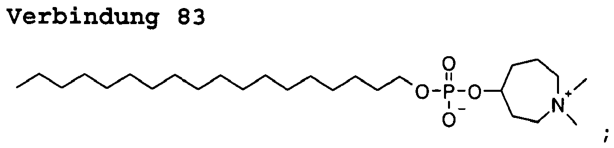

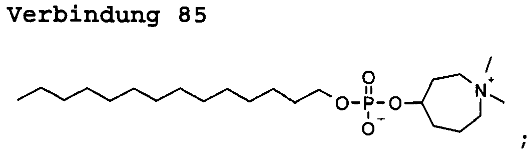

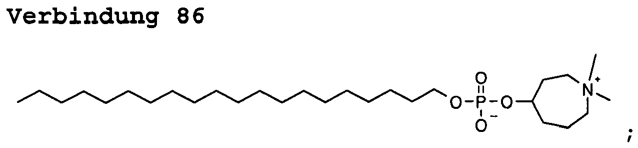

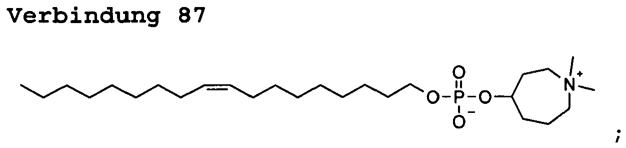

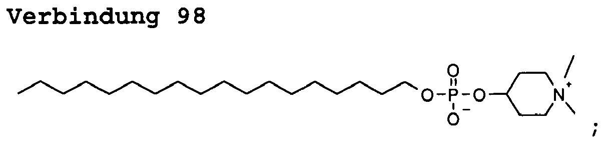

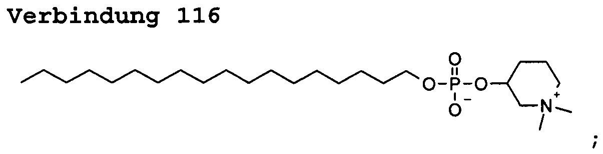

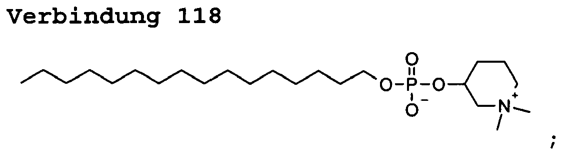

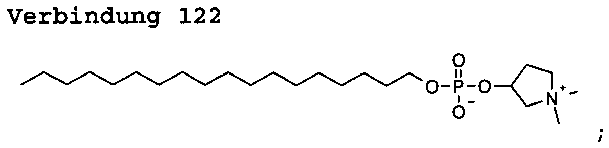

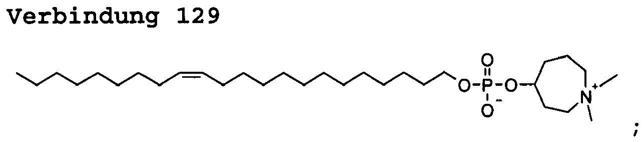

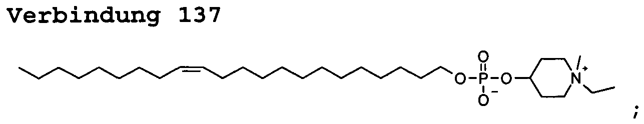

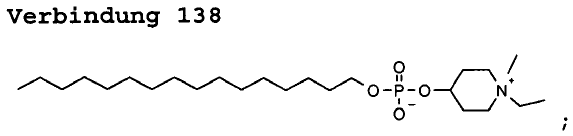

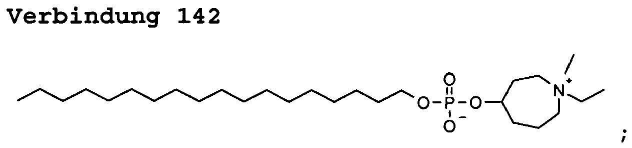

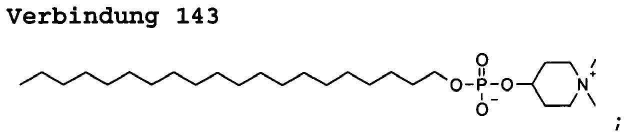

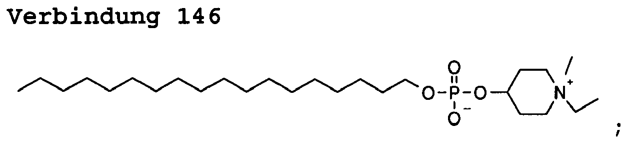

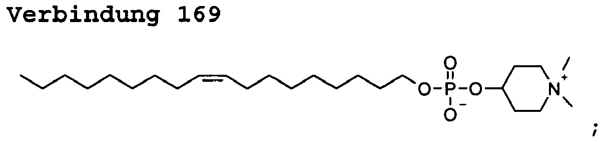

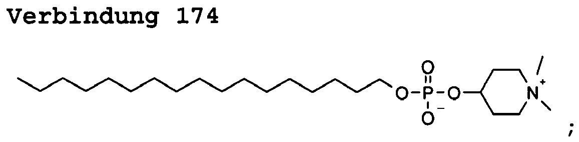

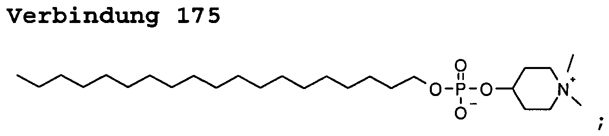

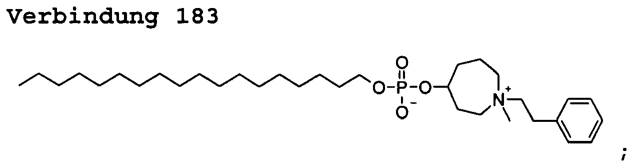

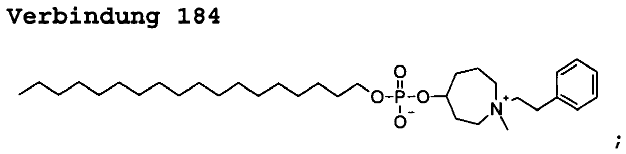

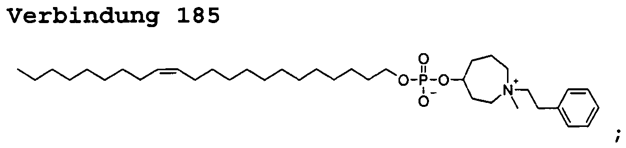

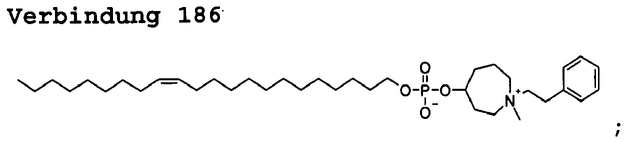

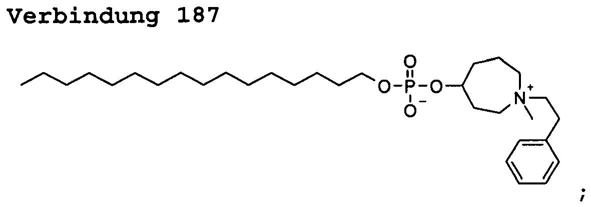

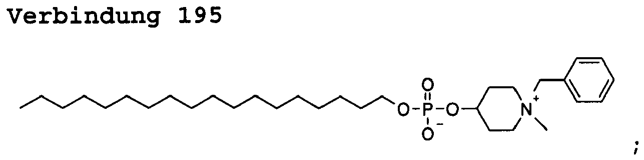

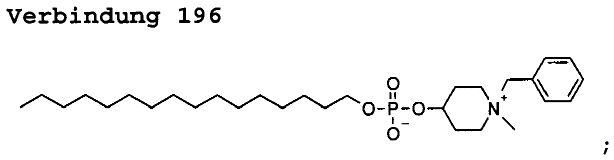

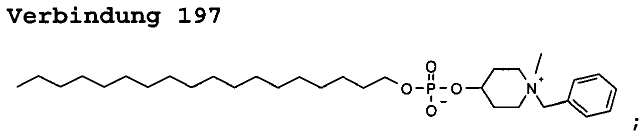

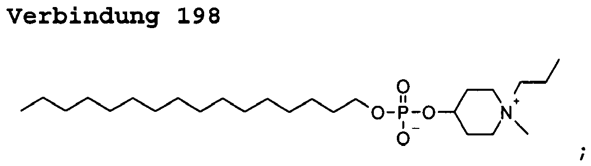

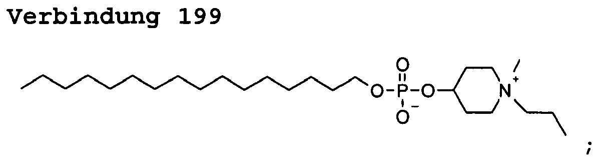

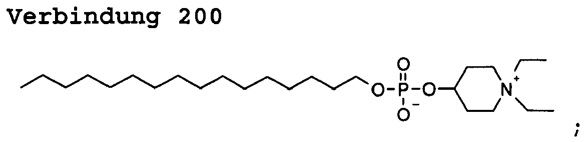

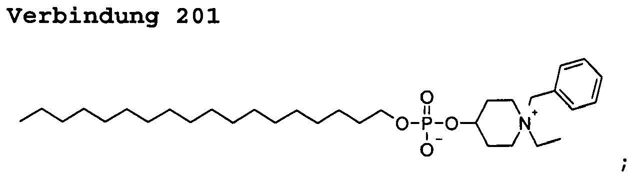

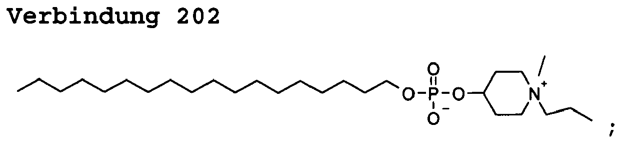

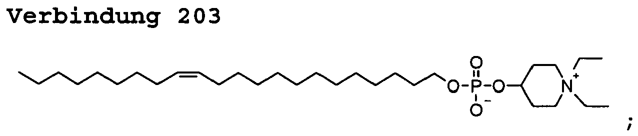

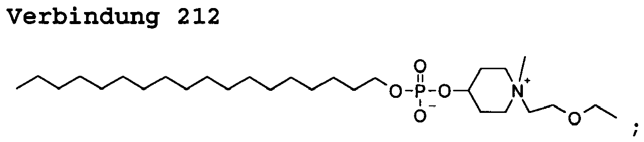

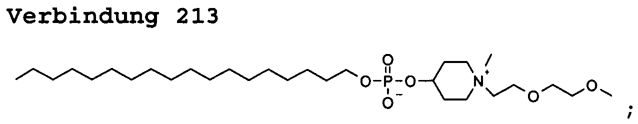

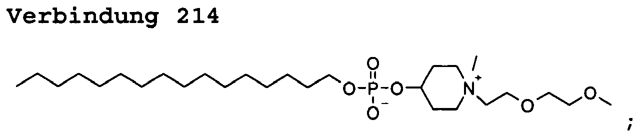

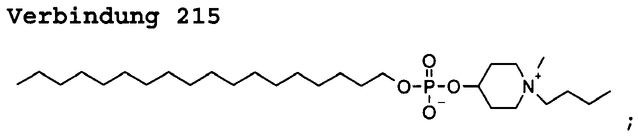

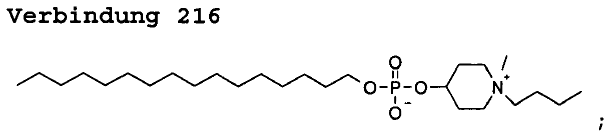

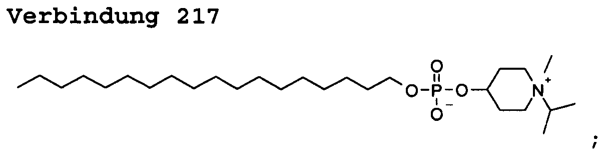

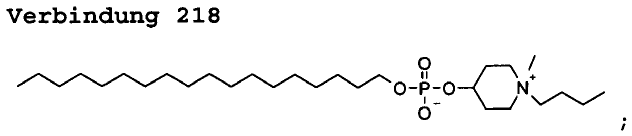

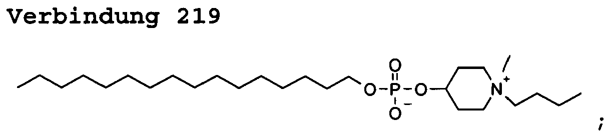

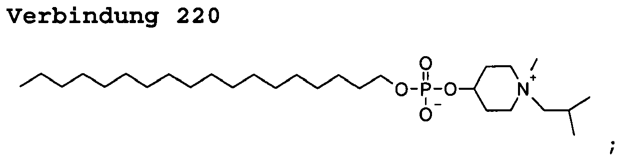

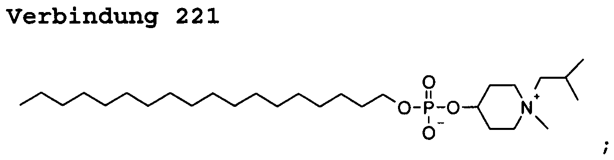

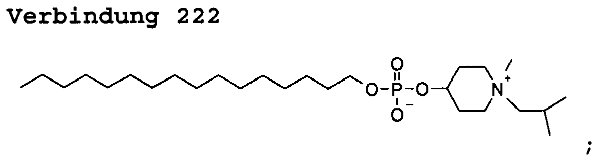

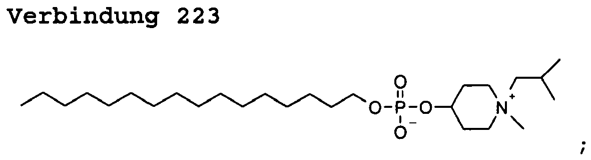

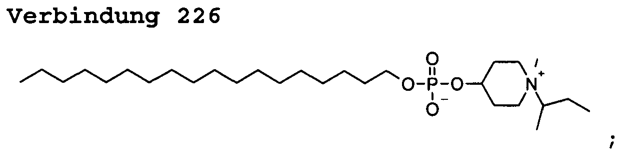

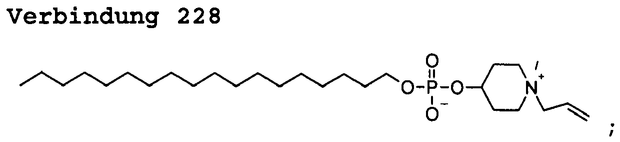

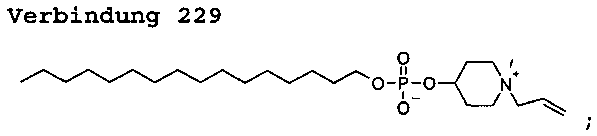

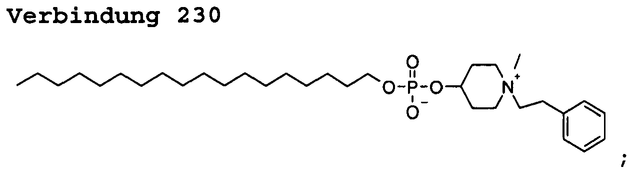

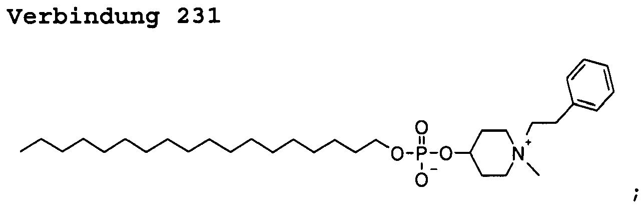

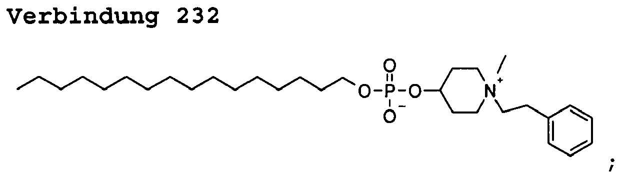

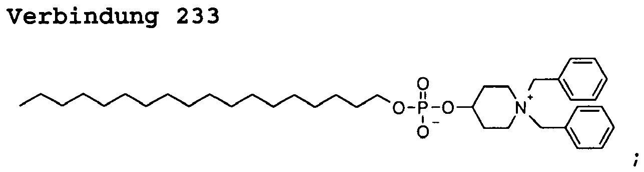

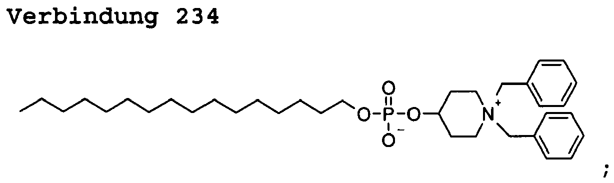

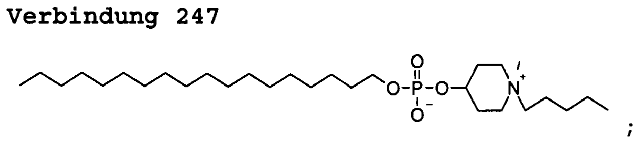

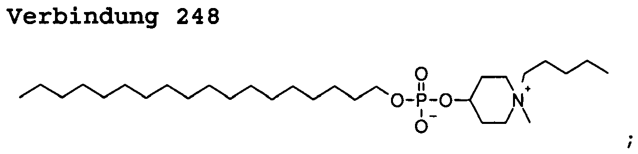

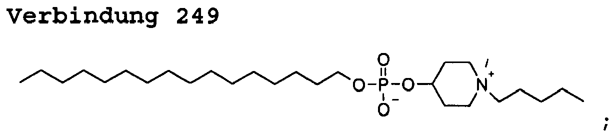

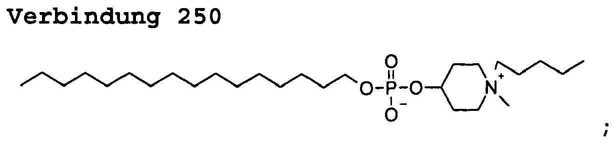

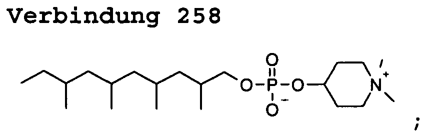

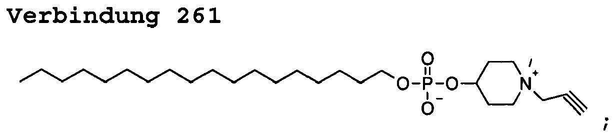

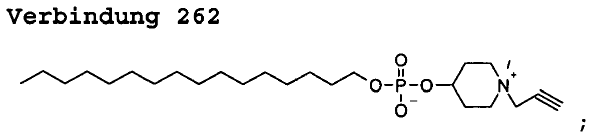

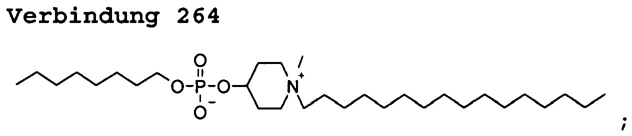

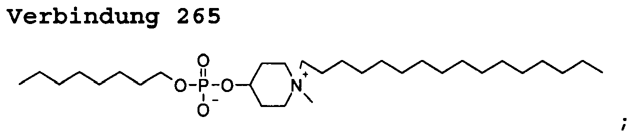

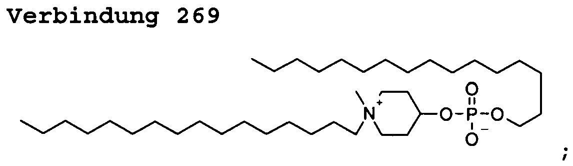

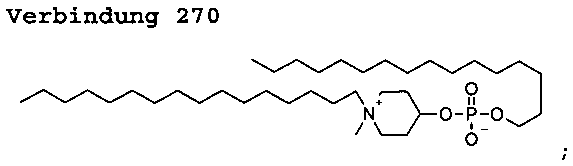

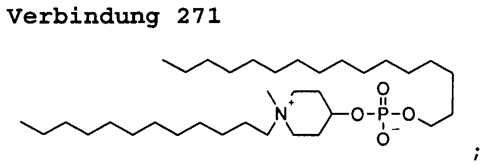

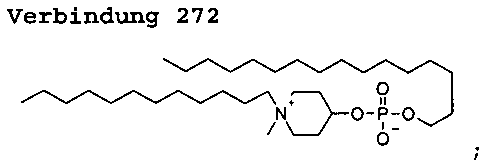

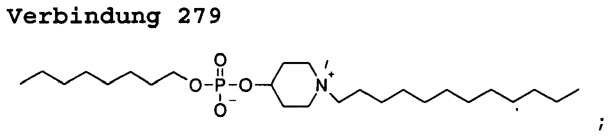

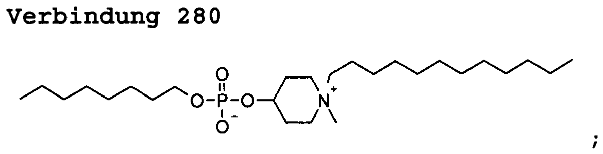

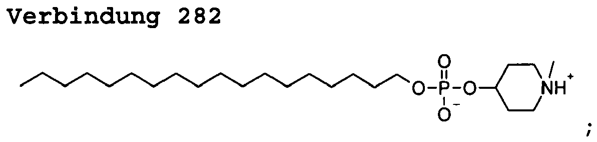

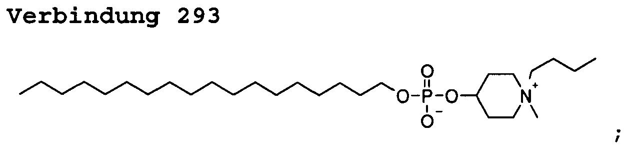

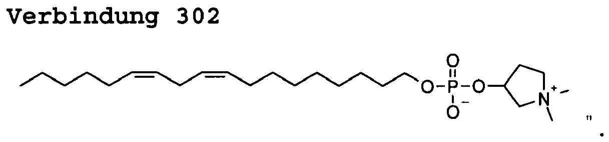

4. Verwendung nach einem der Ansprüche 1 bis 3, wobei das Alkylphospholipidderivat aus der aus: "Verbindung 1, 4, 5 und/oder Verbindung 17" bestehenden Gruppe ausgewählt ist.

5. Verwendung nach einem der Ansprüche 1 bis 4, wobei die Krankheit und/oder das pathophysiologische Leiden aus der aus "Aspergillose, Blastomykose, Kandidose, Chromoblastomykose, Kokzidioidomykose, Kryptokokkose, Dermatomykose, Dermatophytose, Histoplasmose, Lobomykose, Mukormykose, Myzetom,
mykotischer Keratitis, Okulomykose, Onychomykose, Otomykose, Paracoccidiomykose, Phaeohyphomykose, Trichosporose, Pityriasis versicolor, Rhinosporidiose, Sporotrichose, Tinea barbae, Tinea capitis, Tinea corporis, Tinea cruris, Tinea favosa, Tinea nigra, Tinea pedis, Tinea unguium, Zygomykose sowie ihren verschiedenen Formen und Unterformen" bestehenden Gruppe ausgewählt ist.

6. Verwendung nach einem der Ansprüche 1 bis 5, wobei das Säugetier aus der aus "Mensch, domestiken Tieren, Rindern, Nutztieren, Haustieren, Kuh, Schaf, Schwein, Ziege, Pferd, Pony, Esel, Maulesel, Maultier, Hase, Kaninchen, Katze, Hund, Mehrschwein, Hamster, Ratte, Maus" bestehenden Gruppe ausgewählt ist und vorzugsweise ein Mensch ist.

7. Verwendung nach einem der Ansprüche 1 bis 6, wobei ein solches Medikament mindestens eine zusätzliche pharmakologisch wirksame Substanz umfasst.

8. Verwendung nach einem der Ansprüche 1 bis 7, wobei das solches Medikament bevor und/oder während und/oder nach der Behandlung mit mindestens einer zusätzlichen pharmakologisch wirksamen Substanz anzuwenden ist.

9. Verwendung nach einem der Ansprüche 7 bis 8, wobei die mindestens eine zusätzliche pharmakologisch wirksame Substanz aus der aus "Benznidazol (N-benzyl-2-nitroimidazol-1-ylacetamid); Nifurtimox [3-Methyl-4-(5-nitrofurfurylidenamino)-tetrahydro-1,4-thiazin-1,1-dioxid]; Amphotericin B [(1R,3S,5R,6R,9R,11R,15S,16R,17R,18S,19E,21E,23E,-25E,27E,29E,31E,33R,35S,36R,37S)-33-[(3-Amino-3,6-didesoxy-beta-D-mannopyranosyl)oxy]-1,3,5,6,9,11,-17,37-octahydroxy-15,16,18-trimethyl-13-oxo-14,39-dioxabicyclo[33.3.1]-nonatriaconta-19,21,23,25,-27,29,31-heptaen-36-carbonsäure]; liposomalem Amphotericin B, Sitamaquin (N,N-Diethyl-N'-(6-methoxy-4-methyl-8-chinolinyl)-1,6-hexandiamin) und/oder Paromomycin [O-2-Amino-2-desoxy-alpha-D-glucopyranosyl-(1-4)-O-[O-2,6-diamino-2,6-didesoxy-beta-L-idopyranosyl-(1-3)-beta-D-ribofuranosyl-(1-5)]-2-desoxy-D-streptamin]" bestehenden Gruppe ausgewählt ist.

10. Verwendung nach einem der Ansprüche 7 bis 8, wobei es sich bei dem Alkylphospholipidderivat um "Perifosin (Octadecyl-1,1-dimethylpiperidino-4-yl-phosphat)" handelt und die mindestens eine zusätzliche pharmakologisch wirksame Substanz aus der aus "Benznidazol (N-benzyl-2-nitroimidazol-1-ylacetamid); Nifurtimox [3-Methyl-4-(5-nitro-furfurylidenamino)tetrahydro-1,4-thiazin-1,1-dioxid]; Amphotericin B [(1R,3S,5R,6R,9R,11R,15S,-16R,17R,18S,19E,21E,23E,25E,27E,29E,31E,33R,35S,-36R,37S)-33-[(3-Amino-3,6-didesoxy-beta-D-manno-pyranosyl)oxy]-1,3,5,6,9,11,17,37-octahydroxy-15,-16,18-trimethyl-13-oxo-14,39-dioxabicyclo[33.3.1]-nonatriaconta-19,21,23,25,27,29,31-heptaen-36-carbonsäure]; liposomalem Amphotericin B, Sitamaquin (N,N-Diethyl-N'-(6-methoxy-4-methyl-8-chinolinyl)-1,6-hexandiamin) und/oder Paromomycin [O-2-Amino-2-desoxy-alpha-D-glucopyranosyl-(1-4)-O-[O-2,6-diamino-2,6-didesoxy-beta-L-idopyranosyl-(1-3)-beta-D-ribofuranosyl-(1-5)]-2-desoxy-D-streptamin]" bestehenden Gruppe ausgewählt ist.

11. Verwendung nach einem der Ansprüche 7 bis 10, wobei das mindestens eine Alkylphospholipidderivat und die mindestens eine zusätzliche pharmakologisch aktive Substanz als pharmazeutisches Kit anzuwenden sind.

## Revendications

1. Utilisation d'un dérivé d'alkylphospholipides selon la formule (I) 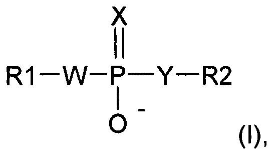 dans laquelle :
W, X, Y indépendamment sont choisis dans le groupe constitué par : « un atome d'oxygène, un atome de soufre » ;
R1 est « -R5 » ;
R2 est « -R8 » ;
R5 est choisi indépendamment dans le groupe constitué par : « C8-C30alkyle substitué ou non substitué » ;
R8 est choisi dans le groupe constitué par : « hétérocycle substitué ou non substitué », hétérocycle étant
(i) un système de cycle carboné saturé, partiellement insaturé ou aromatique à 5, 6 ou 7 chaînons avec au moins un hétéroatome choisi dans le groupe constitué par : « un atome d'azote, un atome d'oxygène, un atome de soufre, un atome d'arsenic », et à condition qu'au moins un hétéroatome est un atome d'azote quaternaire ou un atome d'arsenic quaternaire,
et l'hétérocycle, s'il est substitué, est substitué par au moins un radical R12, qui dans le cas de deux radicaux R12 ou plus sont choisis indépendamment les uns des autres et sont identiques, partiellement identiques ou différents ;
R12 est indépendamment les uns des autres choisi dans le groupe constitué par: « un atome d'hydrogène, C1-C18alkyle substitué ou non substitué, C3-C8cycloalkyle substitué ou non substitué, (C1-C12alkyl)ₛ-B-(C1-C12alkyl)ₜ-C-(C1-C12alkyle)ᵤ substitué ou non substitué, aryle substitué ou non substitué, hétéroaryle substitué ou non substitué, alcoxy substitué ou non substitué, -OH, halogène, -F, -Cl, -Br, -I, =O,
-C(O)O-(C1-C12alkyle), -C(O)O-(C3-C8cycloalkyle),-C(O)O-aryle, -C(O)O-hétéroaryle, -C(O)O-hétérocyclyle,-C(O)-(C1-C12alkyle), -C(O)-(C3-C8cycloalkyle), -C(O)-aryle, -C(O)-hétéroaryle, -C(O)-hétérocyclyle » ;
B, C sont indépendamment l'un de l'autre choisis dans le groupe constitué par « un atome d'oxygène ; un atome de soufre ; S(O₂) » ;
s, t, u indépendamment les uns des autres sont 0 ou 1 ;
pour la fabrication d'un médicament destiné au traitement ou à la prophylaxie des maladies et/ou des affections physiopathologiques chez les mammifères qui sont provoquées par un champignon choisi dans le groupe constitué par « Absidia spp. , Acremonium spp. , Alternaria spp. , Aspergillus spp. , Bipolaris spp. , Candida spp. , Cladophialophora spp. , Cladosporium spp. , Coccidioides spp., Coniothyrium spp., Cryptococcus spp. , Cunninghamella spp. , Curvularia spp. , Epidermophyton spp. , Exophiala spp. , Exserohilum spp., Fonsecaea spp. , Fusarium spp. , Histoplasma spp., Lacazia spp., Lasiodiplodia spp. , Leptosphaeria spp., Madurella spp., Microsporum spp., Mucor spp. , Mucorales spp., Neotestudina spp. , Ochroconis spp. , Onychocola spp., Paecilomyces spp., Paracoccidioides spp. , Penicillium spp., Phialophora spp., Pseudallesheria spp., Pyrenochaeta spp. , Rhizomucor spp. , Rhizopus spp. , Scedosporium spp. , Scopulariopsis spp. , Scytalidium spp. , Sporothrix spp. , Trichophyton spp. et/ou Wangiella spp. »

2. Utilisation d'un dérivé d'alkylphospholipide selon la revendication 1, **caractérisée en ce que** le champignon est de préférence choisi dans le groupe constitué par « Absidia spp. , Aspergillus spp. , Bipolaris spp. , Candida spp. , Cryptococcus spp. , Cunninghamella spp. , Exophiala spp. , Fusarium spp. , Paecilomyces spp. , Rhizopus spp. et/ou Scedosporium spp. ».

3. Utilisation selon l'une ou l'autre des revendications 1 et 2, **caractérisée en ce que** le dérivé d'alkylphospholipide est choisi dans le groupe constitué de :

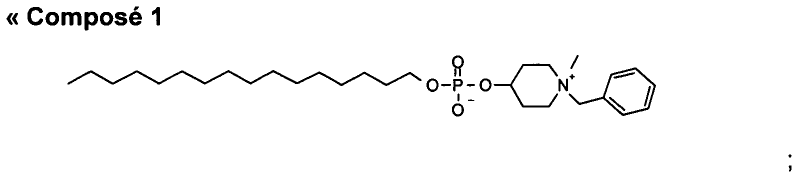

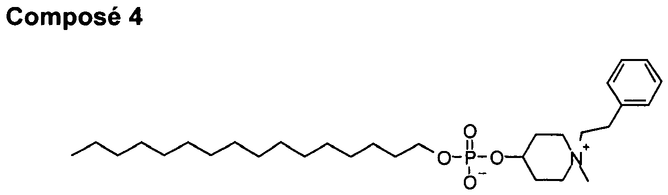

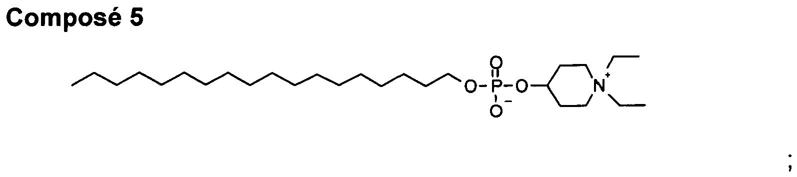

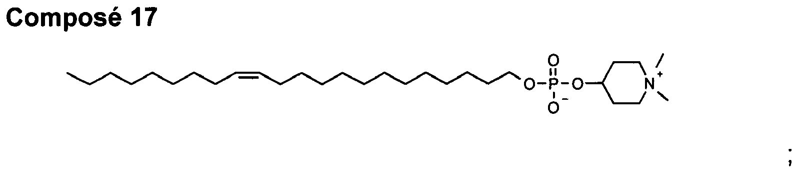

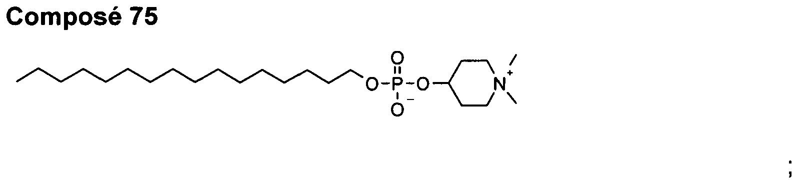

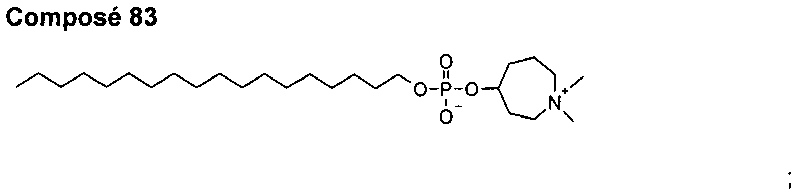

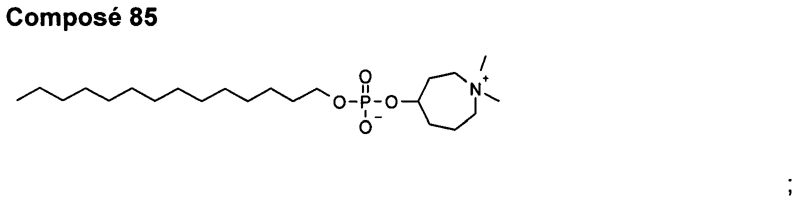

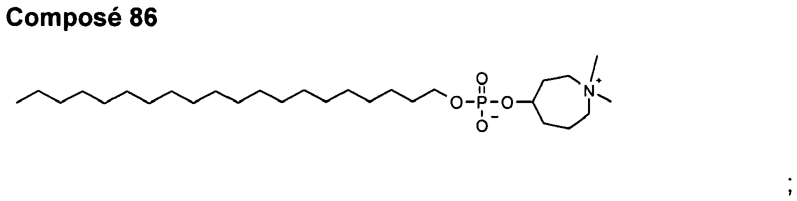

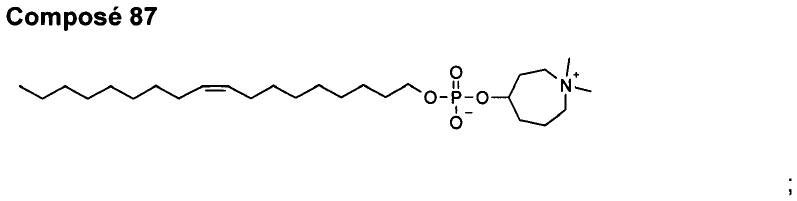

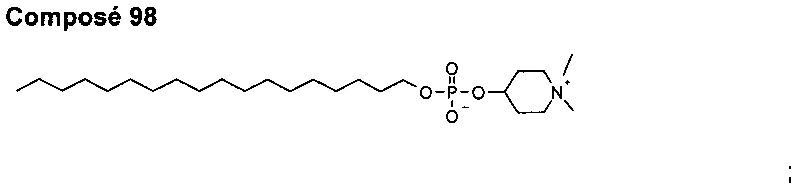

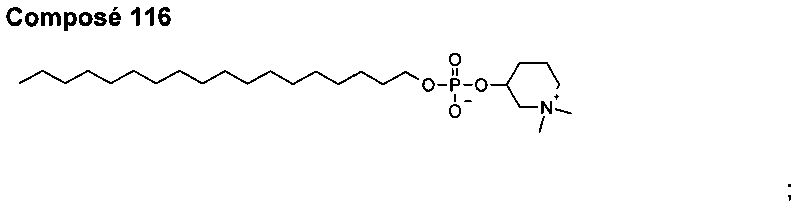

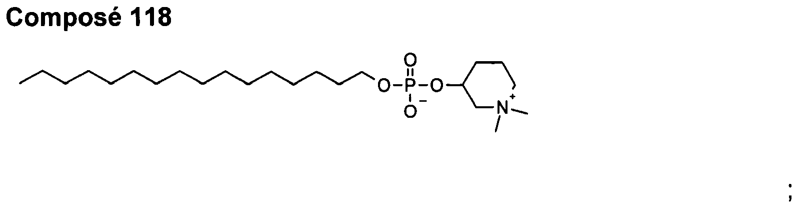

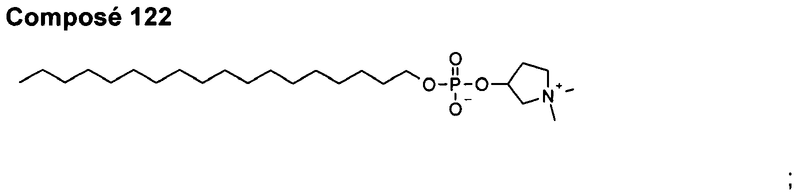

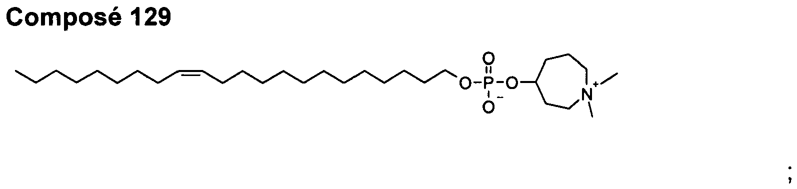

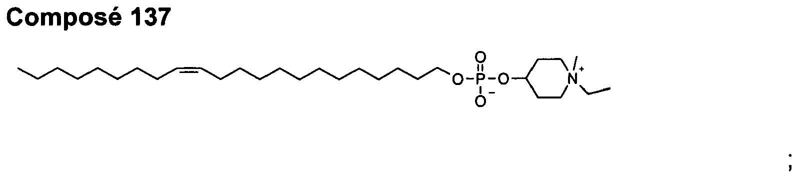

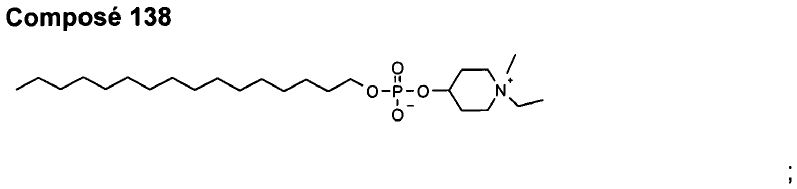

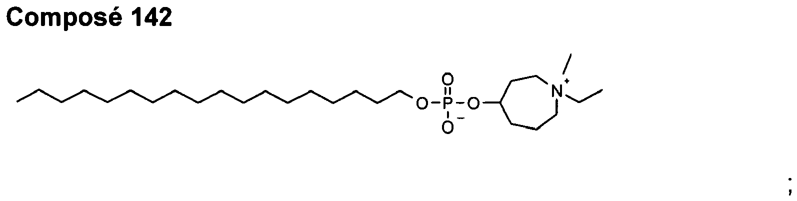

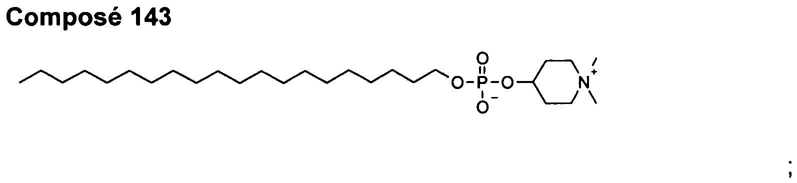

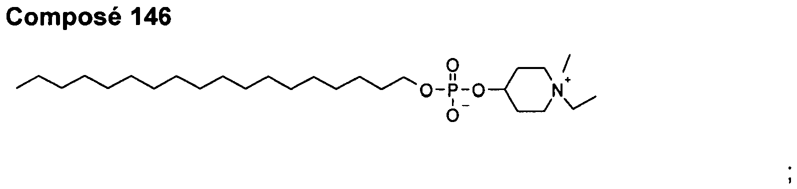

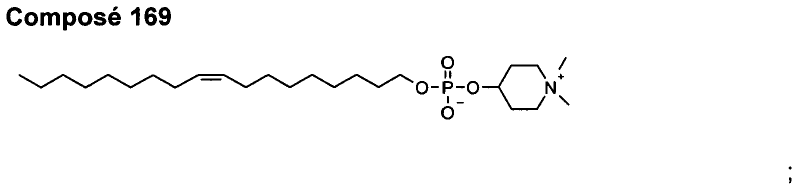

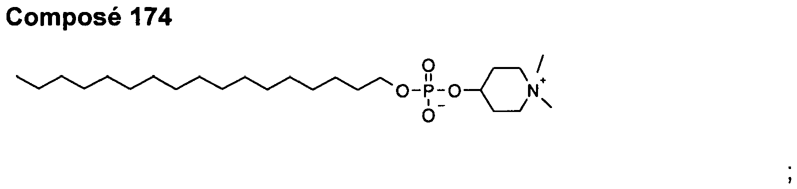

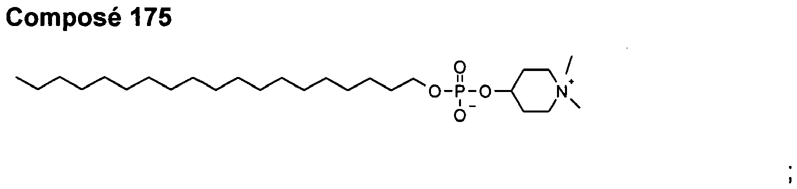

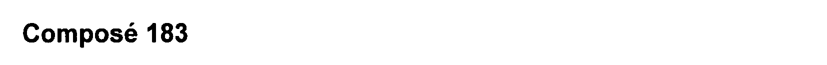

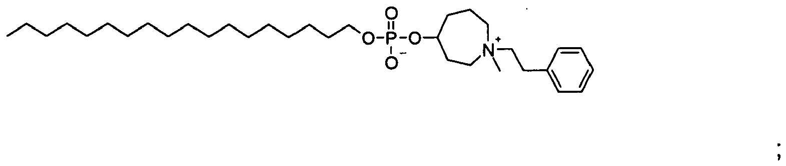

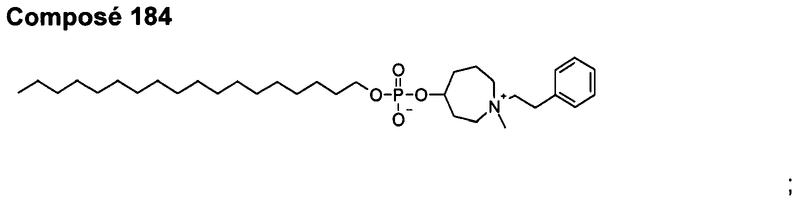

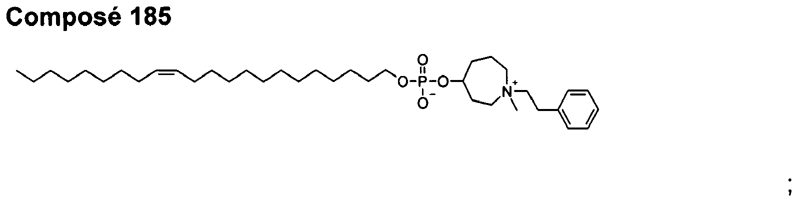

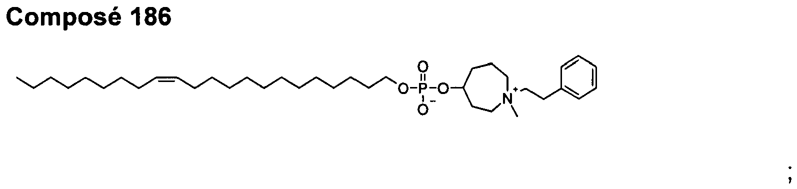

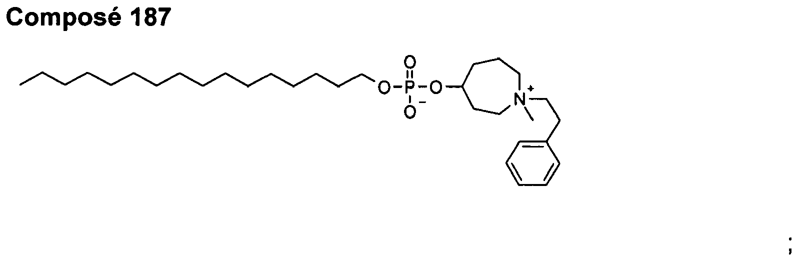

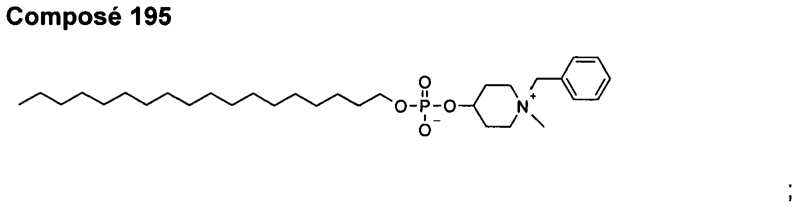

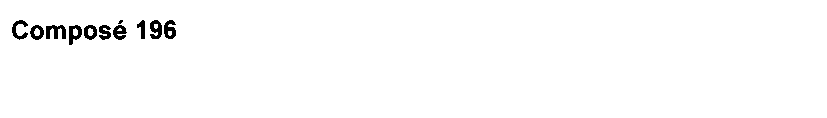

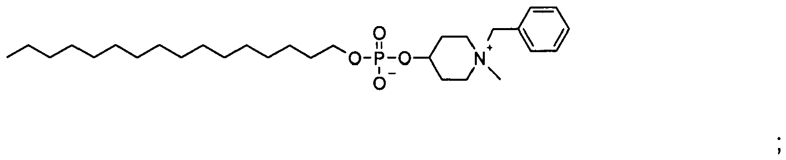

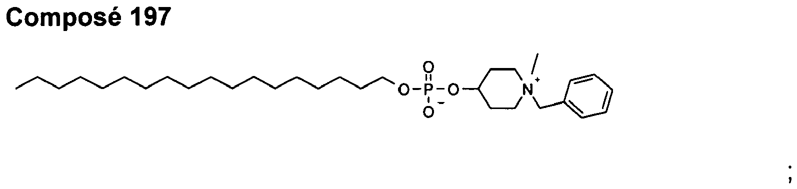

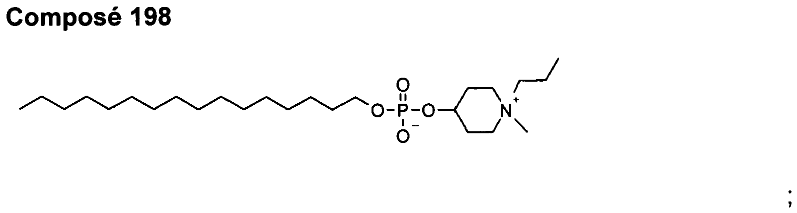

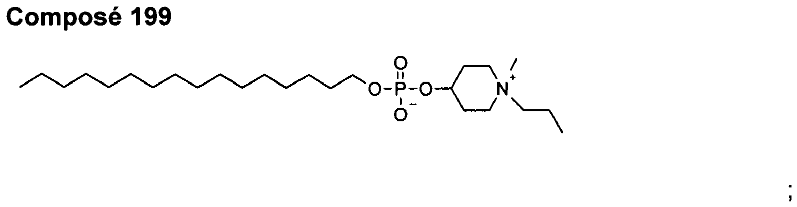

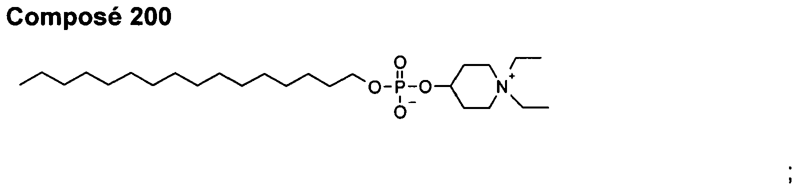

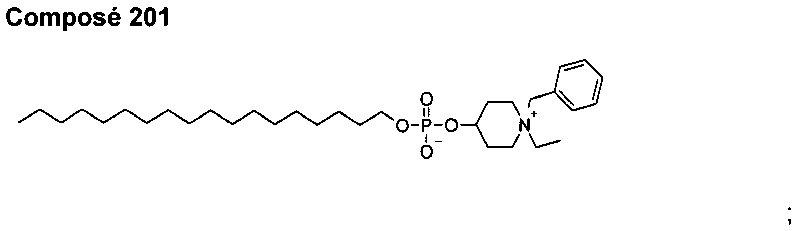

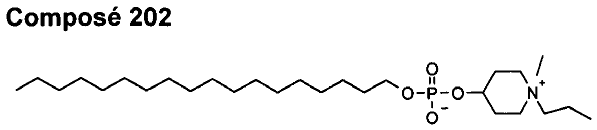

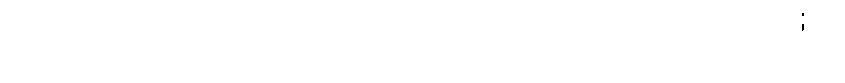

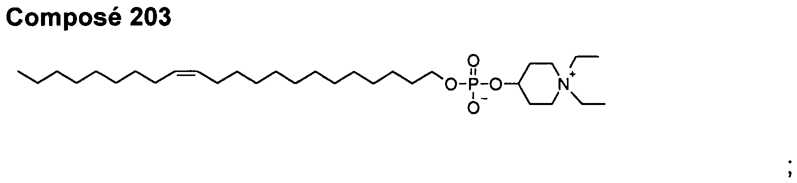

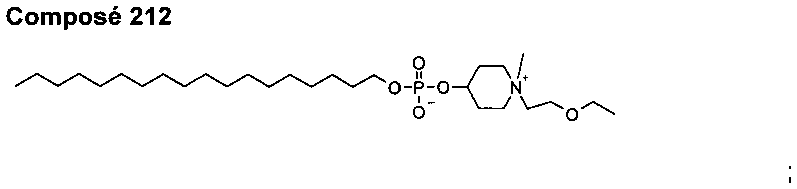

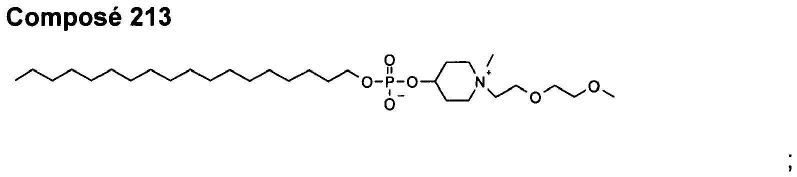

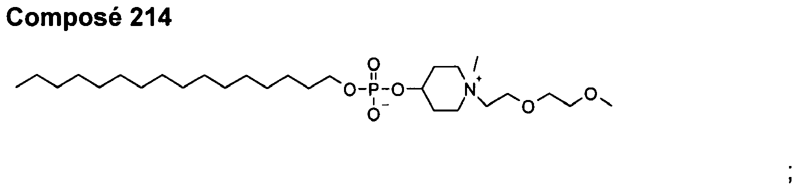

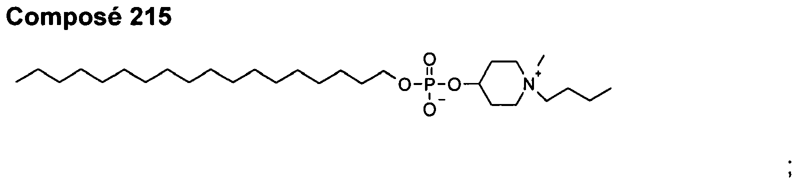

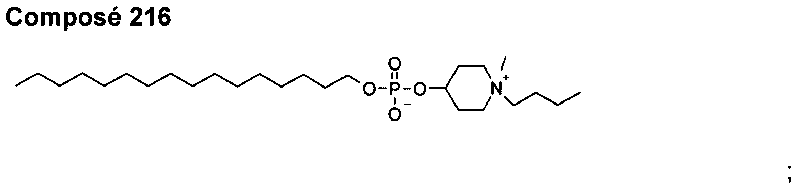

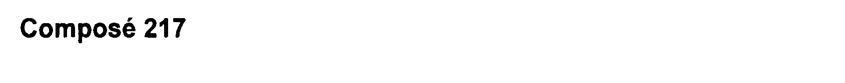

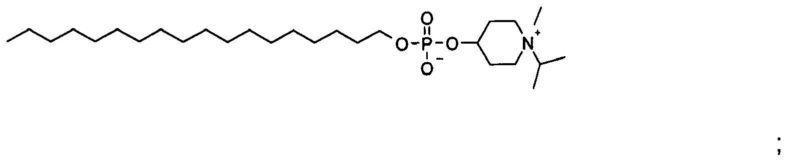

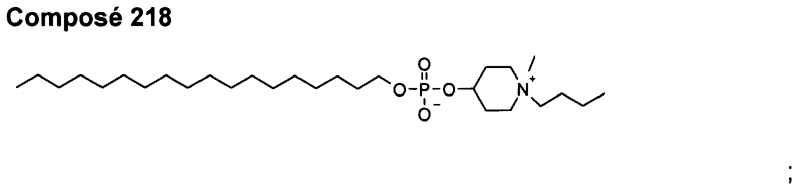

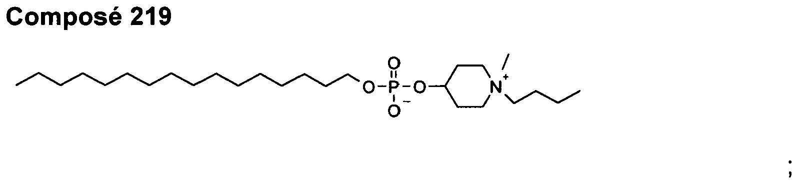

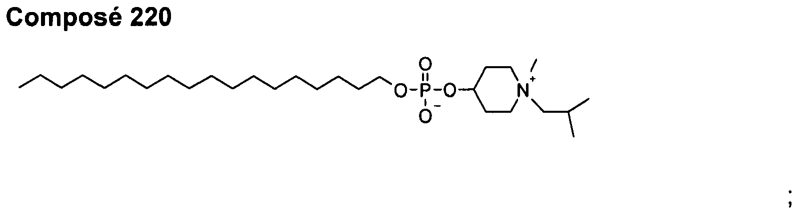

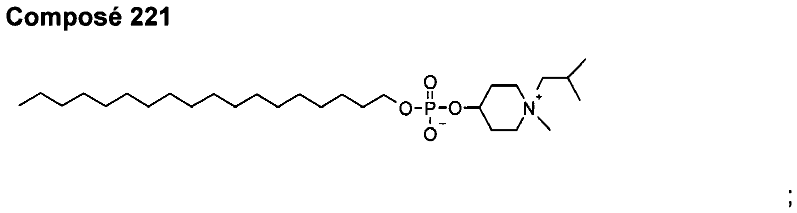

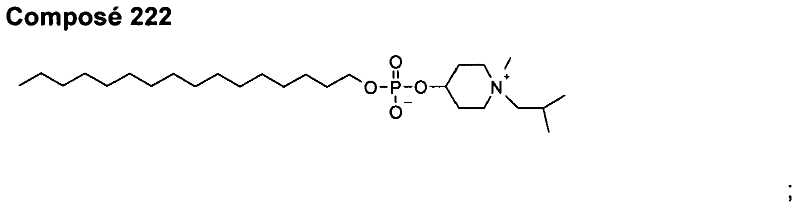

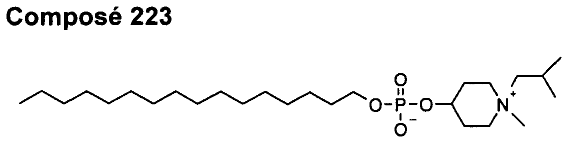

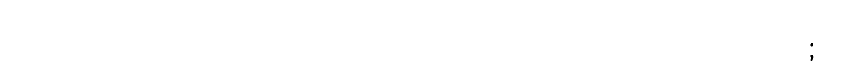

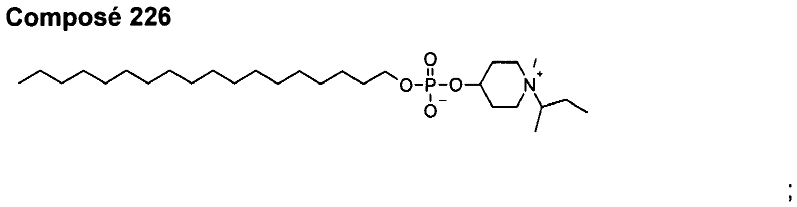

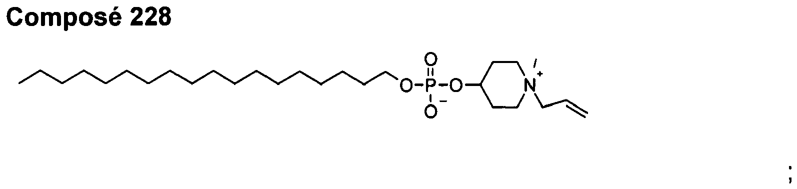

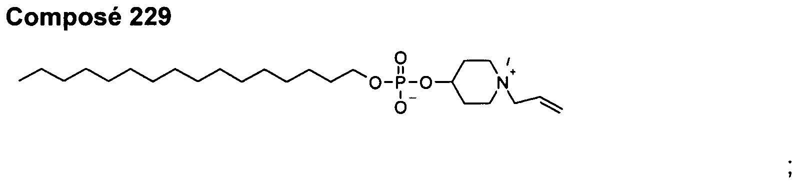

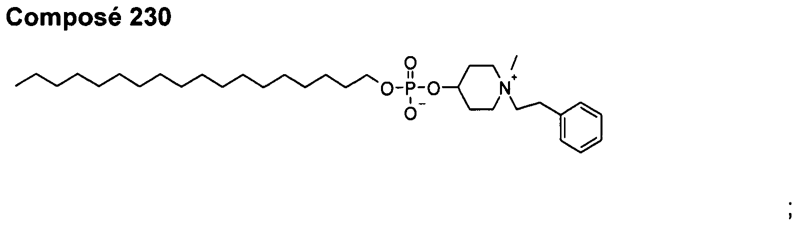

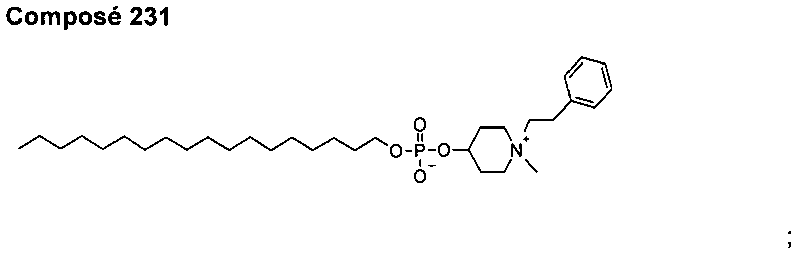

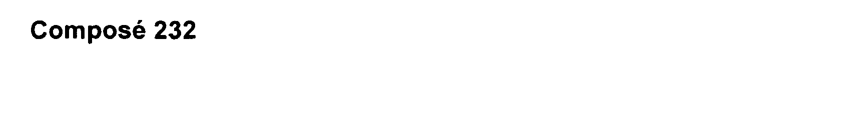

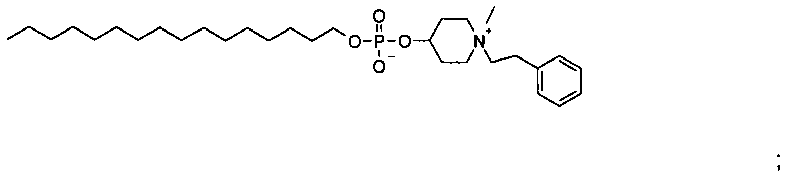

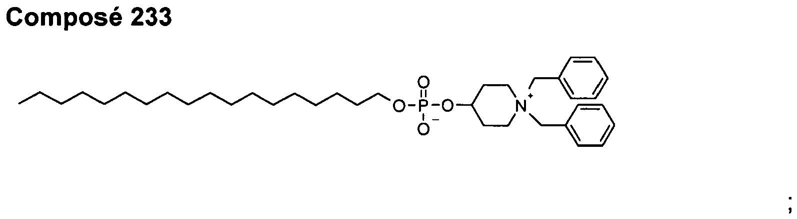

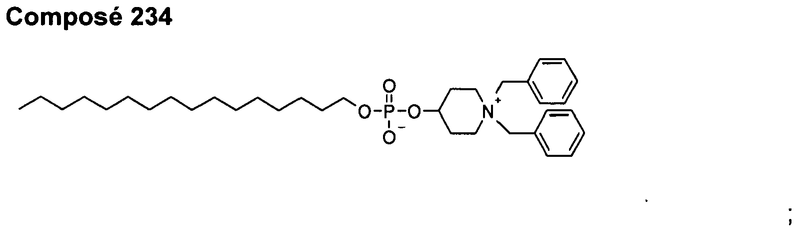

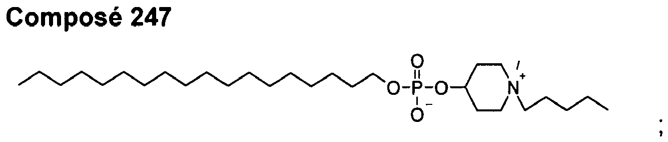

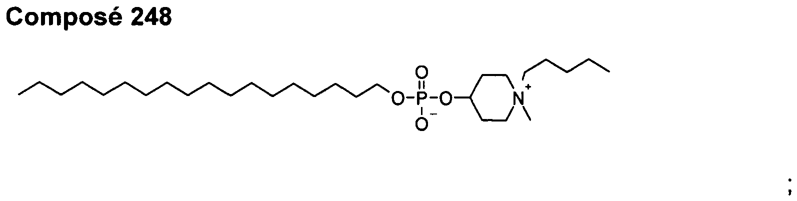

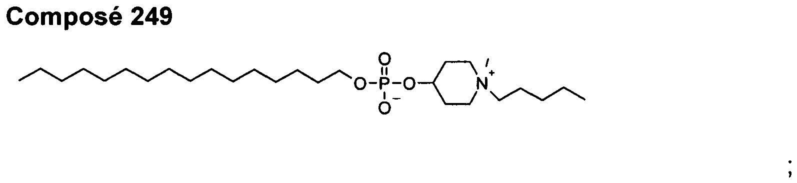

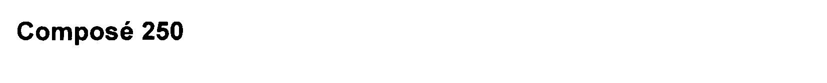

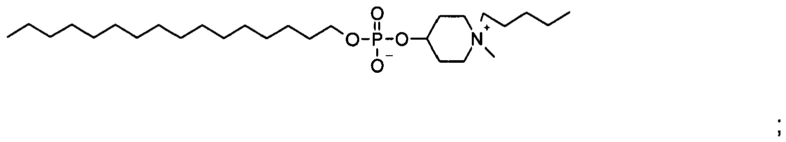

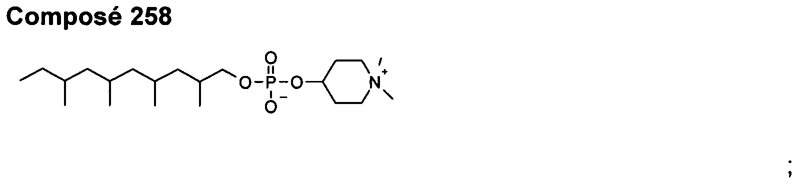

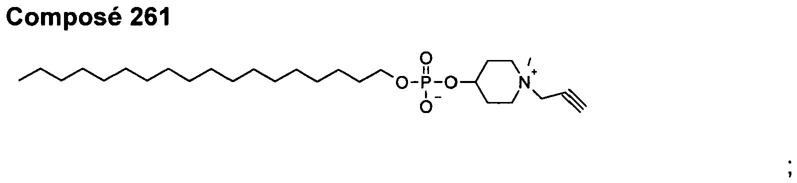

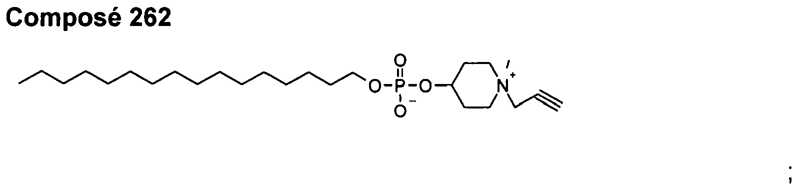

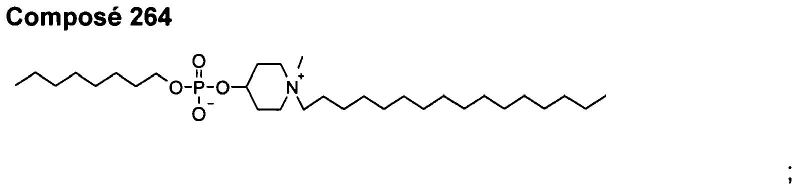

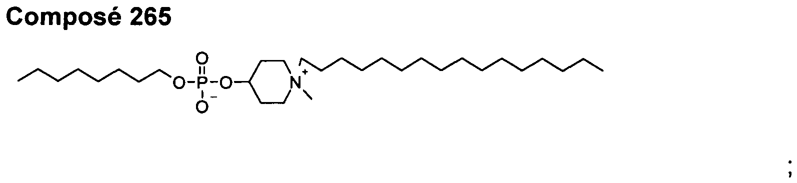

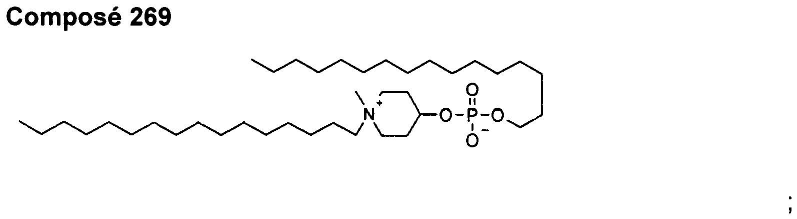

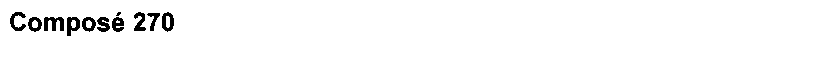

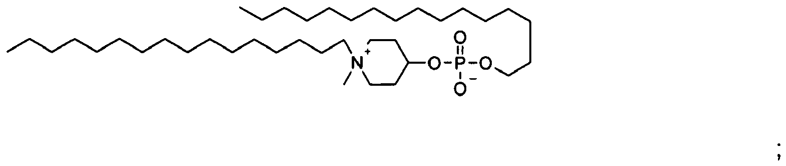

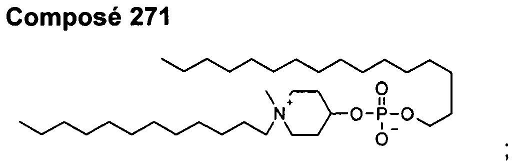

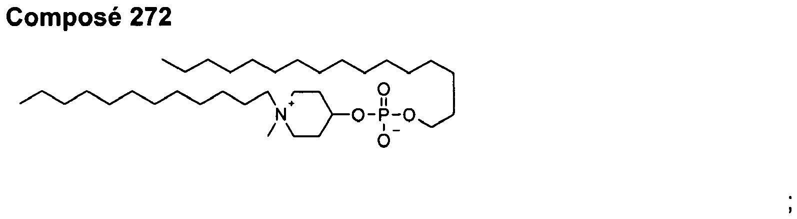

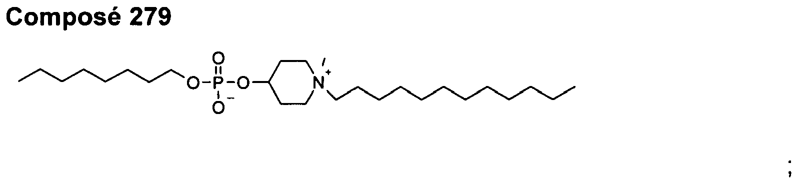

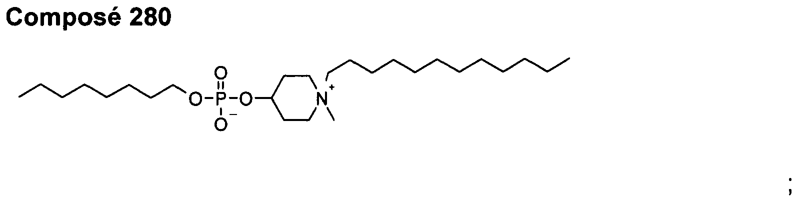

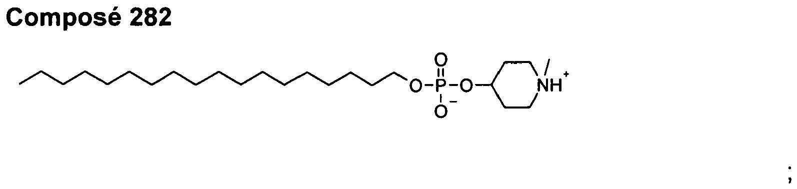

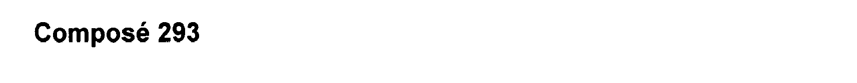

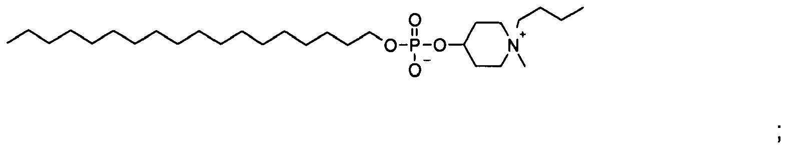

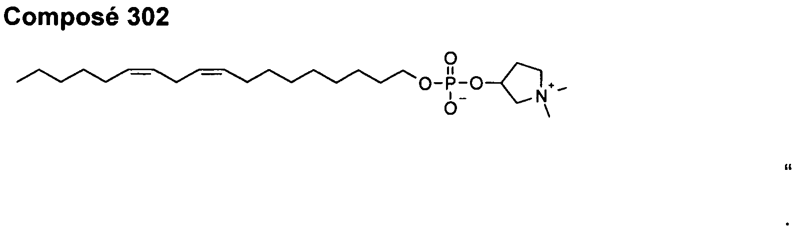

4. Utilisation selon les revendications 1 à 3, **caractérisée en ce que** le dérivé d'alkylphospholipide est choisi dans le groupe constitué de : « **Composé 1, 4, 5** et/ou **composé 17** ».

5. Utilisation selon l'une quelconque des revendications 1 à 4, **caractérisée en ce que** la maladie et/ou l'affection physiopathologique est choisie dans le groupe constitué de « aspergillose, blastomycose, candidose, chromoblastomycose, coccidioidomycose, cryptococcose, dermatomycose, dermatophytose, histoplasmose, lobomycose, mucormycose, mycétome, kératite mycotique, oculomycose, onychomycose, otomycose, paracoccidiomycose, phaeohyphomycose, piedra, pityriasis versicolor, rhinosporidiose, sporotrichose, tinea barbae, tinea capitis, tinea corporis, tinea cruris, tinea favosa, tinea nigra, tinea pedis, tinea unguium, zygomycose ainsi que leurs différentes formes et sous-formes ».

6. Utilisation selon l'une quelconque des revendications 1 à 5, **caractérisée en ce que** le mammifère est choisi dans le groupe constitué par « les humains, les animaux domestiques, les bovins, les animaux d'élevage, les animaux de compagnie, la vache, le mouton, le cochon, la chèvre, le cheval, le poney, l'âne, le bardot, le mulet, le lièvre, le lapin, le chat, le chien, le cobaye, le hamster, le rat, la souris » et de préférence est humain.

7. Utilisation selon l'une quelconque des revendications 1 à 6, **caractérisée en ce qu'**un tel médicament comprend au moins une substance pharmacologiquement active supplémentaire.

8. Utilisation selon l'une quelconque des revendications 1 à 7, **caractérisée en ce que** le médicament est à appliquer avant et/ou pendant et/ou après traitement avec au moins une substance pharmacologiquement active supplémentaire.

9. Utilisation selon l'une ou l'autre des revendications 7 et 8, **caractérisée en ce que** ladite au moins une substance pharmacologiquement active supplémentaire est choisie dans le groupe constitué par « le benznidazole (N-benzyl-2-nitroimidazol-1-yl-acétamide) ; le nifurtimox [3-méthyl-4-(5-nitrofurfuryl-idénamino)tétrahydro-1,4-thiazine-1,1-dioxyde] ; l'amphotéricine B [acide (1 R, 3S, 5R, 6R, 9R, 11 R, 15S, 16R, 17R, 18S, 19E, 21E, 23E, 25E, 27E, 29E, 31E, 33R, 35S, 36R, 37S)-33-[(3-amino-3,6-didésoxy-bêta-D-mannopyranosyl)oxy]-1, 3, 5, 6, 9, 11, 17, 37-octahydroxy-15,16,18-triméthyl-13-oxo-14,39-dioxabicyclo[33,3,1]-nonatriaconta-19, 21, 23, 25, 27, 29, 31-heptène-36-carboxylique] ; l'amphotéricine B liposomale, la sitamaquine (N, N-diéthyl-N'-(6-méthoxy-4-méthyl-8-quinoléinyl)-1,6-hexanediamine) et/ou la paromomycine [O-2-amino-2-désoxy-alpha-D-glucopyranosyl-(1-4)-O-[O-2,6-diamino-2,6-didésoxy-bêta-L-idopyranosyl-(1-3)-bêta-D-ribofuranosyl-(1-5)]-2-désoxy-D-streptamine] ».

10. Utilisation selon l'une ou l'autre des revendications 7 et 8, **caractérisée en ce que** le dérivé d'alkylphospholipide est « la périfosine (octadécyl-1,1-diméthyl-pipéridino-4-yl-phosphate) » et ladite au moins une substance pharmacologiquement active supplémentaire est choisie dans le groupe constitué par « le benznidazole (N-benzyl-2-nitroimidazol-1-yl-acétamide) ; le nifurtimox [3-méthyl-4-(5-nitrofurfuryl-idénamino)tétrahydro-1,4-thiazine-1,1-dioxyde] ; l'amphotéricine B [acide (1 R, 3S, 5R, 6R, 9R, 11 R, 15S, 16R, 17R, 18S, 19E, 21 E, 23E, 25E, 27E, 29E, 31 E, 33R, 35S, 36R, 37S)-33-[(3-amino-3,6-didésoxy-bêta-D-mannopyranosyl)oxy]-1, 3, 5, 6, 9, 11, 17, 37-octahydroxy-15,16,18-triméthyl-13-oxo-14,39-dioxabicyclo[33,3,1]-nonatriaconta-19, 21, 23, 25, 27, 29, 31-heptène-36-carboxylique] ; l'amphotéricine B liposomale, la sitamaquine (N,N-diéthyl-N'-(6-méthoxy-4-méthyl-8-quinoléinyl)-1,6-hexanediamine) et/ou la paromomycine [O-2-amino-2-désoxy-alpha-D-glucopyranosyl-(1-4)-O-[O-2,6-diamino-2,6-didésoxy-bêta-L-idopyranosyl-(1-3)-bêta-D-ribofuranosyl-(1-5)]-2-désoxy-D-streptamine] ».

11. Utilisation selon l'une quelconque des revendications 7 à 10, **caractérisée en ce que** ledit au moins un dérivé d'alkylphospholipide et ladite au moins une substance pharmacologiquement active supplémentaire sont à appliquer en tant que kit pharmaceutique.
